# EUROPEAN PATENT APPLICATION

(11) **EP 4 063 380 A1**
(43) Date of publication of application: **28.09.2022**
(21) Application number: 21164713.6
(22) Date of filing: 24.03.2021
(51) Int. Cl.: C07K 7/54, A61K 38/00, C07K 14/00

(54) **INHIBITORS OF THE WNT SIGNALING PATHWAY AND USES THEREOF**

(71) Applicant: Stichting VU, 1081 HV Amsterdam (NL)
(72) Inventor: GROSSMANN, Tom Norbert, 1081 HV Amsterdam (NL); WENDT, Mathias, 1081 HV Amsterdam (NL); EFREM, Nina-Louisa, 1081 HV Amsterdam (NL); BELLAVITA, Rosa, 1081 HV Amsterdam (NL); VAN RAMSHORST, Thirza, 1081 HV Amsterdam (NL); GERBER, Alan, 1081 HV Amsterdam (NL)
(74) Representative: V.O.

(57) **Abstract**

The invention relates to a cyclic peptide comprising the amino acid sequence N-terminal-X₄X₃X₂X₁₋ₐ-Y₁Y₂Y₃Y₄-C-terminal, wherein -a- represents a linker, and wherein the peptide comprises a linker -c- between the N-terminal amino acid and C-terminal amino acid, and/or wherein the two amino acids of one or two of the following amino acid pairs form a side chain-to-side chain bridged di-amino acid forming a bridge -b-: X₁ and Y₁; X₂ and Y₂; X₃ and Y₃; and X₄ and Y₄. The invention further relates to the use of said cyclic peptide as a medicament, especially for inhibiting or preventing a Wingless/integrase-1 (Wnt)-dependent disease in an individual in need thereof.

## Description

### FIELD

The invention relates to inhibitors of the Wnt signaling pathway in cells. Wnt inhibition finds use in treating Wnt-mediated disease conditions, such as aberrant cell proliferation and/or cell differentiation.

### INTRODUCTION

In protein complexes, binding partners adopt defined three-dimensional structures in which amino acid (aa) side chains are aligned to facilitate intermolecular interactions. The mimicry of underlying secondary and tertiary structures has provided selective and high-affinity ligands that have been used for the elucidation and modulation of biological processes [Horne and Grossmann, 2020. Nat Chem 12: 331-337; Milroy et al., 2014. Chem Rev 114: 4695-47481]. Peptidomimetic structures proved particularly useful for the inhibition of protein-protein interactions (PPIs) [Pelay-Gimeno et al., 2015. Angew Chem Int Ed 54: 8896-8927; Cunningham et al., 2017. Curr Opin Struct Biol 44: 59-662], here mainly recapitulating α-helices [Azzarito et al., 2013. Nat Chem 5: 161-173; Cromm et al., 2015. ACS Chem Biol 10: 1362-1375; Iegre et al., 2018. Adv Therap 1: 1800052; Guarracino et al., 2019. Chem Rev 119: 9915-9949]. Even though β sheets occur frequently in PPIs [Watkins and Arora, 2014. ACS Chem Biol 9: 1747-1754], the corresponding mimetics have been less explored as PPI inhibitors [Laxio Arenas et al., 2019. Curr Opin Chem Biol 52: 157-167; Merritt et al., 2020. Pept Sci 112: e241455]. In general, most β sheet mimetics are based on β-hairpin peptides which comprise two antiparallel β strands connected by a turn. [Merritt et al., 2020. Pept Sci 112: e241455; Robinson, 2013. Chimia 67: 885-8906]. The β-hairpin structure can be stabilized by backbone modifications within the β strands, β-sheet-inducing turn mimetics and by hairpin macrocyclization. [Laxio Arenas et al., 2019. Curr Opin Chem Biol 52: 157-167; Merritt et al., 2020. Pept Sci 112: e241455; Robinson, 2013. Chimia 67: 885-8906; Cheng et al., 2012. Nat Chem 4: 927-933]. Macrocyclization has predominantly been implemented via head-to-tail as well as cross-strand tethers with the latter using different bridges such as disulfides [Wang and Craik, 2018. Nat Chem Biol 14: 417-427], triazoles [White et al., 2020. Angew Chem Int Ed 59: 11273-11277; Celentano et al., 2016. Chem Eur J 22: 5534-5537; Hanold et al., 2015. PLoS One 10: e0118796; Park and Waters, 2013. Org Biomol Chem 11: 69-77; Empting et al., 2011. Angew Chem Int Ed 50: 5207-5211; Holland-Nell and Meldal, 2011. Angew Chem Int Ed 50: 5204-5206], amides[García-Aranda et al., 2011. Bioorg Med Chem 19: 7526-7533; Giddu et al., 2009. J Med Chem 52: 726-736; Yang et al., 2018. J Med Chem 61: 8174-8185], thioethers[Yang et al., 2018. J Med Chem 61: 8174-8185; Zhao et al., 2016. Chembiochem 17: 1416-1420; Cui et al., 2013. Angew Chem Int Ed 52: 9558-9562] and hydrocarbon structures. [Cui et al., 2013. Angew Chem Int Ed 52: 9558-9562; Lingard et al., 2014. Angew Chem Int Ed 53: 3650-3653]. Owing to the often low tendency of β hairpin mimetics to penetrate cells, they have been mainly applied to target extracellular PPIs [Laxio Arenas et al., 2019. Curr Opin Chem Biol 52: 157-167; Merritt et al., 2020. Pept Sci 112: e241455; Wang and Craik, 2018. Nat Chem Biol 14: 417-427] thereby excluding a large number of highly interesting intracellular PPIs.

The inhibition of certain pathological PPIs is considered a promising therapeutic approach for numerous diseases [Vidal et al., 2011. Cell 144: 986-998; Valeur et al., 2017. Angew Chem In. Ed 56: 10294-10323]. Due to the extended nature of involved interaction areas [Keskin et al., 2008. Chem Rev 108: 1225-1244], conventional ligand-discovery approaches that rely on small molecular scaffolds often fail to provide potent PPI inhibitors [Scott et al., 2016. Nat Rev Drug Discov 15: 533-550]. A prime example for a therapeutically interesting protein which participates in numerous PPIs is the transcriptional coactivator β-catenin serving as the central intracellular interaction hub of the Wnt signaling pathway[Clevers and Nusse, 2012. Cell 149: 1192-1205]. Importantly, β-catenin binding to transcription factor proteins of the T-cell factor (TCF) family, including Lymphoid Enhancer Binding Factor 1 (LEF1), activates the expression of Wnt target genes thereby stimulating cell growth and proliferation [Cadigan and Waterman, 2012. Cold Spring Harb Perspect Biol 4: a007906]. Hyperactivation of the Wnt pathway is associated with various forms of cancer rendering the inhibition of the β-catenin/TCF interaction an attractive strategy for therapeutic intervention[Cui et al., 2018. Trends Biochem Sci 43: 623-634]. Due to the challenges associated with the use of small molecules [Hahne and Grossmann, 2013. Bioorg Med Chem 21: 4020-4026; Zhang et al., 2020. Pharmacol Res 160: 104794], the development of peptide-derived PPI inhibitors has been pursued [Huang et al., 2014. ACS Chem Biol 9: 193-201; Dietrich et al., 2017. Cell Chem Biol 24: 958-968; Diderich et al., 2016. ACS Chem Biol 11: 1422-1427; Hsieh et al., 2016. Sci Rep 6: 19156; Schneider et al., 2018. Nat Commun 9: 4396; Grossmann et al., 2012. Proc Natl Acad Sci USA 109: 17942-17947]. However, these efforts have not resulted in therapeutically useful inhibitors of the β-catenin/TCF interaction mainly due to their unfavorable physico-chemical properties and/or low bioavailability [Hahne and Grossmann, 2013. Bioorg Med Chem 21: 4020-4026; Zhang et al., 2020. Pharmacol Res 160: 104794]. The development of inhibitors would greatly benefit from novel cell-permeable and high affinity ligands ideally with a structurally characterized binding mode to support subsequent optimization efforts towards clinical inhibitors.

### BRIEF DESCRIPTION OF THE INVENTION

Here, the structure-based design of β-sheet mimicking mono- and bicyclic molecules is described, that target β-catenin and inhibit its interaction with transcription factors of the TCF/LEF family of transcription factors. Based on the known structure of β-catenin in complex with the protein E-cadherin, a macrocyclic binder of β-catenin was developed which comprises a short antiparallel β sheet. A crystal structure of the macrocycle bound to β-catenin was obtained supporting the design of a library of bicyclic peptidomimetics. Among those mimetics, a bicycle (A-b6) was identified that inhibits the Wnt signaling pathway in a cell-based assay and shows cellular uptake comparable to the cell penetrating Tat peptide.

The invention therefore provides a cyclic peptide comprising the amino acid sequence N-terminal-X₄X₃X₂X₁-a-Y₁Y₂Y₃Y₄-C-terminal, wherein -a- represents a linker, and wherein the peptide comprises a linker -c- between the N-terminal amino acid and C-terminal amino acid, and/or wherein the two amino acids of one or two of the following amino acid pairs form a side chain-to-side chain bridged di-amino acid forming a bridge -b-: X₁ and Y₁; X₂ and Y₂; X₃ and Y₃; and X₄ and Y₄; and wherein the amino acids that are not bridged are as follows: X₄ is a positively charged or polar amino acid (aa); X₃ can be any aa; X₂ is a negatively charged aa or an amide; X₁ is a hydrophobic aa; Y₁ is a is a negatively charged aa, or an amide; Y₂ can be any aa; Y₃ is a hydrophobic aa; and Y₄ is a hydrophobic aa.

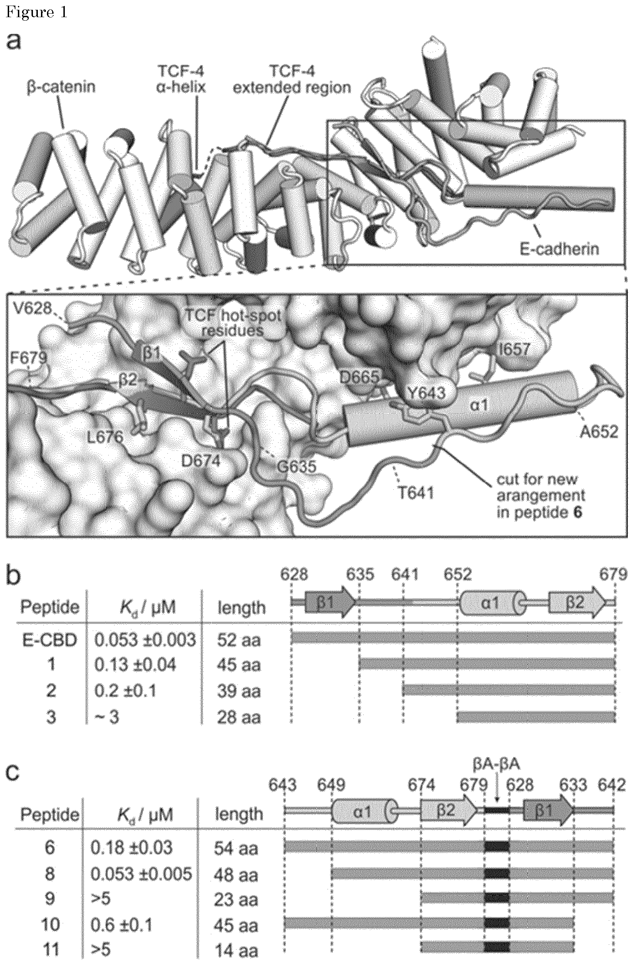

Due to its involvement in the onset and progression of numerous types of cancers, hyperactive Wnt signaling has moved into the focus of drug discovery efforts. In an oncogenic context, Wnt signaling is often activated via mutations in central pathway regulators. For that reason, targeting the downstream β-catenin-containing transcriptional activator complex has been pursued. Transcription factors of the TCF/LEF family recognize the armadillo repeat domain of β-catenin via an elongated peptide sequence (Figure 1a) with two central hot spot residues crucially contributing to binding. Previous structure-based efforts to generate ligands targeting the corresponding site on β-catenin (binding site 3) [Schneider et al., 2012. Proc Natl Acad Sci USA 109: 17942-17947; Wang et al., 2019. J Med Chem 62: 3617-3635] have not resulted in therapeutically useful inhibitors. Herein, we took a novel approach towards the generation of ligands for β-catenin's binding site 3 using the CBD of E-cadherin as a starting point. We noticed that the CBD of E cadherin binds to site 3 via an antiparallel β-sheet which may serve as the basis for inhibitor design. However, while the 52-mer E cadherin epitope (E-CBD) showed high affinity for β-catenin (Kd = 53 nM), we were not able to identify a considerably truncated version with sufficient affinity (e.g. 14-mer 11, Kd > 5 µM). Surprisingly, cyclization of 14 mer peptide 11 using the DP-LP turn motif, however, resulted in a molecule with greatly improved affinity (12, Kd = 156 nM).

Said cyclic peptide may further comprise one or more of X₅ or X₆X₅ N-terminally, and/or Y₅ or Y₅Y₆ C-terminally, wherein X₆ can be any aa; X₅ can be any aa; Y₅ is a hydrophobic aa, and Y₆ is an aromatic aa, and/or, wherein the two amino acids of one or two of the following amino acid pairs form a side chain-to-side chain bridged di-amino acid forming a bridge -b-: X₁ and Y₁; X₂ and Y₂; X₃ and Y₃; X₄ and Y₄; X₅ and Y₅; and X₆ and Y₆.

In a preferred cyclic peptide according to the invention, the linker -a- is a turn mimetic. Said linker -a- preferably is selected from a dipeptide Dextrorotatory-aa (Daa)- Levorotatory aa (Laa), wherein aa refers to any amino acid, optionally wherein one or both aa are N-methylated; a dipeptide aa-Gly or aa-Pro, wherein aa refers to any amino acid, optionally wherein one or both aa are N-methylated; and a non-alpha amino acid. Said linker -a- is more preferably selected from DPro-LPro, DPro-LLeu, DPro-LGly, DPro-LAla, LAsn-Gly, Aib-Gly, Aib-DPro, L-ornithine, 5-aminovaleric acid, dibenzofuran, and benzodiazepine.

In a preferred cyclic peptide according to the invention, linker -c- is selected from a dipeptide Dextrorotatory-aa (Daa)- Levorotatory aa (Laa), wherein aa refers to any amino acid, optionally wherein one or both aa are N-methylated; a dipeptide aa-Gly or aa-Pro, wherein aa refers to any amino acid, optionally wherein one or both aa are N-methylated; a non-alpha amino acid; and a connector comprising 7-12 atoms connecting the N-terminal and C-terminal amino acids of the peptide comprising the amino acid sequence N-terminal-X₄X₃X₂X₁-a-Y₁Y₂Y₃Y₄-C-terminal. Said linker -c- preferably is selected from DPro-LPro, DPro-LLeu, DPro-LGly, DPro-LAla, DPhe-LPro, DPhe-LPro LAsn-Gly, Aib-Gly, Aib-DPro, DPhe-LPro, L-ornithine, 5-aminovaleric acid, dibenzofuran, benzodiazepine, 1-4 beta-alanines, and a PEG based amino acid. Said linker -c- preferably is selected from the following structures:

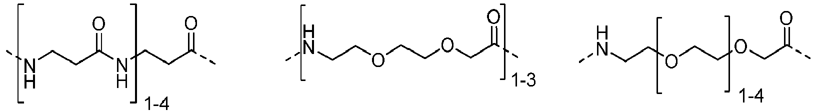

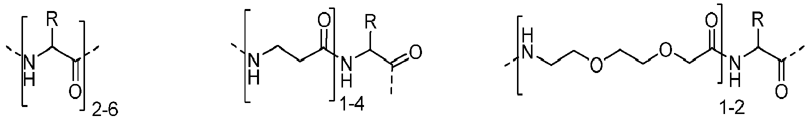

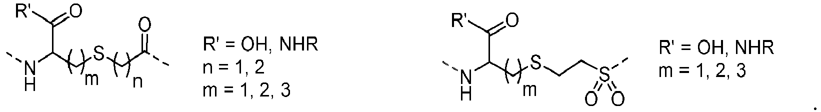

In a preferred cyclic peptide of the invention, the one or two side chain-to-side chain bridged di-amino acids forming one or two bridges -b- have the following structure: 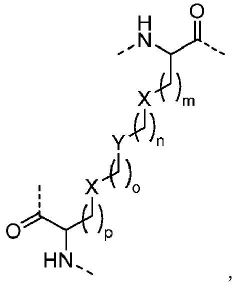 wherein m, n, o and p are each independently selected from 0, 1, 2 or 3, each X is independently CH₂, S, O or NH, and Y is absent or selected from the following structures:

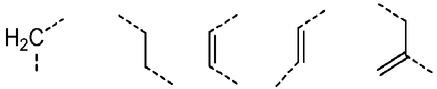

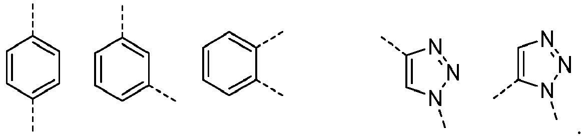

Macrocycle 12 was co-crystalized with β-catenin providing a crystal structure that confirms the expected binding site 3 and an antiparallel β-sheet arrangement of 12. This is the first crystal structure of a synthetic ligand bound to this site of β-catenin. In fact, only two crystal structures of synthetic ligands bound to β-catenin had been reported so far [Grossmann et al., 2012. Proc Natl Acad Sci USA 109: 17942-17947; Kessler et al., 2021. ChemMedChem 16: doi.org/10.1002/cmdc.20200083931]. Based on the structure of 12 bound to β-catenin, a focused library of bicyclic inhibitors was synthesized applying a late-stage diversification strategy. The library was obtained by reacting unprotected monocyclic peptides bearing two cross-strand cysteines with seven different bis-electrophiles. Three distinct monocyclic precursors were used providing a total of 21 thioether bridged bicycles. A competition assay using β-catenin and the CBD of TCF-4 as tracer, revealed diminished inhibitory activity for macrocyclic double-cysteine variants while the installation of four-carbon atom bridges restored inhibitory activity for two of the three scaffolds (A-b5/ A-b6/ A-b7 and C-b5/ C-b7). Best performing library members showed affinities in the range of the 52-mer starting sequence (E-CBD) which highlights the impact of conformational constrain on binding.

Said one or two bridges -b- are preferably independently selected from a structure containing one or more thioether bonds and the following structures: 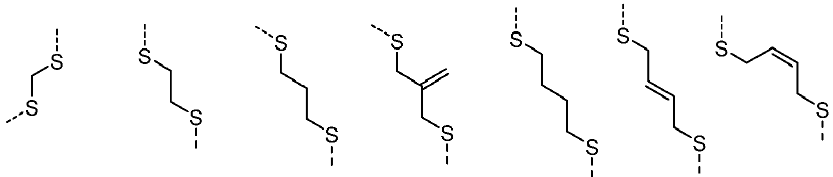 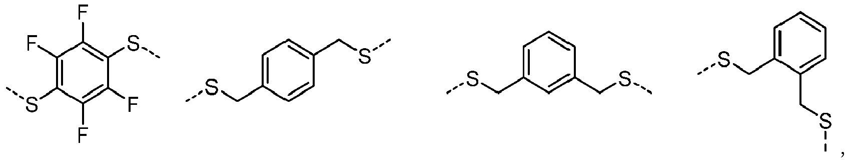 or the one or two side chain-to-side chain bridged di-amino acids forming a bridge -bare independently selected from the following structures:

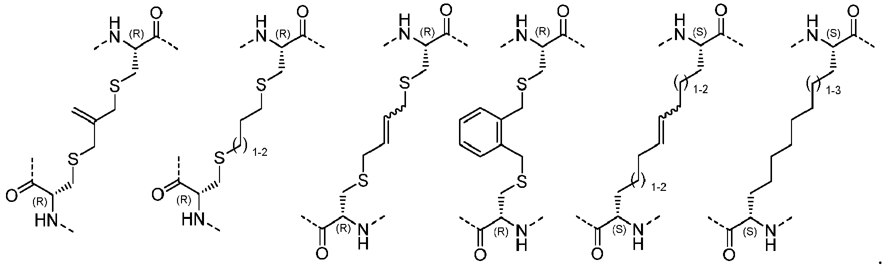

In a preferred cyclic peptide according to the invention, the amino acids that are not bridged are as follows: X₁ is selected from Val, Leu and Ile, or corresponding non-natural amino acids; X₂ is selected from Asp, Glu, Asn, and Gln, or corresponding non-natural amino acids; X₄ is Arg; Y₁ is selected from Asp, Glu, Asn, and Gln, or corresponding non-natural amino acids; Y₃ is selected from Val, Leu, Ile and Cys, or corresponding non-natural amino acids; Y₄ is selected from Val, Leu, Ile, and Cys, or corresponding non-natural amino acids; Y₅ is selected from Val, Leu, Ile, and Cys, or corresponding non-natural amino acids; and Y₆ is selected from Phe, Tyr, His and Trp, or corresponding non-natural amino acids.

The invention further provides a cyclic peptide according to the invention, for use as a medicament.

Said medicament preferably is for treating a disease selected from osteoporosis, a chronic lung disease, and a psychiatric disorder.

A cyclic peptide according to the invention preferably is for use in a method of preventing a pre-malignant condition in an individual to become cancerous, said pre-malignant condition preferably selected from colorectal adenoma and Barrett's esophagus, or for use in a method of treating an individual suffering from a tumor, wherein the tumor preferably is selected from a carcinoma such as a colorectal carcinoma, esophageal adenocarcinoma, pancreatic ductal adenocarcinoma and breast carcinoma.

The invention further provides an use of the cyclic peptide according to any one of claims 1-11, for inhibiting Wingless/integrase-1 (Wnt)-signaling in isolated tissues or cells.

### FIGURE LEGENDS

Figure 1. (a) Top: Crystal structure (PDB ID 2gl7) of β-catenin in complex with the CBD of TCF-4 and superimposed with the CBD of E-cadherin (PDB ID 1i7x, chain B). Bottom: Close-up of indicated region with β-catenin in surface representation. Hot-spot amino acids of E-CBD (D665, 1657, Y643, D674, L676) and TCF (D16, E17) are shown as stick. Terminal amino acids of peptides E-CBD, 1, 2 and 3 are indicated. Position where sequence of E-CBD was opened to generate peptide 6 is highlighted with a black bar. (b) Peptide affinities and lengths are shown including the visualization of involved peptide regions. Kd-values were obtained from FP measurements (n = 3, ± std. error). (c) Table shows peptides derived from 6. The β-alanine crosslink (βA-βA) is highlighted in black. Measurements in analogy to Figure 1b. Peptide sequences can be found in Table 1.

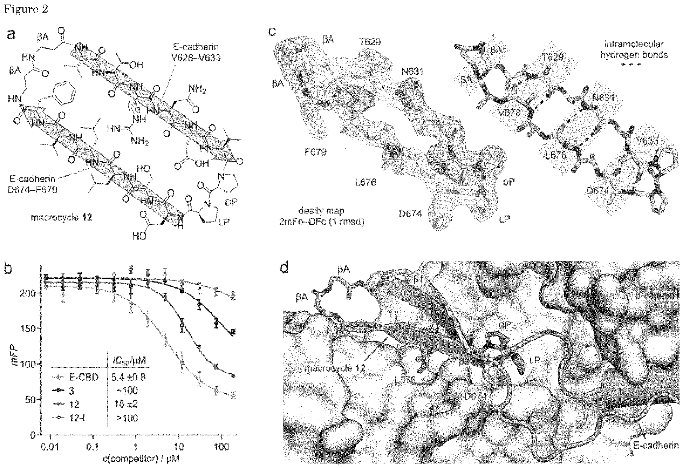

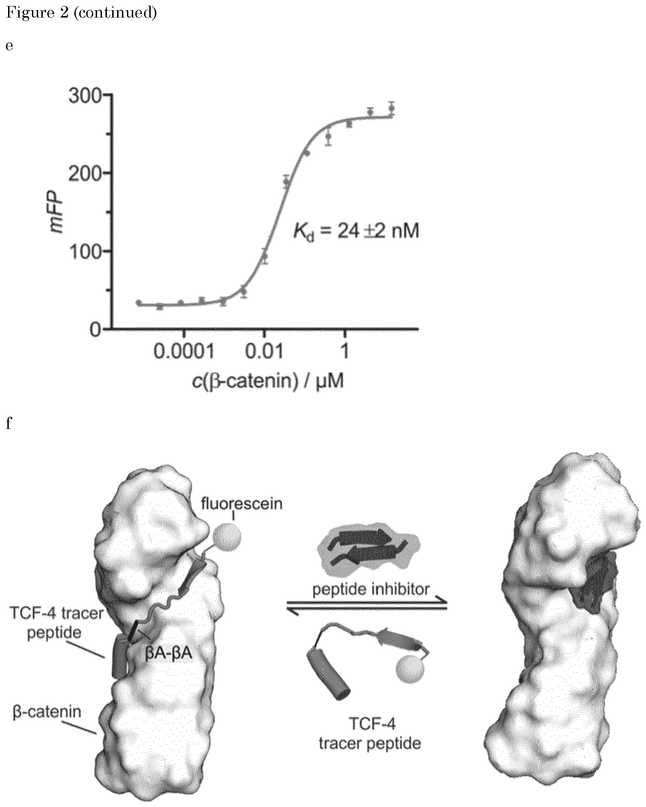

Figure 2. (a) Chemical structure of the macrocycle 12. Amino acids of β-strands β1 and β2 are highlighted. (b) FP competition assay with TCF-4-derived tracer peptide (c = 10 nM) and full-length β-catenin (c = 250 nM. Competitor concentration was varied (c = 7.9·10-3-188 µM). Peptide sequences can be found in the Tables 1 and 2. (n = 3, ± std. error) (c) Left: Electron density map (2mFo-DFc, contoured at 1 rmsd) of cyclic peptide inhibitor 12 (PDB ID 7ar4). Right: Stick representation of peptide 12 when bound to β-catenin, showing backbone atoms and Cβ-atoms (PDB ID 7ar4). Intramolecular hydrogen bonds are indicated with dashed lines. (d) Crystal structure of β-catenin (surface representation) in complex with peptide 12 (cartoon representation, PDB ID 7ar4). The hot spot residues as well as βA βA and the DP-LP turn are shown as stick representation. 12 is superimposed with the CBD of E-cadherin, cartoon representation, PDB ID 1i7x). Residues L676 and D674 are shown explicitly. (e) Direct FP measurement of TCF-4 tracer peptide FI-PEG2-D-E-L-I-S-F-K-D-EG-E-Q-E-βA-βA-E-R-D-L-A-DV-K-S-S-L-V-N. Measurement was performed with a constant TCF-4 tracer concentration (10 nM), while β-catenin was titrated (7.5 · 10⁻⁶ - 14.1 µM). Sequence of TCF-4 tracer peptide is shown (Fl: fluorescein, PEG2: 2-[2-(2-aminoethoxy)ethoxy]acetic acid, βA: β alanine). (f) Concept of the FP competition experiment with β-catenin (surface representation) and fluorescently labeled TCF-4 tracer peptide (cartoon representation). Inhibitory peptide is shown in blue (surface and cartoon representation) and displaces the TCF-4 tracer peptide.

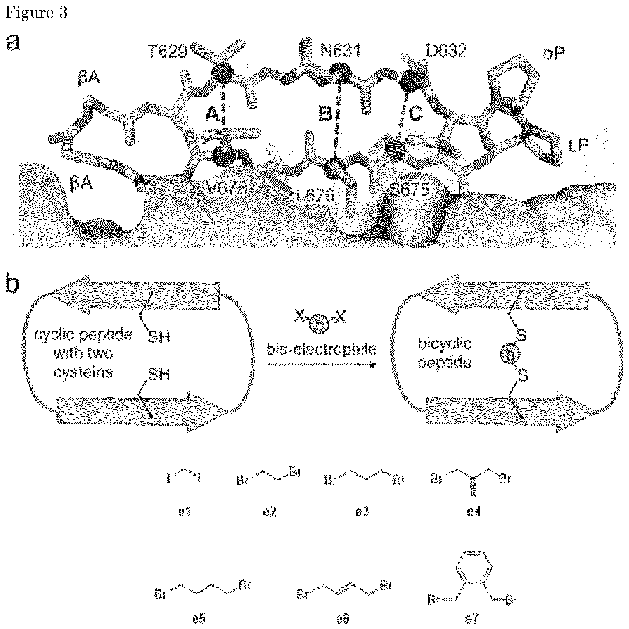

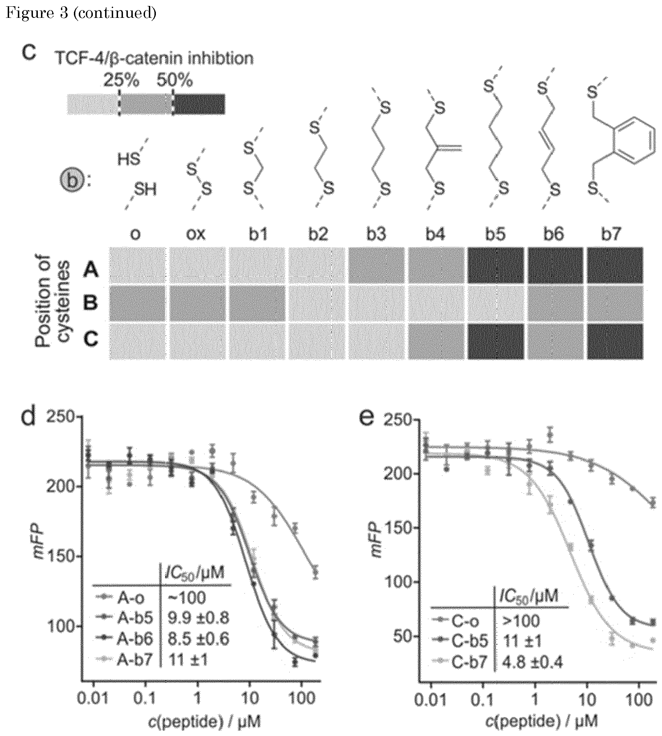

Figure 3: (a) Crystal structure of 12 bound to β-catenin (PDB ID 7ar4) highlighting positions in 12 selected for cross-strand bridges (A-C). Ca-atoms of the corresponding amino acids are highlighted. (b) Bicyclization of monocyclic 12 derivatives bearing two cysteines at cross-strand positions. Bridging involves reaction with bis-electrophiles (e1-e7). Sequences of bicycles can be found in Table 2. (c) Chemical structures of bridges b1-7, which have been introduced within macrocycles A-o, B-o, and C-o, are shown (derived from 12 by introduction of two cysteines at positions A, B and C, respectively). Heat map depicts ability of resulting compounds to compete with the TCF-4/β-catenin interaction. Data is based on an FP competition assay in analogy to Figure 2b but employing a single competitor concentration (c = 30 µM, 0% inhibition = absence of competitor, 100% inhibition = 30 µM E-CBD (lower plateau, Figure 2b. (d) Concentration-dependent inhibition in FP competition assay (see Figure 2(e) of A-b5, A b6 and A-b7 including their monocyclic precursor A-o (c = 7.9 · 10⁻³-188 µM, n = 3, ± std. error). Constant concentration of TCF-4 tracer peptide (10 nM) and β-catenin (250 nM) was employed. (e) Concentration-dependent inhibition in FP competition assay (see Figure 2e) of C-b5 and C-b7 including their monocyclic precursor C-o (c = 7.9 · 10⁻³-188 µM, n = 3, ± std. error). Constant concentration of TCF-4 tracer peptide (10 nM) and β-catenin (250 nM) was employed.

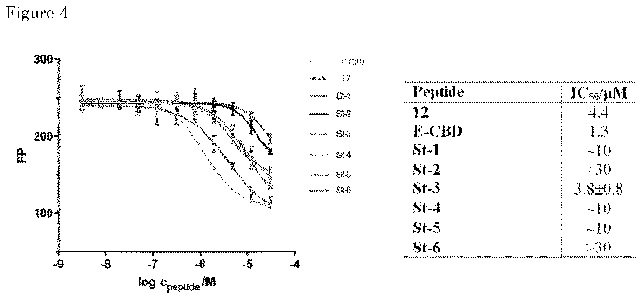

Figure 4: Competition of fluorescently labeled E-Cadherin tracer peptide (E-CBD) with β-catenin. St-1 - St-6 were titrated to determine IC₅₀-values.

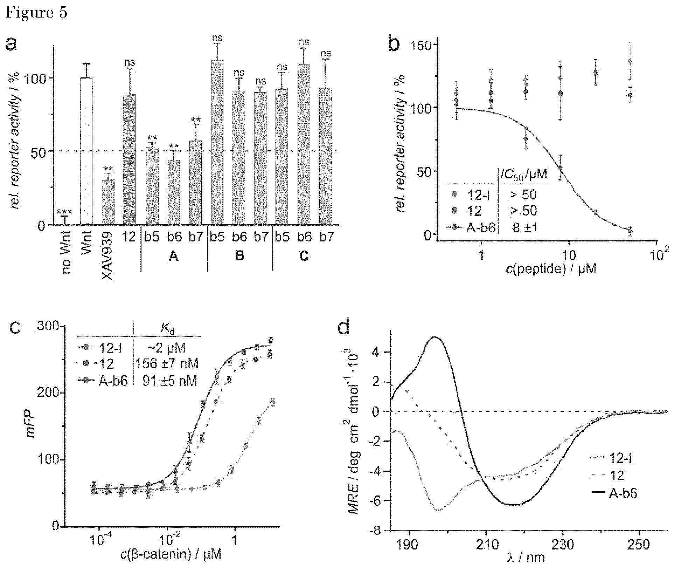

Figure 5: (a) TOPFLSH reporter gene assay in HEK293T cells testing inhibition of the Wnt pathway for macrocycle 12 as well as selected bicycles from series A, B and C (n = 3, error shows std. dev., ns: p > 0.05, *: p < 0.05, **: p < 0.01, ***: p < 0.001). Wnt signaling was activated with Wnt-3a (c = 100 ng/mL). Cells were incubated with compound (c = 20 µM, t = 24 h), before luciferase activity was measured. (b) Concentration-dependent effect on reporter activity for 12-1, 12, A-b6 (c = 0.512-50 µM, incubation t = 24 h, n = 3, ± std. error). (c) FP titration measurements of the fluorescein-labeled analogues of 12-1, 12 and A-b6 (c = 10 nM, (Tables 1 and 2) and β-catenin (7 · 10⁻⁵-12.6 µM, n = 3, ± std. error). (d) CD spectra of linear 12-1, cyclic 12 and bicyclic A-b6 (c = 75 µM) in sodium phosphate buffer (5 mM, pH 7.5).

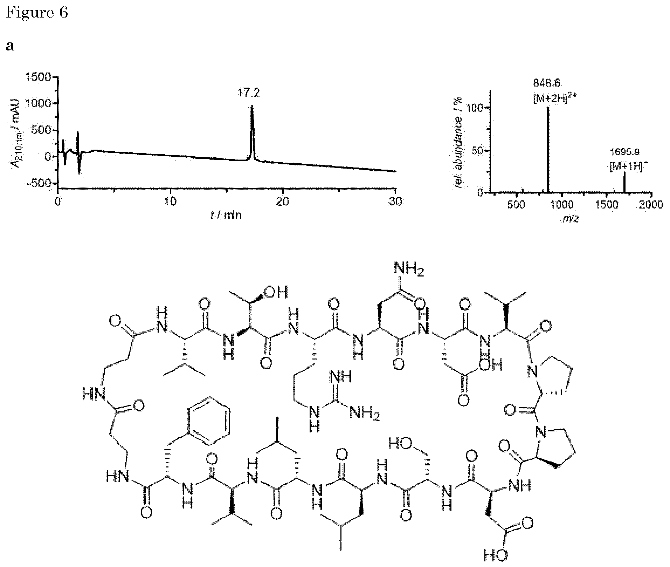

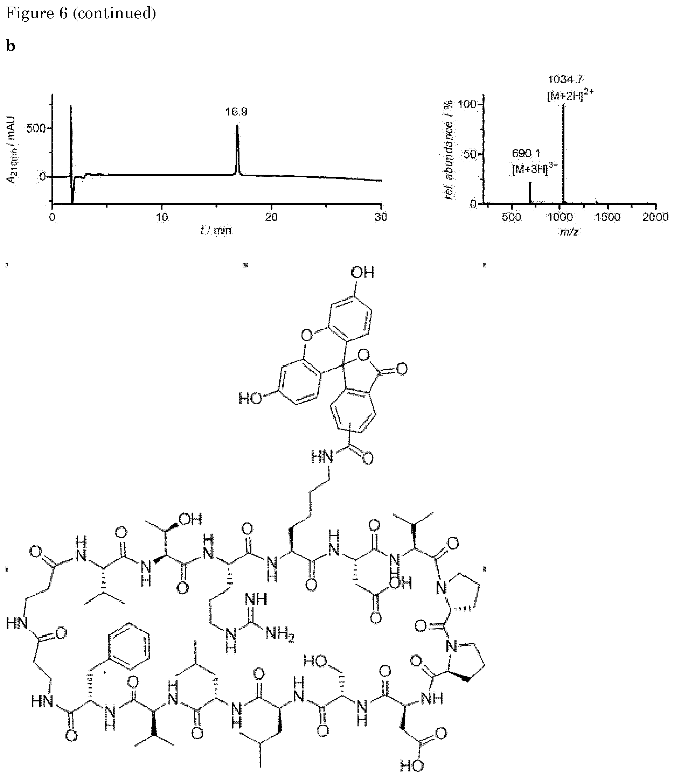

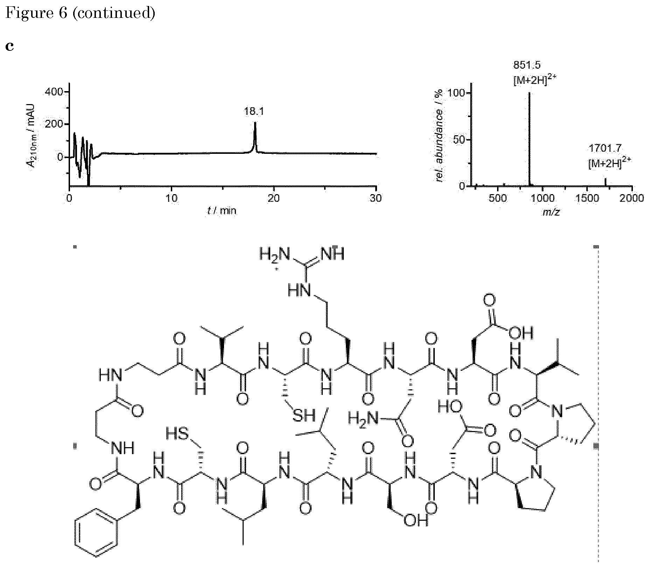

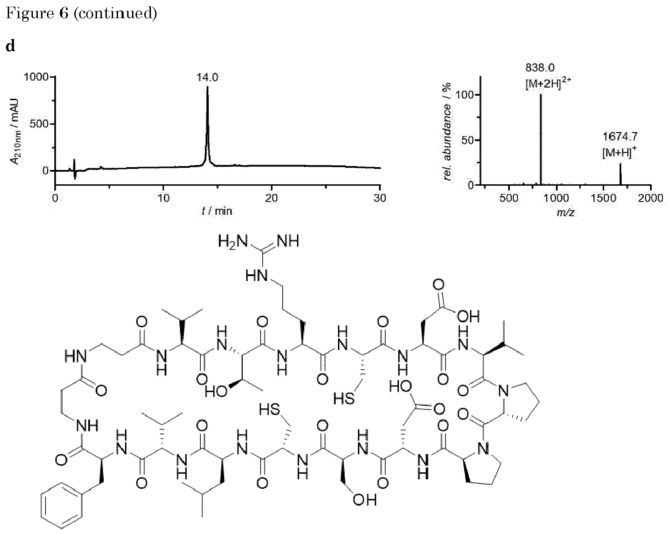

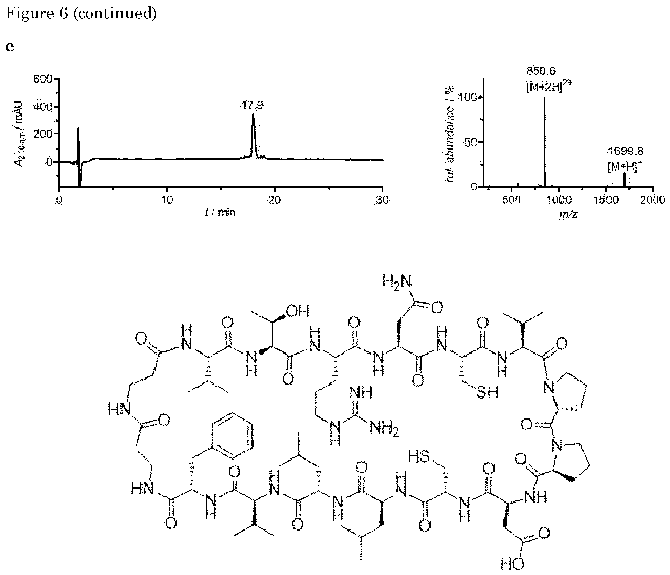

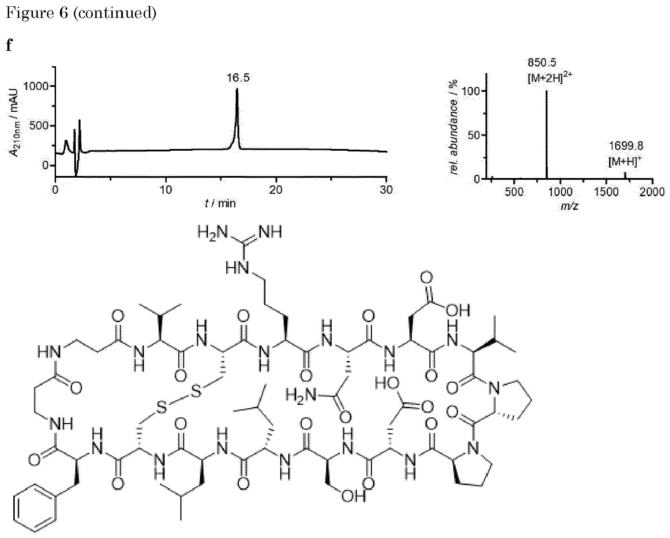

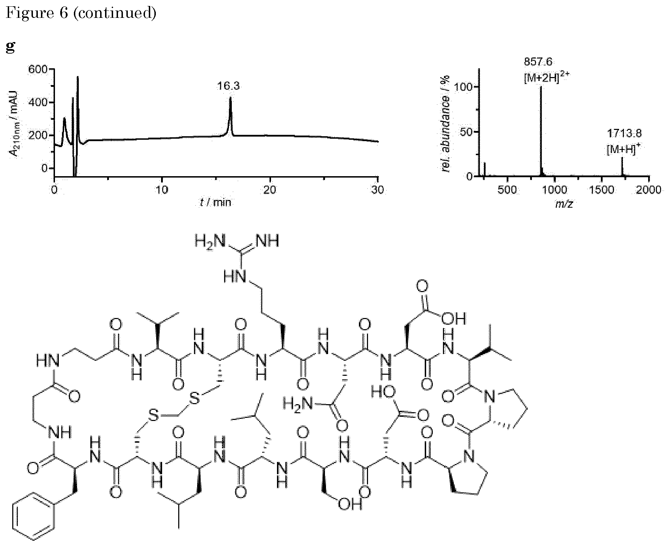

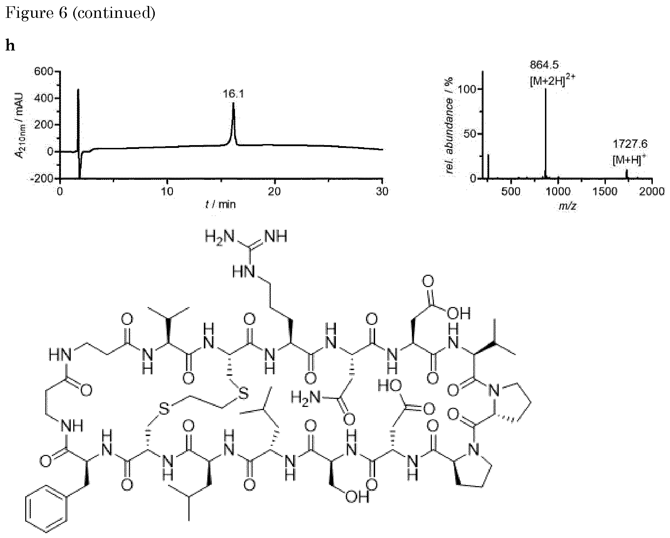

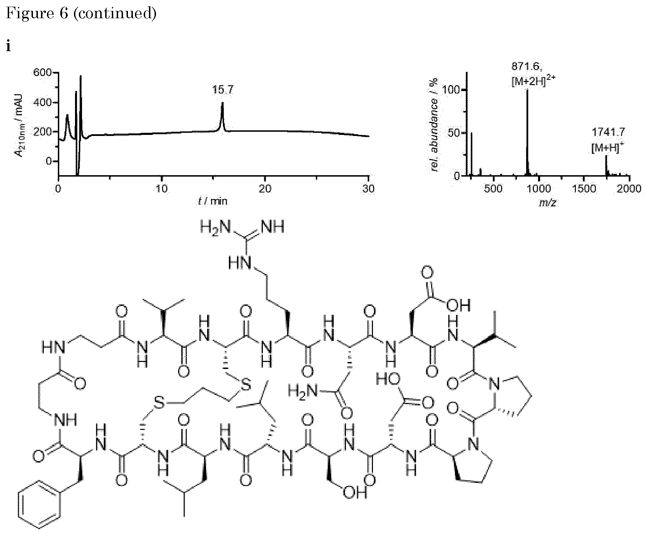

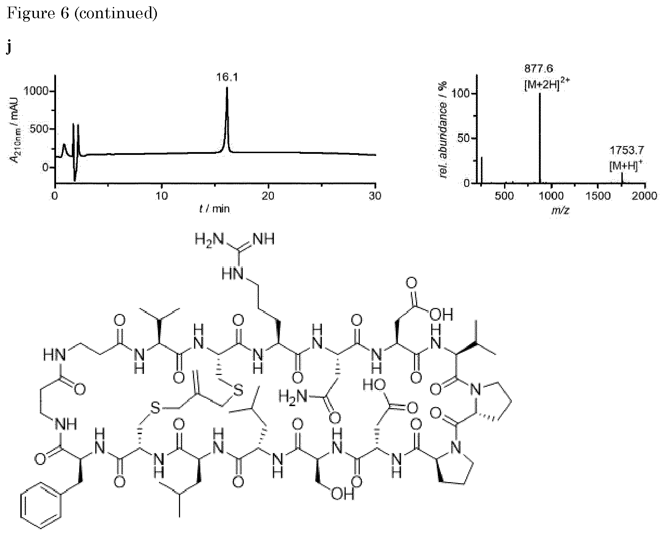

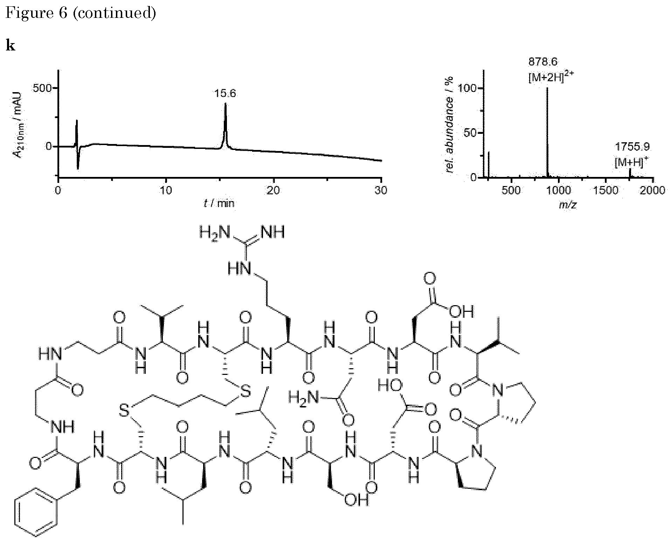

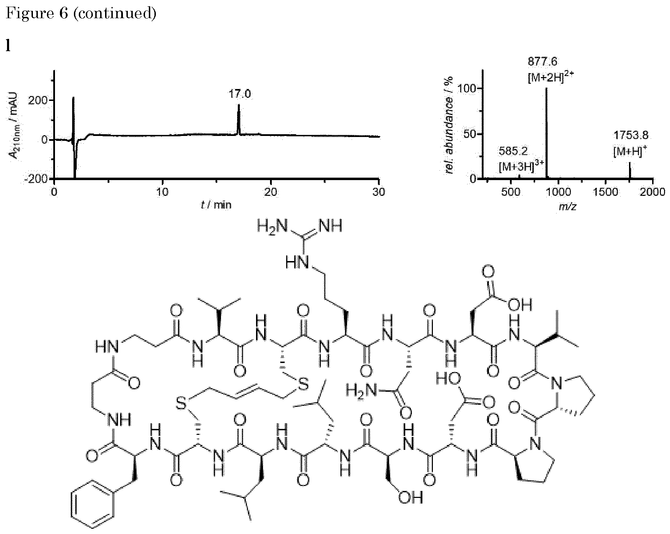

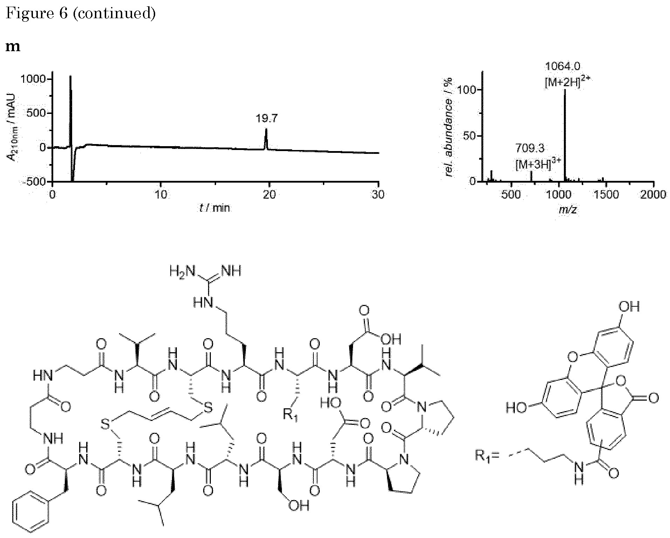

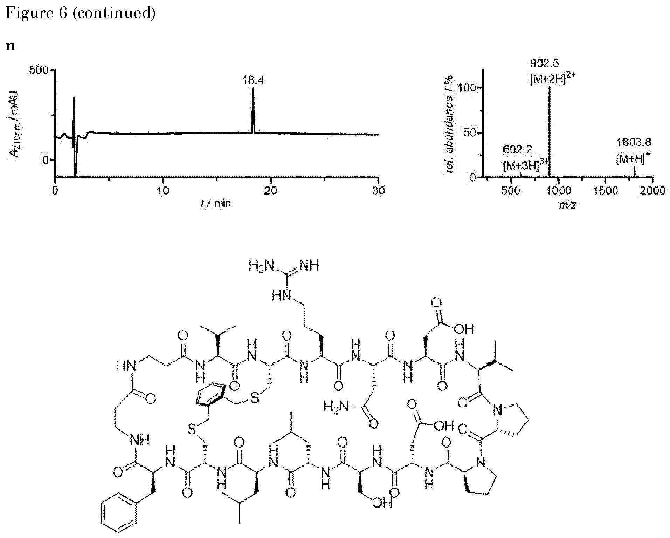

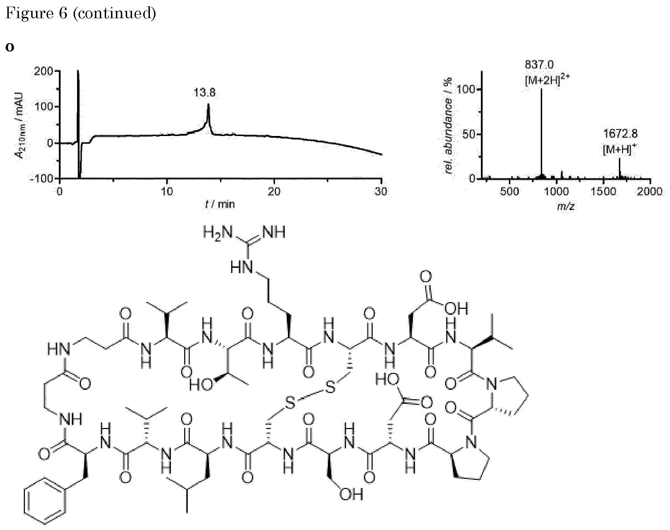

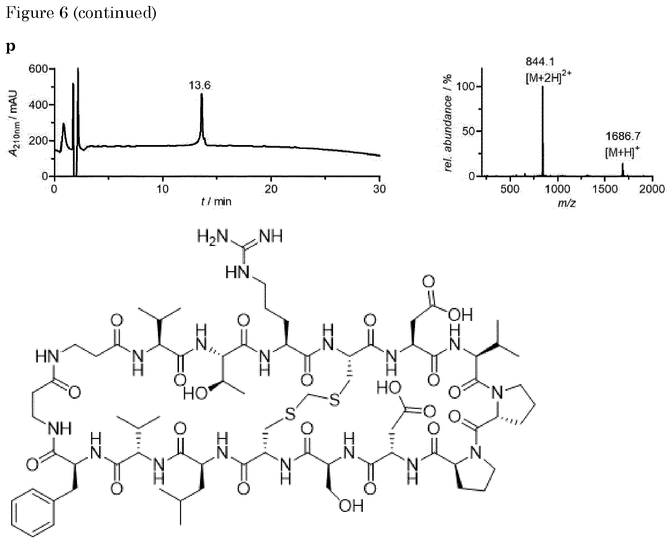

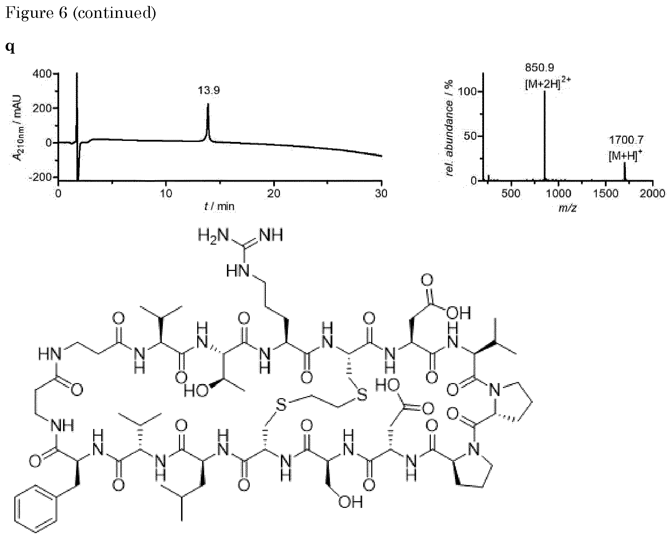

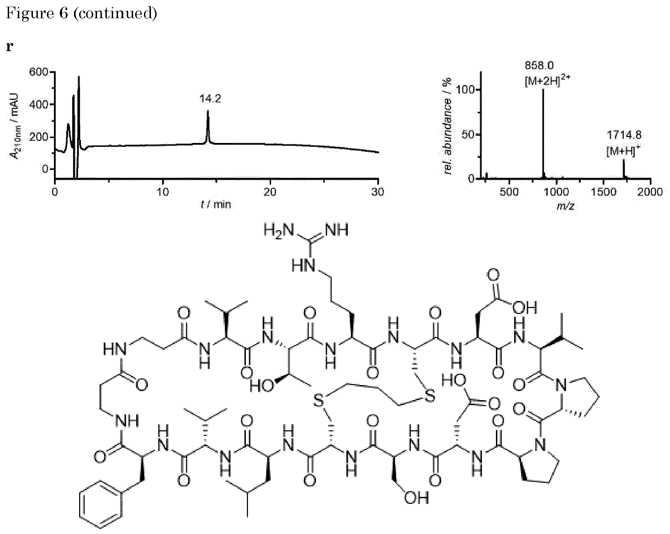

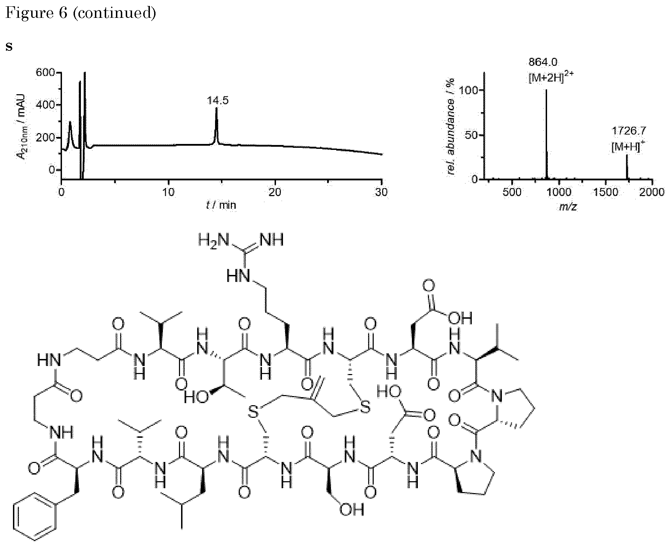

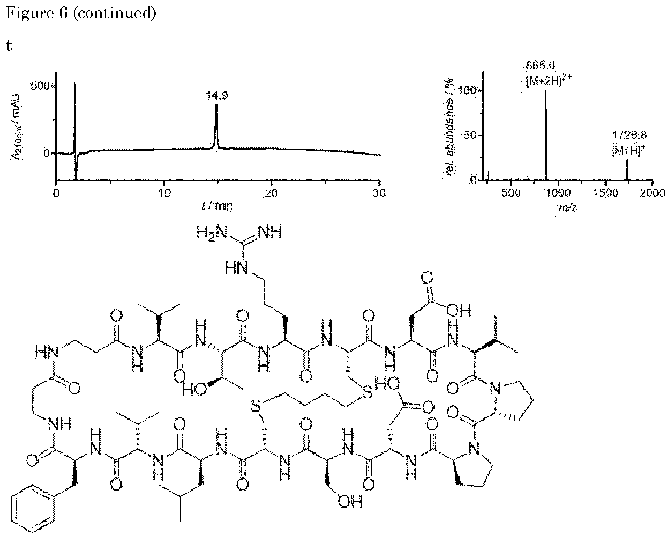

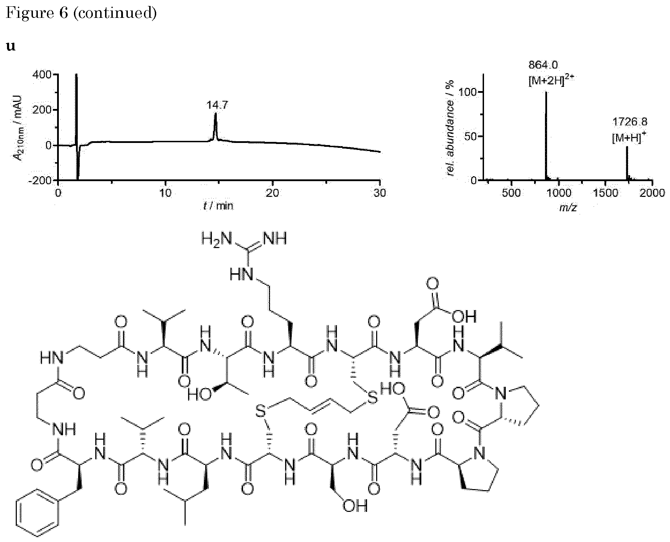

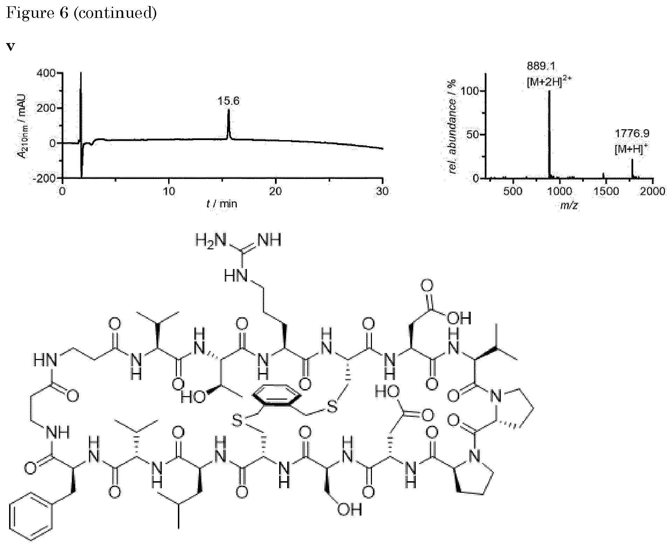

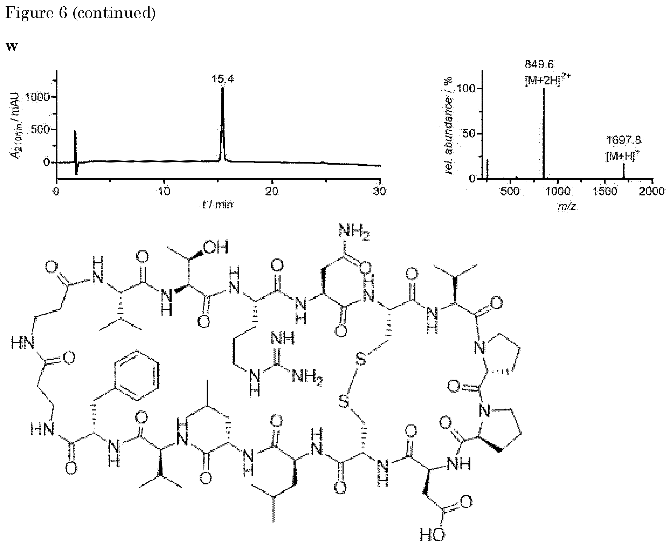

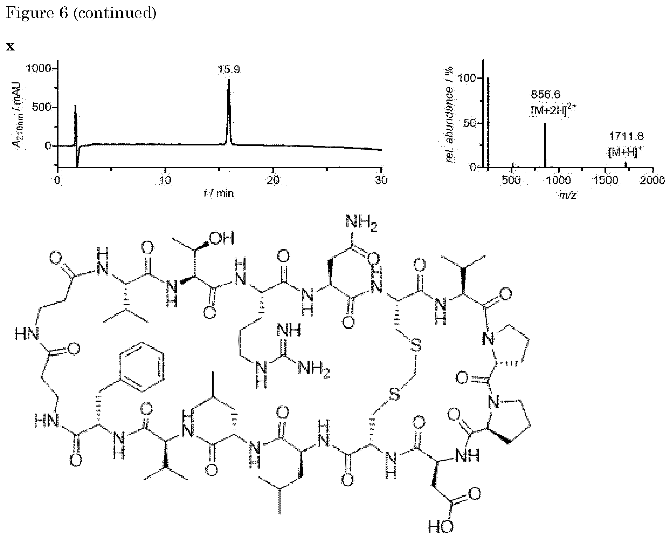

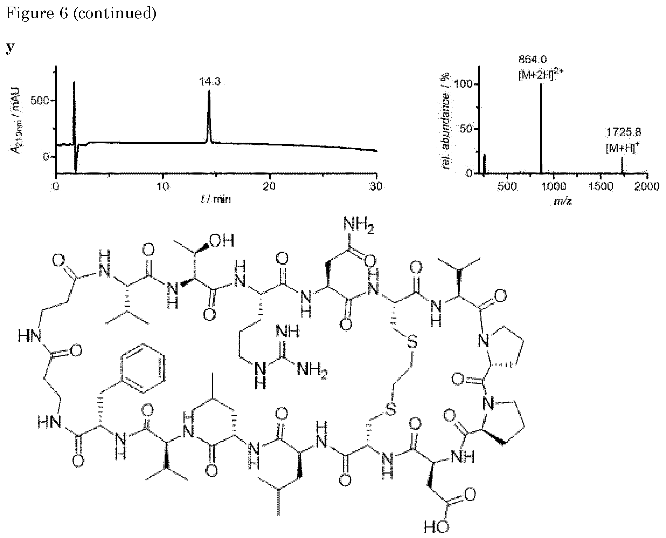

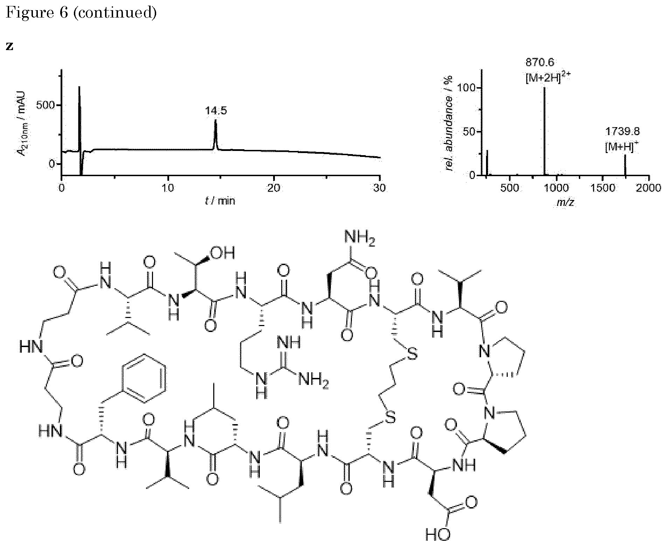

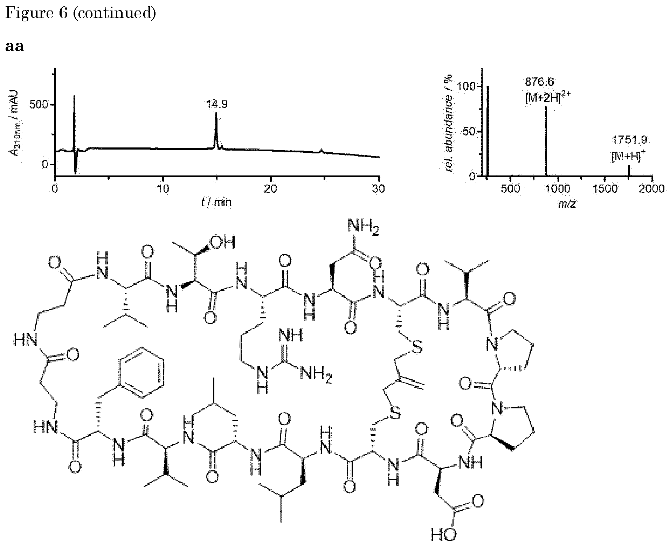

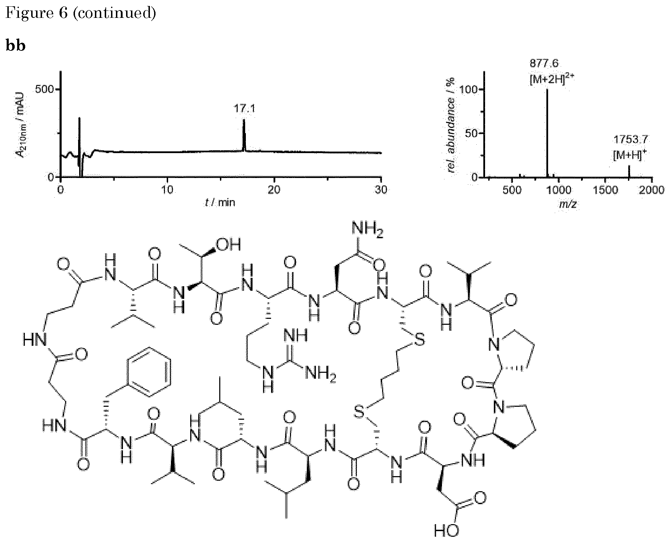

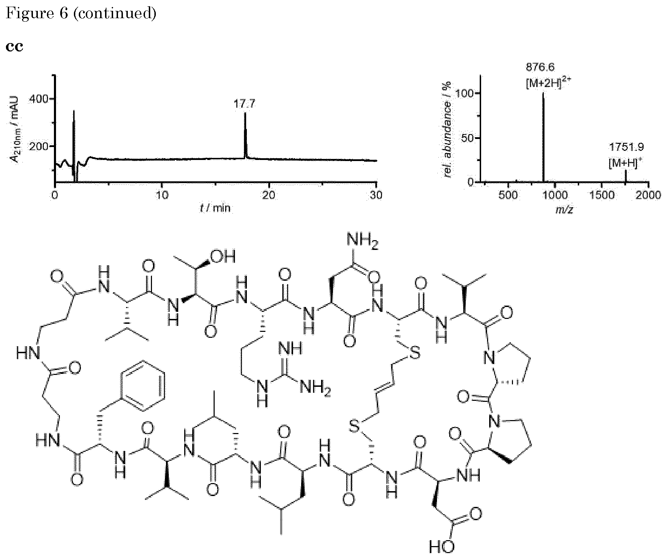

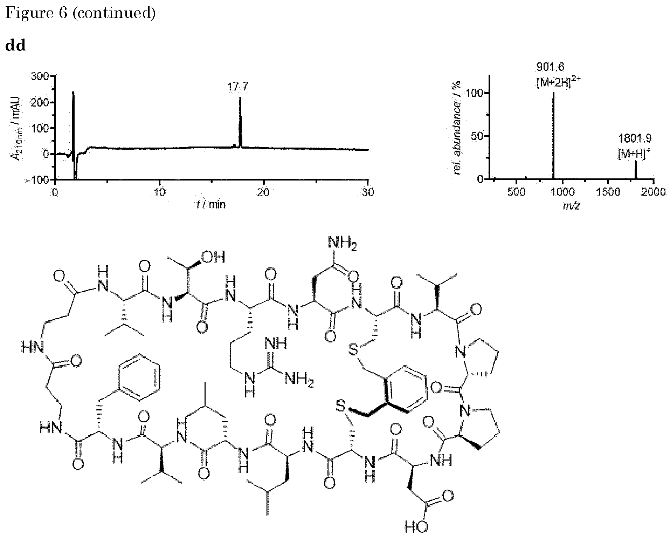

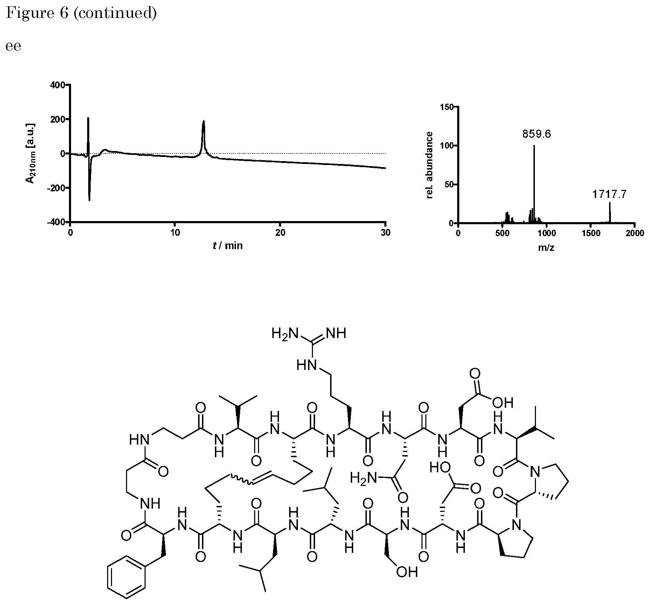

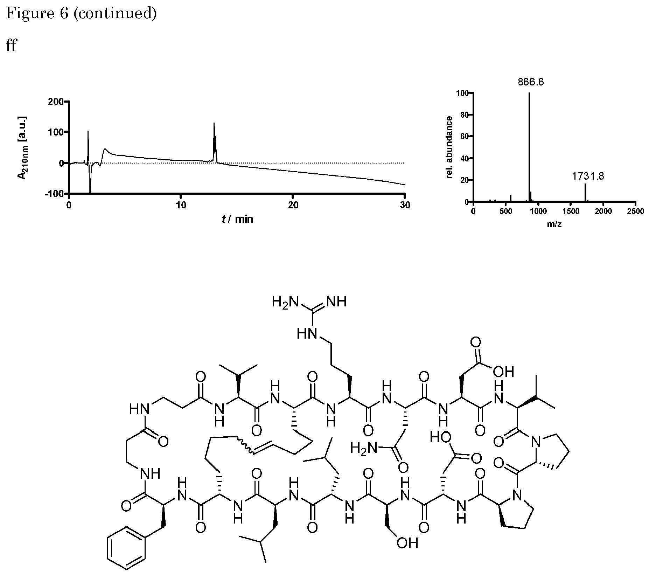

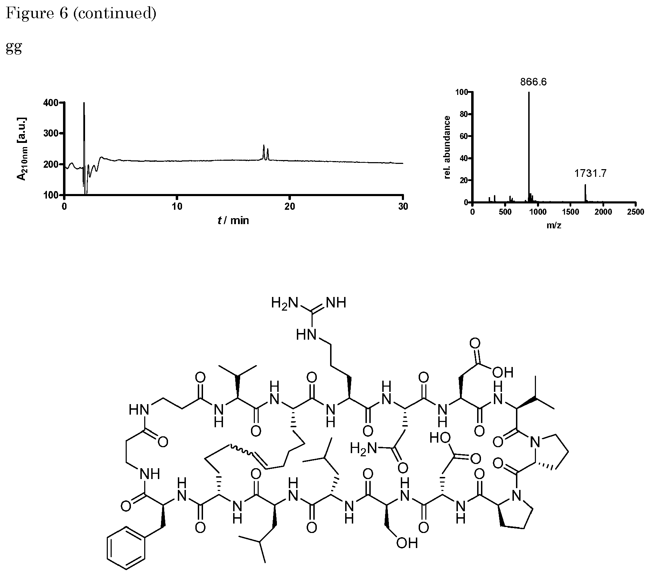

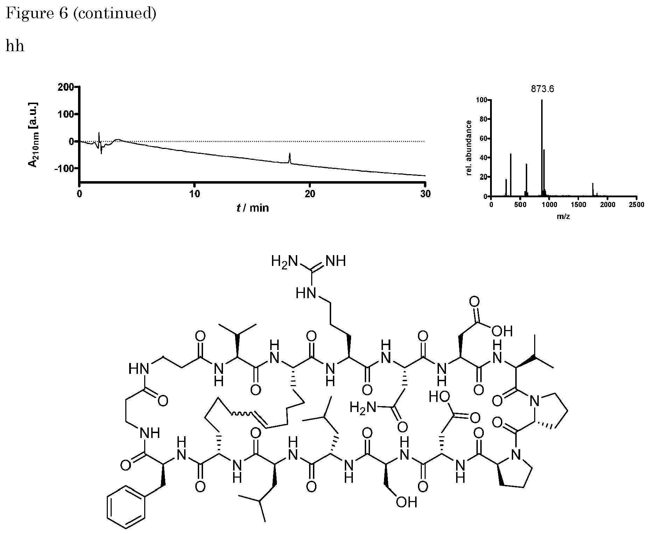

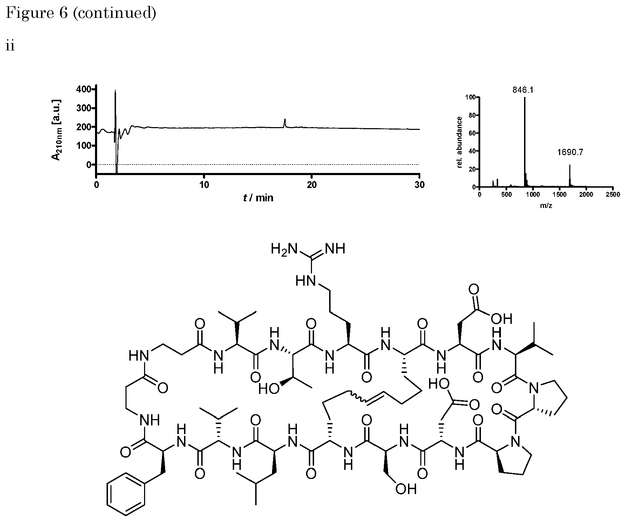

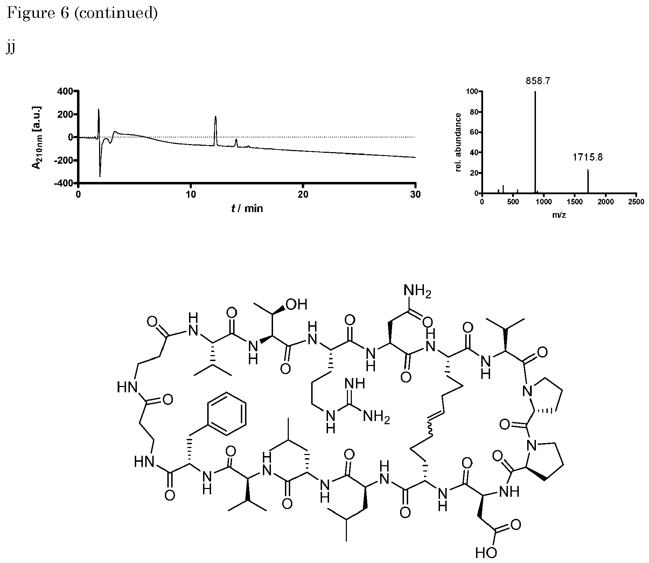

Figure 6. (a) Peptide 12. Chemical formula: C₇₇H₁₂₂N₂₀O₂₃. Molecular weight 1695.94. [βA-V-T-R-N-D-V-p-P-D-S-L-L-V-F-βA]. HPLC chromatogram: 5 - 65% in 30 min solvent D (ACN + 0.1% FA + 0.01% TFA), solvent C (H₂O + 0.1% FA + 0.01% TFA), MS spectrum (positive mode), and structural formula. (b) Peptide 12^{K631-Fl}. Chemical formula: C₁₀₀H₁₃₈N₂₀O₂₈. Molecular weight 12068.32. [βA-V-T-R-K(FI)-D-V-p-P-D-S-L-L-V-F-βA]. HPLC chromatogram: 10 - 75% in 30 min solvent B (ACN + 0.1% TFA), solvent A (H₂O + 0.1% TFA), MS spectrum (positive mode), and structural formula. (c) Peptide A-o. Chemical formula: C₇₄H₁₁₆N₂₀O₂₂S₂. Molecular weight 1701.98. [βA-V-C-R-N-D-V-p-P-D-S-L-L-C-F-βA]. HPLC chromatogram: 5 - 65% in 30 min solvent D (ACN + 0.1% FA + 0.01% TFA), solvent C (H₂O + 0.1% FA + 0.01% TFA), MS spectrum (positive mode), and structural formula. (d) Peptide B-o. Chemical formula: C₇₃H₁₁₅N₁₉O₂₂S₂. Molecular weight 1674.95. [βA-V-T-R-C-D-V-p-P-D-S-C-L-V-F-βA]. HPLC chromatogram: 10 - 75% in 30 min solvent B (ACN + 0.1% TFA), solvent A (H₂O + 0.1% TFA) and MS spectrum (positive mode), and structural formula. (e) Peptide C-o. Chemical formula: C₇₆H₁₂₂N₂₀O₂₀S₂. Molecular weight 1700.05. [βA-V-T-R-N-C-V-p-P-D-C-L-L-V-F-βA]. HPLC chromatogram: 5 - 65% in 30 min solvent D (ACN + 0.1% FA + 0.01% TFA), solvent C (H₂O + 0.1% FA + 0.01% TFA) and MS spectrum (positive mode), and structural formula. (f) Peptide A-ox. Chemical formula: C₇₄H₁₁₄N₂₀O₂₂S₂. Molecular weight 1699.96. [βA-V-[C-R-N-D-V-p-P-D-S-L-L-C]-F-βA]. HPLC chromatogram: 10 - 75% in 30 min solvent B (ACN + 0.1% TFA), solvent A (H₂O + 0.1% TFA) and MS spectrum (positive mode), and structural formula. (g) Peptide A-b1. Chemical formula: C₇₅H₁₁₆N₂₀O₂₂S₂. Molecular weight 1713.99. [βA-V-[C-R-N-D-V-p-P-D-S-L-L-C]-F-βA] /b1. HPLC chromatogram: 10-75% in 30 min solvent B (ACN + 0.1% TFA), solvent A (H₂O + 0.1% TFA) and MS spectrum (positive mode), and structural formula. (h) Peptide A-b2. Chemical formula: C₇₆H₁₁₈N₂₀O₂₂S₂. Molecular weight 1728.02. [βA-V-[C-R-N-D-V-p-P-D-S-L-L-C]-F-βA] / b2. HPLC chromatogram: 10 - 75% in 30 min solvent B (ACN + 0.1% TFA), solvent A (H₂O + 0.1% TFA) and MS spectrum (positive mode), and structural formula. (i) Peptide A-b3. Chemical formula: C₇₇H₁₂₀N₂₀O₂₂S₂. Molecular weight 1742.05. [βA-V-[C-R-N-D-V-p-P-D-S-L-L-C]-F-βA] / b3. HPLC chromatogram: 10 - 75% in 30 min solvent B (ACN + 0.1% TFA), solvent A (H₂O + 0.1% TFA) and MS spectrum (positive mode), and structural formula. (j) Peptide A-b4. Chemical formula: C₇₈H₁₂₀N₂₀O₂₂S₂. Molecular weight 1754.06. [βA-V-[C-R-N-D-V-p-P-D-S-L-L-C]-F-βA] / b4. HPLC chromatogram: 10 - 75% in 30 min solvent B (ACN + 0.1% TFA), solvent A (H₂O + 0.1% TFA) and MS spectrum (positive mode), and structural formula. (k) Peptide A-b5. Chemical formula: C₇₈H₁₂₂N₂₀O₂₂S₂. Molecular weight 1756.07. [βA-V-[C-R-N-D-V-p-P-D-S-L-L-C]-F-βA] / b5. HPLC chromatogram: 10 - 75% in 30 min solvent B (ACN + 0.1% TFA), solvent A (H₂O + 0.1% TFA) and MS spectrum (positive mode), and structural formula. (1) Peptide A-b6. Chemical formula: C₇₈H₁₂₀N₂₀O₂₂S₂. Molecular weight 1754.06. [βA-V-[C-R-N-D-V-p-P-D-S-L-L-C]-F-βA] / b6. HPLC chromatogram: 5 - 65% in 30 min solvent D (ACN + 0.1% FA + 0.01% TFA), solvent C (H₂O + 0.1% FA + 0.01% TFA) and MS spectrum (positive mode), and structural formula. (m) Peptide A-b6^{K631-F1}. Chemical formula: C₁₀₁H₁₃₆N₂₀O₂₇S₂. Molecular weight 2126.43. [βA-V-[C-R-K(Fl)-D-V-p-P-D-S-L-L-C]-F-βA] / b6. HPLC chromatogram: 5 - 65% in 30 min solvent D (ACN + 0.1% FA + 0.01% TFA), solvent C (H₂O + 0.1% FA + 0.01% TFA) and MS spectrum (positive mode), and structural formula. (n) Peptide A-b7. Chemical formula: C₈₂H₁₂₂N₂₀O₂₂S₂. Molecular weight 1804.12. [βA-V-[C-R-N-D-V-p-P-D-S-L-L-C]-F-βA] /b7. HPLC chromatogram: 5 - 65% in 30 min solvent D (ACN + 0.1% FA + 0.01% TFA), solvent C (H₂O + 0.1% FA + 0.01% TFA) and MS spectrum (positive mode), and structural formula. (o) Peptide B-ox. Chemical formula: C₇₃H₁₁₃N₁₉O₂₂S₂. Molecular weight 1672.94. [βA-V-T-R-[C-D-V-p-P-D-S-C]-L-V-F-βA]. HPLC chromatogram: 10 - 75% in 30 min solvent B (ACN + 0.1% TFA), solvent A (H₂O + 0.1% TFA) and MS spectrum (positive mode), and structural formula. (p) Peptide B-b1. Chemical formula: C₇₄H₁₁₅N₁₉O₂₂S₂. Molecular weight 1686.97. [βA-V-T-R-[C-D-V-p-P-D-S-C]-L-V-F-βA] /b1.HPLC chromatogram: 10 - 75% in 30 min solvent B (ACN + 0.1% TFA), solvent A (H₂O + 0.1% TFA) and MS spectrum (positive mode), and structural formula. (q) Peptide B-b2. Chemical formula: C₇₅H₁₁₇N₁₉O₂₂S₂. Molecular weight 1700.99. [βA-V-T-R-[C-D-V-p-P-D-S-C]-L-V-F-βA] / b2. HPLC chromatogram: 10 - 75% in 30 min solvent B (ACN + 0.1% TFA), solvent A (H₂O + 0.1% TFA) and MS spectrum (positive mode), and structural formula. (r) Peptide B-b3. Chemical formula: C₇₆H₁₁₉N₁₉O₂₂S₂. Molecular weight 1715.02. [βA-V-T-R-[C-D-V-p-P-D-S-C]-L-V-F-βA] / b3. HPLC chromatogram: 10 - 75% in 30 min solvent B (ACN + 0.1% TFA), solvent A (H₂O + 0.1% TFA) and MS spectrum (positive mode), and structural formula. (s) Peptide B-b4. Chemical formula: C₇₇H₁₁₉N₁₉O₂₂S₂. Molecular weight 1727.03. [βA-V-T-R-[C-D-V-p-P-D-S-C]-L-V-F-βA] / b4. HPLC chromatogram: 10 - 75% in 30 min solvent B (ACN + 0.1% TFA), solvent A (H₂O + 0.1% TFA) and MS spectrum (positive mode), and structural formula. (t) Peptide B-b5. Chemical formula: C₇₇H₁₂₁N₁₉O₂₂S₂. Molecular weight 1729.05. [βA-V-T-R-[C-D-V-p-P-D-S-C]-L-V-F-βA] / b5. HPLC chromatogram: 10 - 75% in 30 min solvent B (ACN + 0.1% TFA), solvent A (H₂O + 0.1% TFA) and MS spectrum (positive mode), and structural formula. (u) Peptide B-b6. Chemical formula: C₇₇H₁₁₉N₁₉O₂₂S₂. Molecular weight 1727.03. [βA-V-T-R-[C-D-V-p-P-D-S-C]-L-V-F-βA] / b6. HPLC chromatogram: 10 - 75% in 30 min solvent B (ACN + 0.1% TFA), solvent A (H₂O + 0.1% TFA) and MS spectrum (positive mode), and structural formula. (v) Peptide B-b7. Chemical formula: C₈₁H₁₂₁N₁₉O₂₂S₂. Molecular weight 1777.09. [βA-V-T-R-[C-D-V-p-P-D-S-C]-L-V-F-βA] / b7. HPLC chromatogram: 10 - 75% in 30 min solvent B (ACN + 0.1% TFA), solvent A (H₂O + 0.1% TFA) and MS spectrum (positive mode), and structural formula. (w) Peptide C-ox. Chemical formula: C₇₆H₁₂₀N₂₀O₂₀S₂. Molecular weight 1698.04. [βA-V-T-R-N-[C-V-p-P-D-C]-L-L-V-F-βA]. HPLC chromatogram: 10 - 75% in 30 min solvent B (ACN + 0.1% TFA), solvent A (H₂O + 0.1% TFA) and MS spectrum (positive mode), and structural formula. (x) Peptide C-b1. Chemical formula: C₇₇H₁₂₂N₂₀O₂₀S₂. Molecular weight 1712.06. [βA-V-T-R-N-[C-V-p-P-D-C]-L-L-V-F-βA] / b1. HPLC chromatogram: 10 - 75% in 30 min solvent B (ACN + 0.1% TFA), solvent A (H₂O + 0.1% TFA) and MS spectrum (positive mode), and structural formula. (y) Peptide C-b2. Chemical formula: C₇₈H₁₂₄N₂₀O₂₀S₂. Molecular weight 1726.09. [βA-V-T-R-N-[C-V-p-P-D-C]-L-L-V-F-βA] / b2. HPLC chromatogram: 10 - 75% in 30 min solvent B (ACN + 0.1% TFA), solvent A (H₂O + 0.1% TFA) and MS spectrum (positive mode), and structural formula. (z) Peptide C-b3. Chemical formula: C₇₉H₁₂₆N₂₀O₂₀S₂. Molecular weight 1740.12. [βA-V-T-R-N-[C-V-p-P-D-C]-L-L-V-F-βA] / b3. HPLC chromatogram: 10 - 75% in 30 min solvent B (ACN + 0.1% TFA), solvent A (H₂O + 0.1% TFA) and MS spectrum (positive mode), and structural formula. (aa) Peptide C-b4. Chemical formula: C₈₀H₁₂₆N₂₀O₂₀S₂. Molecular weight 1752.13. [βA-V-T-R-N-[C-V-p-P-D-C]-L-L-V-F-βA] / b4. HPLC chromatogram: 10 - 75% in 30 min solvent B (ACN + 0.1% TFA), solvent A (H₂O + 0.1% TFA) and MS spectrum (positive mode), and structural formula. (bb) Peptide C-b5. Chemical formula: C₈₀H₁₂₈N₂₀O₂₀S₂. Molecular weight 1754.14. [βA-V-T-R-N-[C-V-p-P-D-C]-L-L-V-F-βA] / b5. HPLC chromatogram: 5 - 65% in 30 min solvent D (ACN + 0.1% FA + 0.01% TFA), solvent C (H₂O + 0.1% FA+ 0.01% TFA) and MS spectrum (positive mode), and structural formula. (cc) Peptide C-b6. Chemical formula: C₈₀H₁₂₆N₂₀O₂₀S₂. Molecular weight 1752.13. [βA-V-T-R-N-[C-V-p-P-D-C]-L-L-V-F-βA] / b6. HPLC chromatogram: 5 - 65% in 30 min solvent D (ACN + 0.1% FA + 0.01% TFA), solvent C (H₂O + 0.1% FA + 0.01% TFA) and MS spectrum (positive mode), and structural formula. (dd) Peptide C-b7. Chemical formula: C₈₄H₁₂₈N₂₀O₂₀S₂. Molecular weight 1802.19. [βA-V-T-R-N-[C-V-p-P-D-C]-L-L-V-F-βA] /b7. HPLC chromatogram: 5 - 65% in 30 min solvent D (ACN + 0.1% FA + 0.01% TFA), solvent C (H₂O + 0.1% FA + 0.01% TFA) and MS spectrum (positive mode), and structural formula. (ee) Peptide St-1. Chemical formula: C₈₀H₁₂₄N₂₀O₂₂. Molecular weight 1717.99. [βA-V-[S5*-R-N-D-V-p-P-D-S-L-L-S5*]-F-βA] HPLC chromatogram and MS spectrum (positive mode), and structural formula. (ff) Peptide St-2. Chemical formula: C₈₁H₁₂₆N₂₀O₂₂. Molecular weight 1732.02. [βA-V-[S5*-R-N-D-V-p-P-D-S-L-L-S6*]-F-βA]HPLC chromatogram and MS spectrum (positive mode), and structural formula. (gg) Peptide St-3. Chemical formula: C₈₁H₁₂₆N₂₀O₂₂. Molecular weight 1732.02. [βA-V-[S6*-R-N-D-V-p-P-D-S-L-L-S5*]-F-βA] HPLC chromatogram and MS spectrum (positive mode), and structural formula. (hh) Peptide St-4. Chemical formula: C₈₂H₁₂₈N₂₀O₂₂. Molecular weight 1746.04. [βA-V-[S6*-R-N-D-V-p-P-D-S-L-L-S6*]-F-βA] HPLC chromatogram and MS spectrum (positive mode), and structural formula. (ii) Peptide St-5. C₇₉H₁₂₃N₁₉O₂₂. Molecular weight 1690.96. [βA-V-T-R-[S5*-D-V-p-P-D-S-S5*]-L-V-F-βA] HPLC chromatogram and MS spectrum (positive mode), and structural formula. (jj) Peptide St-6. C₈₂H₁₃₀N₂₀O₂₀. Molecular weight 1716.06. [βA-V-T-R-N-[S5*-V-p-P-D-S5*]-L-L-V-F-βA] HPLC chromatogram and MS spectrum (positive mode), and structural formula.

### DETAILED DESCRIPTION OF THE INVENTION

The term "peptide" as used herein refers to a compound comprising amino acids joined via amide bonds. As used herein "cyclic peptide" refers to a peptide comprising at least one cycle. Such cycle encompasses structures formed solely by amide bonds and structures formed when two amino acids in a peptide that are not directly joined via an amide bond are connected by a bridging moiety, for instance between the side chains of the amino acids. The cycle comprises the bridged amino acids and the amino acids along the polypeptide present between the bridged amino acids. A cycle may comprise 3, 4, 5, 6, 7, 8, 9, 10, 11, 12, 13, 14, 15, 16 or more amino acids. The cyclic peptide according to the invention may be a monocyclic peptide, comprising a single cycle or a multicyclic peptide, comprising two or more cycles.

Preferably, the cyclic peptide of the invention is a monocyclic, bicyclic or tricyclic peptide, more preferably a monocyclic or bicyclic peptide.

As used herein, the term "monocyclic peptide" refers to a peptide comprising one cycle. A cyclic peptide according to the invention is a monocyclic peptide if the peptide contains linker -c- or one bridge -b-. A preferred monocyclic peptide of the invention comprises a linker -c- and no bridges -b-.

As used herein, the term "bicyclic peptide" refers to a peptide comprising two cycles. A cyclic peptide according to the invention is a bicyclic peptide if the peptide contains linker -c- and one bridge -b-, and if the peptide contains two bridges -b-. A preferred bicyclic peptide of the invention comprises a linker -c- and one bridge -b-.

As used herein, the term "tricyclic peptide" refers to a peptide comprising three cycles. A tricyclic peptide according to the invention is a peptide that contains linker -c- and two bridges -b-.

"N-terminal" and "C-terminal" as used herein indicate the direction of amino acids joined via amide bonds in a peptide or cyclic peptide of the invention. E.g. N-terminal-X₄X₃ indicates that amino acid X₄ is attached via its carboxylic acid to amino acid X₃, while X₄ may be attached via an amide bond involving its amine to a further amino acid. Similarly, Y₃Y₄-C-terminal indicates that amino acid Y₄ is attached via its amine to amino acid Y₃, while Y₄ may be attached via an amide bond involving its carboxylic acid to a further amino acid.

A cyclic peptide of the invention may be modified, such as N-terminally and/or C-terminally modified, in particular if linker -c- is not present. Examples of modifications include acetylation, amidation, acylation, phosphorylation and methylation.

A cyclic peptide according to the invention efficiently enters a cell and blocks transcriptional activation of a Wnt-dependent reporter gene. If required, a cyclic peptide according to the invention may be attached to a cell penetrating peptide. Such cell penetrating peptide is a peptide sequence that, when linked to a cyclic peptide of the invention, facilitates efficient translocation of the peptide across cell membranes. Any cell penetrating peptide known in the art can be used with a cyclic peptide of the invention. Examples of cell penetrating peptides include, but are not limited to, polyarginine, TAT, HIV-Tat, R9-TAT, Pep-1, Pep-7, penetratin, transportan, Antp, Rev, FHV coat protein, buforin II, MAP, K-FGF, Ku70, SynBl, HN-1, TP10, pVEC, BGSC, and BGTC.

The term "amino acid" (also abbreviated as "aa") as used herein encompasses both naturally occurring and non-naturally occurring amino acids, turn mimetic structures and PEG based amino acids, and both Dextrorotatory amino acids (D-amino acids) and Levorotatory amino acids (L-amino acids).

In amino acid sequences as defined herein amino acids are denoted by three-letter symbols. These three-letter symbols and single-letter symbols are well known to the person skilled in the art and have the following meaning: A (Ala) is alanine, C (Cys) is cysteine, D (Asp) is aspartic acid, E (Glu) is glutamic acid, F (Phe) is phenylalanine, G (Gly) is glycine, H (His) is histidine, I (Ile) is isoleucine, K (Lys) is lysine, L (Leu) is leucine, M (Met) is methionine, N (Asn) is asparagine, P (Pro) is proline, Q (Gln) is glutamine, R (Arg) is arginine, S (Ser) is serine, T (Thr) is threonine, V (Val) is valine, W (Trp) is tryptophan, Y (Tyr) is tyrosine. Non-natural amino acids may be represented by their full names.

As used herein, a "positively charged amino acid" refers to a natural or non-natural amino acid that has a positive charge at physiological pH, i.e. at a pH of 7.3-7.4. Preferred positively charged amino acids are Arg, His and Lys and corresponding non-natural amino acids. More preferably a positively charged amino acid is Arg, His or Lys.

As used herein, a "polar amino acid" refers to a hydrophilic natural or non-natural amino acid that has a side chain that is uncharged at physiological pH, i.e. at a pH of 7.3-7.4, but which have an uneven distribution of electrons over their surface. Preferred polar amino acids are Ser, Thr, Asn and Gln and corresponding non-natural amino acids. More preferably a polar amino acid is Ser, Thr, Asn or Gln.

As used herein, a "negatively charged amino acid" refers to a natural or non-natural amino acid that has positive charge at physiological pH, i.e. at a pH of 7.3-7.4. Preferred negatively charged amino acids are Asp and Glu and corresponding non-natural amino acids. More preferably a negatively charged amino acid is Asp or Glu.

As used herein, a "hydrophobic amino acid" refers to a natural or non-natural amino acid that is not charged or polar at physiological pH, i.e. at a pH of 7.3-7.4. Preferred hydrophobic amino acids are Ala, Ile, Leu, Met, Phe, Trp, Tyr and Val and corresponding non-natural amino acids. More preferably a hydrophobic amino acid is Ala, Ile, Leu, Met, Phe, Trp, Tyr or Val.

As used herein, an "aromatic amino acid" refers to a hydrophobic amino acid having a side chain containing at least one aromatic or heteroaromatic ring. Preferred hydrophobic amino acids are Phe, Trp, His or Tyr and corresponding non-natural amino acids. More preferably a hydrophobic amino acid is Phe, Trp, His or Tyr.

As used herein, a "non-natural amino acid" refers to a non-genetically encoded amino acid, irrespective of whether it appears in nature or not. Non-natural amino acids that can be present in a peptide according to the invention include: β-amino acids; p-acyl-L-phenylalanine; N-acetyl lysine; O-4-allyl-L-tyrosine; 2-aminoadipic acid; 3-aminoadipic acid; beta-alanine; 4-tert-butyl hydrogen 2-azidosuccinate; beta-aminopropionic acid; 2-aminobutyric acid; 4-aminobutyric acid; 2, 4,-diamino butyric acid; 6-aminocaproic acid; 2-aminoheptanoic acid; 2-aminoisobutyric acid ; 3-aminoisobutyric acid ; 2-aminopimelic acid; p-aminophenylalanine; 2, 3-diaminobutyric acid; 2, 3-diamino propionic acid; 2, 2'-diaminopimelic acid; p-amino-L-phenylalanine; p-azido-L-phenylalanine; D-allylglycine; p-benzoyl-L-phenylalanine; 3-benzothienyl alanine p-bromophenylalanine; t-butylalanine; t-butylglycine; 4-chlorophenylalanine; cyclohexylalanine; cysteic acid; D-citrulline; thio-L-citrulline; desmosine; epsilon-amino hexanoic acid; N-ethylglycine; N-ethylasparagine; 2-fluorophenylalanine; 3-fluorophenylalanine; 4-fluorophenylalanine; homoarginine; homocysteine; homoserine; hydroxylysine; allo- hydroxylysine; 3-(3-methyl-4-nitrobenzyl)-L-histidine methyl ester; isodesmosine; allo-isoleucine; isopropyl-L-phenylalanine; 3-methyl-phenylalanine; N-methylglycine; N-methylisoleucine; 6-N-methyllysine; O-methyl-L-tyrosine; N-methylvaline; methionin sulfoxide; 2-napthylalanine; L-3-(2-naphthyl)alanine; isoserine; 3-phenylserine; norvaline; norleucine; 5,5,5-trifluoro-DL-leucine; ornithine; 3-chloro-tyrosine; N5-carbamoylornithine; penicillamine; phenylglycine; piperidinic acid; pyridylalanine; 1, 2, 3, 4-tetrahydro-isoquinoline-3-carboxylix acid; beta-2-thienylalanine; γ-carboxy-DL-glutamic acid; 4-fluoro-DL-glutamic acid; D-thyroxine; allo-threonine; 5-hydroxy-tryptophan; 5-methoxy-tryptophan; 5-fluoro-tryptophan; 3-fluoro-valine; (*S*)-2-(4'-pentenyl)glycine, (*S*)-2-(5'-hexenyl)glycine, PEG-based amino acids and turn mimetics.

As used herein, a "corresponding non-natural amino acid" refers to a non-natural amino acid that is a derivative of the reference natural amino acid. As a general examples, β-amino acids are corresponding non-natural amino acids of the reference natural amino acid. For instance, β-alanine is a corresponding non-natural amino acid of α-alanine, etc. Other examples of corresponding non-natural amino acid for specific natural amino acids are the following: beta-alanine, t-butylalanine, 2-napthylalanine, L-3-(2-naphthyl)alanine, or 2-aminoisobutyric acid for alanine; homoarginine, ornithine, N5-carbamoylornithine, or 3-amino-propionic acid for arginine; N-ethylasparagine for asparagine; 4-tert-butyl hydrogen 2-azidosuccinate for aspartic acid; cysteic acid, homocysteine, or selenocysteine for cysteine; γ-carboxy-DL-glutamic acid, or 4-fluoro-DL-glutamic acid for glutamic acid; D-citrulline, or thio-L-citrulline for glutamine; N- methylglycine, t-butylglycine, N-methylglycine, or D-allylglycine for glycine; 3-(3-methyl-4-nitrobenzyl)-L-histidine methyl ester for histidine; isodesmosine, N-methylisoleucine, or allo-isoleucine for isoleucine; norleucine, desmosine, or 5,5,5-trifluoro-leucine for leucine; 6-N-methyllysine, 2-aminoheptanoic acid, N-acetyl lysine, hydroxylysine, or allo-hydroxylysine for lysine; methionin sulfoxide for methionine; p-amino-L-phenylalanine, 3-benzothienyl alanine p-bromophenylalanine, p-acyl-L-phenylalanine, 2-fluorophenylalanine, 3-fluorophenylalanine, or 4-fluorophenylalanine for phenylalanine; 3-hydroxyproline, 4-hydroxyproline, or 1-acetyl-4-hydroxy-L-proline for proline; homoserine, isoserine, or 3-phenylserine for serine; D-thyroxine, or allo-threonine for threonine; 5-hydroxy-tryptophan, 5-methoxy-tryptophan, or 5-fluoro-tryptophan for tryptophan; O-methyl-L-tyrosine, O-4-allyl-L-tyrosine, or 3-chloro-tyrosine for tyrosine; and norvaline, N-methylvaline, or 3-fluoro-valine for valine.

α-Amino acids contain a single central carbon atom, bound to the primary amine and to the carboxylic acid. Hence, as used herein, a "non-alpha amino acid" refers to any amino acid that is not an alpha amino acid. The term includes beta amino acids, which have two carbon atoms between the amine and carboxylic acid, and gamma amino, which have three carbon atoms between the amine and carboxylic acid.

The term "independently selected" means that if multiple variables are selected from the indicated group, these multiple variables may be the same or different from each other.

The term "treating" or "treatment", as is used herein, refers to the application of an agent, such as a cyclic peptide according to the invention, in an attempt to cure or mitigate a disease or condition, or to prevent progression of a disease or condition.

In a first aspect, the invention provides a cyclic peptide comprising the amino acid sequence N-terminal-X₄X₃X₂X₁-a-Y₁Y₂Y₃Y₄-C-terminal, wherein -a- represents a linker, and wherein the peptide comprises a linker -c- between the N-terminal amino acid and C-terminal amino acids, and/or wherein the two amino acids of one or two of the following amino acid pairs form a side chain-to-side chain bridged di-amino acid forming a bridge -b-: X₁ and Y₁; X₂ and Y₂; X₃ and Y₃; and X₄ and Y₄; and wherein the amino acids that are not bridged are as follows: X4 is a positively charged or polar amino acid (aa); X₃ can be any aa; X₂ is a negatively charged aa or an amide; X₁ is a hydrophobic aa; Y₁ is a is a negatively charged aa, or an amide; Y₂ can be any aa; Y₃ is a hydrophobic aa; and Y₄ is a hydrophobic aa.

In one embodiment, the cyclic peptide further comprises one or more of X₅ or X₆X₅ N-terminally, and/or Y₅ or Y₅Y₆ C-terminally, wherein X₆ can be any aa; X₅ can be any aa; Y₅ is a hydrophobic aa, and Y₆ is an aromatic aa or, wherein the two amino acids of one or two of the following amino acid pairs form a side chain-to-side chain bridged di-amino acid forming a bridge -b-: X₁ and Y₁; X₂ and Y₂; X₃ and Y₃; X₄ and Y₄; X₅ and Y₅; and X₆ and Y₆.

Preferably, the number of amino acids X and the number of amino acids Y in a cyclic peptide of the invention is the same. I.e. either both X₅ and Y₅ are both present in the cyclic peptide or both X₆X₅ and Y₅Y₆ are both present in the cyclic peptide. Thus, a cyclic peptide of the invention preferably comprises the amino acid sequence N-terminal-X₄X₃X₂X₁-a-Y₁Y₂Y₃Y₄-C-terminal, N-terminal- X₅X₄X₃X₂X₁-aY₁Y₂Y₃Y₄Y₅-C-terminal or N-terminal- X₆X₅X₄X₃X₂X₁-a-Y₁Y₂Y₃Y₄Y₅Y₆-C-terminal.

A cyclic peptide of the invention comprises linker -a-, and at least either linker -c- or one bridge -b-. Optionally, a cyclic peptide of the invention comprises linker -a-, linker -c- and one or two bridges -b-, preferably one bridge -b-. As another example, a cyclic peptide of the invention comprises two bridges -b-. Hence, a cyclic peptide is for instance represented by one of the following structures, wherein X₅, X₆,Y₅ and Y₆ are optional amino acids and one or two of bridges b1, b2, b3, b4, b5 and b6 are present: 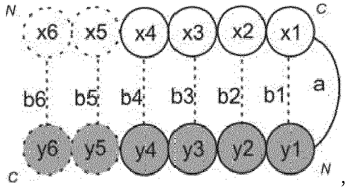 and

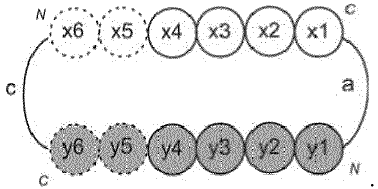

The amino acids present in a cyclic peptide of the invention that are not bridged are preferably as follows: X₁ is selected from Val or a corresponding non-natural amino acid, Leu or a corresponding non-natural amino acid, and Ile or a corresponding non-natural amino acid; X₂ is selected from Asp or a corresponding non-natural amino acid, Glu or a corresponding non-natural amino acid, Asn or a corresponding non-natural amino acid, and Gln or a corresponding non-natural amino acid; X₄ is Arg or a corresponding non-natural amino acid; Y₁ is selected from Asp or a corresponding non-natural amino acid, Glu or a corresponding non-natural amino acid, Asn or a corresponding non-natural amino acid, and Gln or a corresponding non-natural amino acid; Y₃ is selected from Val or a corresponding non-natural amino acid, Leu or a corresponding non-natural amino acid, Ile or a corresponding non-natural amino acid and Cys or a corresponding non-natural amino acid; Y₄ is selected from Val or a corresponding non-natural amino acid, Leu or a corresponding non-natural amino acid, Ile or a corresponding non-natural amino acid, and Cys or a corresponding non-natural amino acid; Y₅ is selected from Val or a corresponding non-natural amino acid, Leu or a corresponding non-natural amino acid, Ile or a corresponding non-natural amino acid, and Cys or a corresponding non-natural amino acid; and Y₆ is selected from Phe or a corresponding non-natural amino acid, Tyr or a corresponding non-natural amino acid, His or a corresponding non-natural amino acid and Trp or a corresponding non-natural amino acid.

In a preferred embodiment, the amino acids present in a cyclic peptide of the invention that do not participate in a bridge are as follows: X₁ is selected from Val, Leu and Ile; X₂ is selected from Asp, Glu, Asn, and Gln; X₄ is Arg; Y₁ is selected from Asp, Glu, Asn, and Gln; Y₃ is selected from Val, Leu, Ile and Cys; Y₄ is selected from Val, Leu, Ile, and Cys; Y₅ is selected from Val, Leu, Ile, and Cys; and Y₆ is selected from Phe, Tyr, His and Trp.

In one particular embodiment, the amino acids present in a cyclic peptide of the invention that do not participate in a bridge are as follows: X₁ is Val; X₂ is Asp; X₃ is Asn; X₄ is Arg; X₅ is Thr; X₆ is Val; Y₁ is Asp; Y₂ is Ser; Y₃ is Leu; Y₄ is Leu; Y₅ is Val; and Y₆ is Phe.

As used herein the term "linker" refers to a moiety that connects two amino acids in the cyclic peptide of the invention. Linkers for connecting two amino acids are well known in the art. Linkers -a- and -c- can be any moiety that covalently connects two amino acids that is not an amide bond.

Linker -a- is preferably a turn mimetic, preferably a β-turn mimetic. As used herein, the term "turn mimetic" refers to a moiety that mimics a turn in a peptide or protein, i.e. a secondary structural motif found in proteins and peptides that occurs where the peptide strand reverses direction. Small molecule mimetics of turns, and in particular β-turns, have been extensively investigated and are well known in the art.

In a preferred embodiment, linker -a- in a cyclic peptide of the invention is selected from:
- a dipeptide Dextrorotatory-aa (Daa)- Levorotatory aa (Laa), wherein aa refers to any amino acid, optionally wherein one or both aa are N-methylated;
- a dipeptide aa-Gly or aa-Pro, wherein aa refers to any amino acid, optionally wherein one or both aa are N-methylated; and
- a non-alpha amino acid.

Any dipeptide Daa-Laa can be used as linker -a-, wherein aa refers to any natural or non-natural amino acid or a combination of one natural and one non-natural amino acid. Preferably, Daa is attached to the N-terminal amino acid and Laa is attached to the C-terminal amino acid. Preferred examples include DPro-LPro, DPro-LLeu, DPro-LGly, DPro-LAla, as represented by the following structures:

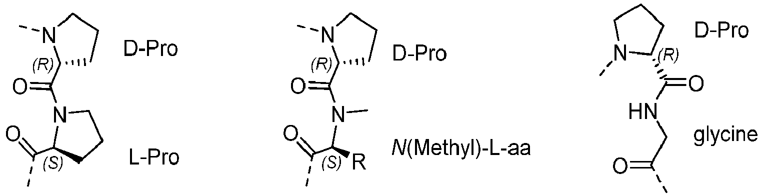

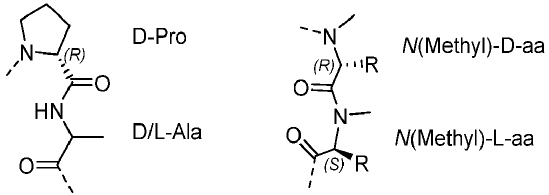

Similarly, any dipeptide aa-Gly or aa-Pro can be used as linker -a-, wherein aa refers to any natural or non-natural amino acid. Preferably, the aa of aa-Gly or aa-Pro is attached to the N-terminal amino acid and Gly or Pro is attached to the C-terminal amino acid. Preferred examples include LAsn-Gly, Aib-Gly, Aib-DPro, wherein Aib indicates 2-Aminoisobutyric acid, preferably as represented by the following structures:

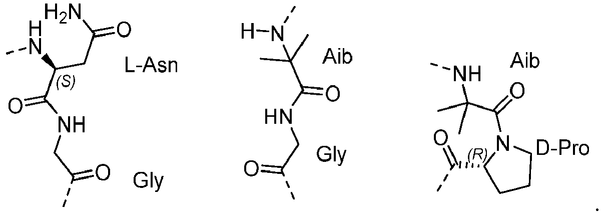

Any non-alpha amino acid can be used as linker -a-. Preferred examples thereof include L-ornithine, 5-aminovaleric acid, dibenzofuran and benzodiazepine, as represented by the following structures:

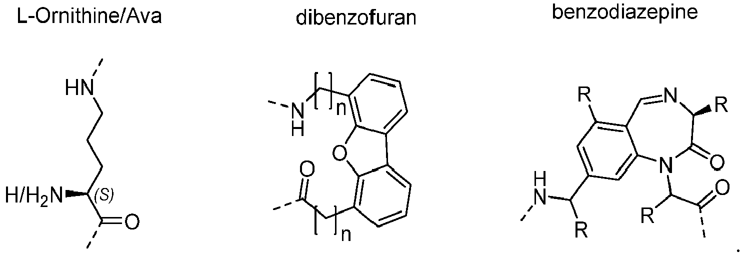

Hence, in a preferred embodiment, linker -a- is selected from DPro-LPro, DPro-LLeu, DPro-LGly, DPro-LAla, Asn-Gly, Aib-Gly, Aib-DPro, L-ornithine, 5-aminovaleric acid, dibenzofuran, and benzodiazepine.

If present, linker -c- connects the N-terminal amino acid and C-terminal amino acid of a peptide comprising the amino acid sequence N-terminal-X₄X₃X₂X₁-a-Y₁Y₂Y₃Y₄-C-terminal of the invention. Preferably X₄ and Y₄, X₅ and Y₅ or X₆ and Y₆ are connected via linker -c-. Coupling of the N- and C-terminal amino acids is also referred to as "head-to-tail cyclization" or "backbone cyclization".

If present, linker -c- is preferably a turn mimetic, preferably a β-turn mimetic. In a preferred embodiment, linker -c- in a cyclic peptide of the invention is selected from:
- a dipeptide Dextrorotatory-aa (Daa)- Levorotatory aa (Laa), wherein aa refers to any amino acid, optionally wherein one or both aa are N-methylated;
- a dipeptide aa-Gly or aa-Pro, wherein aa refers to any amino acid, optionally wherein one or both aa are N-methylated;
- a non-alpha amino acid; and
- a connector comprising 7-12 atoms connecting the N-terminal and C-terminal amino acids of the peptide comprising the amino acid sequence N-terminal-X₄X₃X₂X₁-a-Y₁Y₂Y₃Y₄-C-terminal.

Any dipeptide Daa-Laa can be used as linker -c-, wherein aa refers to any natural or non-natural amino acid or a combination of one natural and one non-natural amino acid. Preferably, the Daa is attached to the N-terminal amino acid and Laa is attached to the C-terminal amino acid. Preferred examples thereof include DPro-LPro, DPro-LLeu, DPro-LGly, DPro-LAla, DPhe-LPro as represented by the following structures:

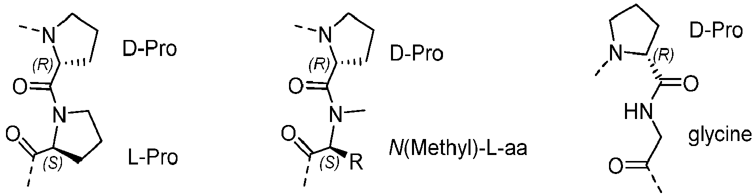

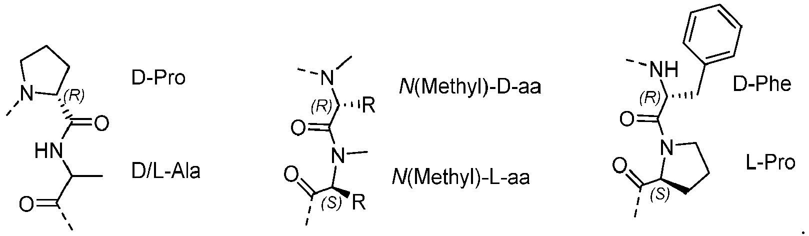

Similarly, any dipeptide aa-Gly or aa-Pro can be used as linker -c-, wherein aa refers to any natural or non-natural amino. Preferably, the aa of aa-Gly or aa-Pro is attached to the N-terminal amino acid and Gly or Pro is attached to the C-terminal amino acid. Preferred examples include Asn-Gly, Aib-Gly, Aib-DPro, wherein Aib indicates 2-Aminoisobutyric acid preferably as represented by the following structures:

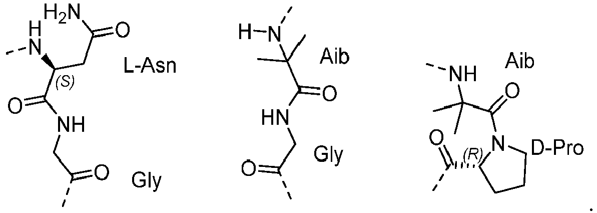

Any non-alpha amino acid can be used as linker -c-. Preferred examples thereof include L-ornithine, 5-aminovaleric acid, dibenzofuran and benzodiazepine. Any connector consisting of 7-30 atoms, connecting the N-terminal and C-terminal amino acids of the peptide comprising the amino acid sequence N-terminal-X₄X₃X₂X₁-a-Y₁Y₂Y₃Y₄-C-terminal can be used as linker -c-, preferably comprising 7-12 atoms connecting the N-terminal and C-terminal amino acids thereof. Preferably such connector is a polyethylene glycol (PEG) amino acid, 1, 2, 3 or 4 beta-alanines. As used herein, a "PEG based amino acid" refers to a non-natural amino acid comprising at least two ethylene glycol moieties, represented by the formula -(O-CH₂-CH₂)n- between the amine and carboxylic acid of the amino acid. Preferably, such PEG based amino acid is a PEG2-4 based amino acid, i.e. comprising the structure -(O-CH₂-CH₂)ₙ-, wherein n = 2, 3 or 4. Preferred examples of such linkers -c- are the following structures:

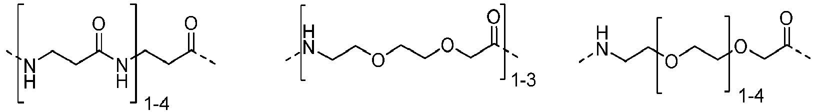

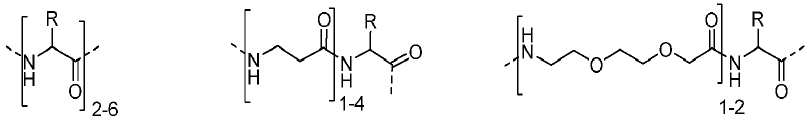

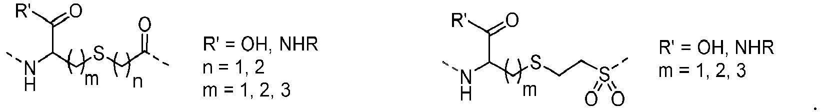

Hence, in a preferred embodiment, linker -c- is selected from DPro-LPro, DPro-LLeu, DPro-LGly, DPro-LAla, DPhe-LPro, Asn-Gly, Aib-Gly, Aib-DPro, L-ornithine, 5-aminovaleric acid, dibenzofuran, benzodiazepine, 1-4 beta-alanines and a PEG based amino acid, preferably a PEG2-4 based amino acid.

In a particularly preferred embodiment, linker -c- is selected from the following moieties:

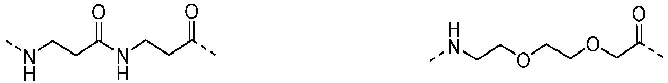

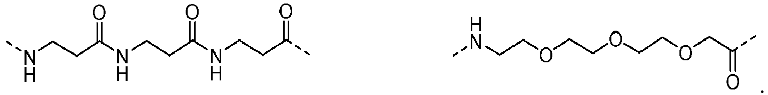

In a cyclic peptide of the invention optionally one or two pairs of amino acids form a side chain-to-side chain bridged di-amino acid forming one or two bridges -b-. These pairs of amino acids are the following: X₁ and Y₁; X₂ and Y₂; X₃ and Y₃; X₄ and Y₄; X₅ and Y₅; and X₆ and Y₆. In a preferred embodiment, the pairs of amino acids that form a side chain-to-side chain bridged di-amino acid are X₂ and Y₂, X₃ and Y₃ and/or X₅ and Y₅, more preferably X₂ and Y₂ and/or X₅ and Y₅.

The term "side chain-to-side chain bridged di-amino acid" indicates that the side chains of the two amino acids in an amino acid pair are covalently connected. The term "covalent" is well known in the art and refers to a form of chemical bonding that is characterized by the sharing of pairs of electrons between atoms. As a result of the connecting of the side chains of the amino acids between the two amino acids of a pair of amino acids a bridge -b- is formed. I.e. a cyclic peptide of the invention optionally comprises one or two bridges -b-.

As used herein "bridge -b-" refers to any moiety that connects two amino acids in a peptide, either natural or non-natural amino acids or one natural and one non-natural amino acid. If present, bridges -b- are between two amino acids, one amino acid X and one amino acid Y, with the same number in the amino acid sequence N-terminal- X₄X₃X₂X₁-a-Y₁Y₂Y₃Y₄-C-terminal, amino acid sequence N-terminal-X₅X₄X₃X₂X₁-a-Y₁Y₂Y₃Y₄Y₅-C-terminal or amino acid sequence N-terminal-X₆X₅X₄X₃X₂X₁-a-Y₁Y₂Y₃Y₄Y₅Y₆-C-terminal. I.e. if present, bridges -b- are between X₁ and Y₁; between X₂ and Y₂; between X₃ and Y₃; between X₄ and Y₄; between X₅ and Y₅; and/or between X₆ and Y₆. In a preferred embodiment, bridges -b- are present between X₂ and Y₂, between X₃ and Y₃ and/or between X₅ and Y₅, more preferably between X₂ and Y₂ and/or between X₅ and Y₅. If two bridges -b- are present in a cyclic peptide of the invention, they can be identical or different.

Bridges -b- can be any moiety that is suitable for connecting two amino acids, which moieties are well known in the art. In a preferred embodiment, the one or two side chain-to-side chain bridged di-amino acids forming one or two bridges -b-have the following structure: 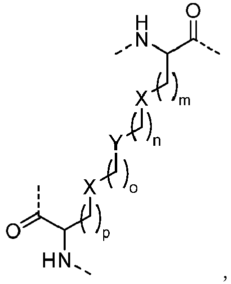 wherein m, n, o and p are independently 0, 1, 2 or 3, each X is independently CH₂, S, Se, O or NH, and Y is absent or selected from the following structures:

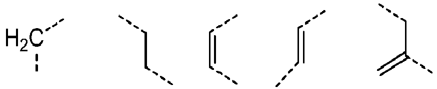

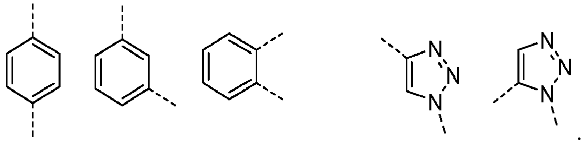

In a preferred embodiment, m and p are both 1. In another preferred embodiment, each X is S. In another preferred embodiment, n and o are independently 0, 1 or 2. In another preferred embodiment, n and o together are 0, 1, 2 or 3. In another preferred embodiment, Y is absent or selected from the following structures:

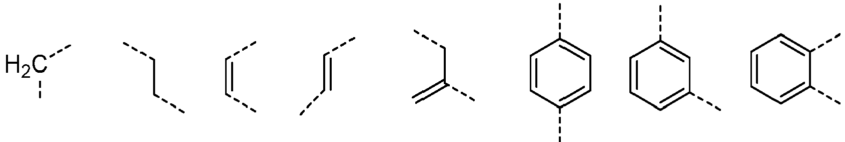

In another preferred embodiment, m and p are both 1, each X is S, n and o together are 0, 1, 2 or 3 and Y is absent or selected from the following structures:

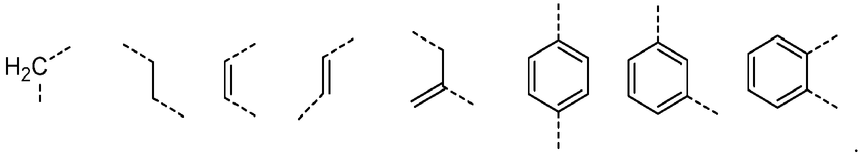

In a preferred embodiment, the one or two bridges -b- are independently selected from a thioether bond, a disulfide bond or 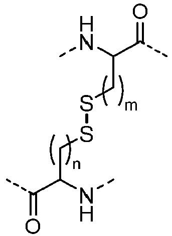 , wherein m and n are independently 1, 2 or 3, preferably independently 1 or 2.

In another preferred embodiment, the one or two bridges -b- involve a connector of at least 7 atoms between backbone of the peptide. As used herein a "connector of at least x atoms" means that the longest linear chain of atoms between the backbone of the peptide has at least x atoms, but further atoms may be present as e.g. substituents or in cyclic moieties. These at least 7 atoms include the atoms of the side chain of the two amino acids that for the side chain-to-side chain bridged di-amino acid. For instance, in case of two cysteine residues that are bridged the two side chains including the sulfur atom are 4 atoms and thus at least 3 atoms are present between the sulfur atoms. In a further preferred embodiment, the one or two bridges -b-involve a connector of 7-12 atoms between backbone of the peptide, more preferably 8-12 atoms, more preferably 8-10 atoms, such as 8 atoms. These bridges are preferably present between X₁ and Y₁; X₂ and Y₂; X₃ and Y₃; X₄ and Y₄; X₅ and Y₅; and X₆ and Y₆. In a preferred embodiment, the pairs of amino acids that form a side chain-to-side chain bridged di-amino acid are X₂ and Y₂, X₃ and Y₃ and/or X₅ and Y₅, more preferably X₂ and Y₂ and/or X₅ and Y₅.

In one preferred embodiment, the one or two bridges -b- comprise or are independently a thioether bond, a disulfide bond, or one of the following structures: 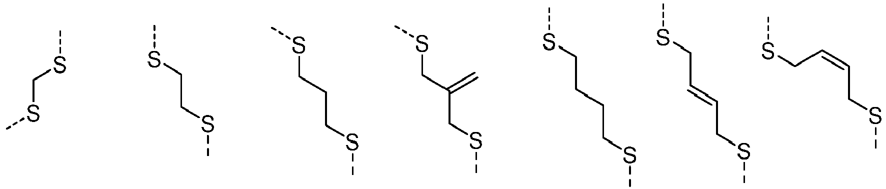 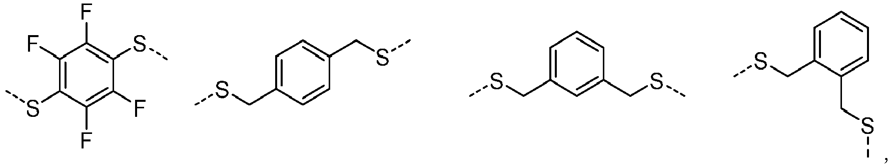 more preferably

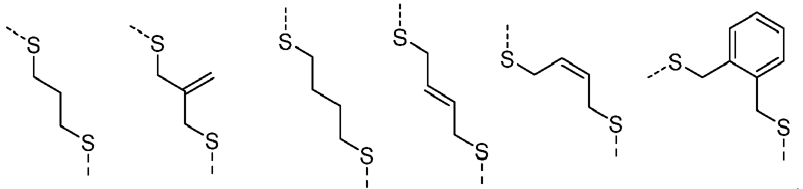

In a particularly preferred embodiment, the one or two bridges -b- comprise or are

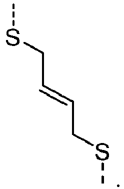

The two amino acids of one or two of the following amino acid pairs form a side chain-to-side chain bridged di-amino acid forming a bridge -b- can be any natural or non-natural amino acids that are capable of forming a side chain-to-side chain bridged di-amino acid. These two amino acids within a single pair of amino acids can be identical or different. The amino acids of two pairs amino acids can also identical or different. Natural and non-natural amino acids that are capable of forming a side chain-to-side chain bridged di-amino acid are known in the art. Hence, in one preferred embodiment, the one or two side chain-to-side chain bridged di-amino acid are independently selected from one of the following structures:

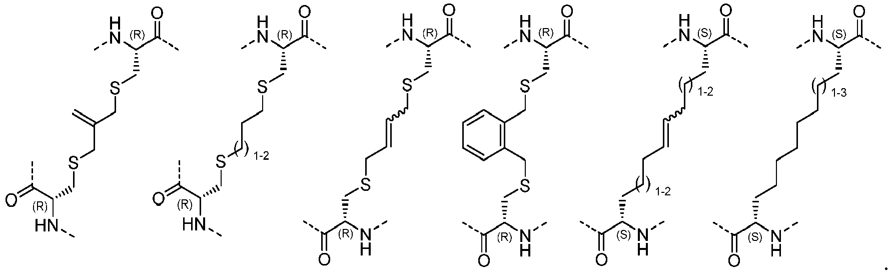

In one embodiment, said amino acids are independently a cysteine, homocysteine, penicillamine, any thiol derivative of a natural amino acid, (*S*)-2-(4'-pentenyl)glycine, (*S*)-2-(5'-hexenyl)glycine. In one embodiment, both of said amino acids are cysteines, homocysteines or a cysteine and a homocysteine.

In a particularly preferred embodiment, the two amino acids involved in bridge -b- are both cysteines, and connected by one of the following structures:

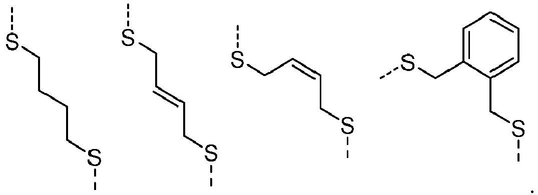

In another preferred embodiment the cyclic peptide, preferably monocyclic or bicyclic peptide, of the invention comprises the amino acid sequence N-terminal-X₄X₃X₂X₁-a-Y₁Y₂Y₃Y₄-C-terminal, N-terminal- X₅X₄X₃X₂X₁-a-Y₁Y₂Y₃Y₄Y₅-C-terminal or N-terminal- X₆X₅X₄X₃X₂X₁-a-Y₁Y₂Y₃Y₄Y₅Y₆-C-terminal wherein -a- represents a linker, wherein the peptide comprises a linker -c- between the N-terminal amino acid and C-terminal amino acid, and optionally wherein the two amino acids of one or two, preferably one, of the following amino acid pairs form a side chain-to-side chain bridged di-amino acid forming a bridge -b-: X₁ and Y₁; X₂ and Y₂; X₃ and Y₃; and X₄ and Y₄; X₅ and Y₅; X₆ and Y₆; and wherein the amino acids that are not bridged are as follows: X₆ can be any aa; X₅ can be any aa; X₄ is a positively charged or polar amino acid (aa); X₃ can be any aa; X₂ is a negatively charged aa or an amide; X₁ is a hydrophobic aa; Y₁ is a is a negatively charged aa, or an amide; Y₂ can be any aa; Y₃ is a hydrophobic aa; Y₄ is a hydrophobic aa, Y₅ is a hydrophobic aa, and Y₆ is an aromatic aa, wherein the one or two, preferably one, bridges -b-result have the structure: 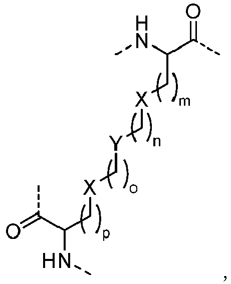 wherein m, n, o and p are independently 0, 1, 2 or 3, each X is independently CH2, S, O or NH, and Y is absent or selected from the following structures:

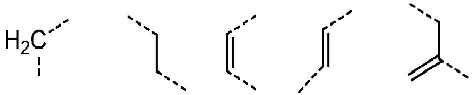

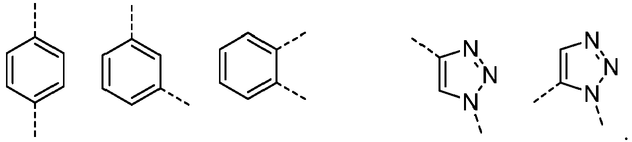

The one or two, preferably one, bridges -b- preferably involve a connector of at least 7 atoms, preferably 8-12, more preferably 8-0, more preferably 8, atoms between backbone of the peptide. These one or two bridges -b- are preferably present between the amino acids X₂ and Y₂, between X₃ and Y₃ and/or between X₅ and Y₅, more preferably between X₂ and Y₂ and/or between X₅ and Y₅ more preferably between X₂ and Y₂ or between X₅ and Y₅. The linkers -a- and -c- are preferably a turn mimetic.

In another preferred embodiment the cyclic peptide, preferably monocyclic or bicyclic peptide, of the invention comprises the amino acid sequence N-terminal-X₄X₃X₂X₁-a-Y₁Y₂Y₃Y₄-C-terminal, N-terminal- X₅X₄X₃X₂X₁-a-Y₁Y₂Y₃Y₄Y₅-C-terminal or N-terminal- X₆X₅X₄X₃X₂X₁-a-Y₁Y₂Y₃Y₄Y₅Y₆-C-terminal, wherein -a- represents a linker, wherein the peptide comprises a linker -c- between the N-terminal amino acid and C-terminal amino acid, and optionally wherein the two amino acids of one or two, preferably one, of the following amino acid pairs form a side chain-to-side chain bridged di-amino acid forming a bridge -b-: X₁ and Y₁; X₂ and Y₂; X₃ and Y₃; X₄ and Y₄; X₅ and Y₅; X₆ and Y₆; and wherein the amino acids that are not bridged are as follows: X6 can be any aa; X5 can be any aa; X₄ is a positively charged or polar amino acid (aa); X₃ can be any aa; X₂ is a negatively charged aa or an amide; X₁ is a hydrophobic aa; Y₁ is a is a negatively charged aa, or an amide; Y₂ can be any aa; Y₃ is a hydrophobic aa; Y₄ is a hydrophobic aa, Y₅ is a hydrophobic aa, and Y₆ is an aromatic aa, wherein the one or two, preferably one, bridges -b- result in a connector of at least 7 atoms between backbone of the peptide. The one or two, preferably one, bridges -b- preferably result in a connector of at least 7 atoms, preferably 8-12, more preferably 8-10, more preferably 8, atoms between backbone of the peptide. These one or two bridges -b- are preferably present between the amino acids X₂ and Y₂, between X₃ and Y₃ and/or between X₅ and Y₅, more preferably between X₂ and Y₂ and/or between X₅ and Y₅ more preferably between X₂ and Y₂ or between X₅ and Y₅. The linkers -a- and -c- are each preferably a turn mimetic.

In another preferred embodiment the cyclic peptide, preferably monocyclic or bicyclic peptide, of the invention comprises the amino acid sequence N-terminal-X₄X₃X₂X₁-a-Y₁Y₂Y₃Y₄-C-terminal, N-terminal- X₅X₄X₃X₂X₁-a-Y₁Y₂Y₃Y₄Y₅-C-terminal or N-terminal- X₆X₅X₄X₃X₂X₁-a-Y₁Y₂Y₃Y₄Y₅Y₆-C-terminal, wherein -a- represents a linker, wherein the peptide comprises a linker -c- between the N-terminal amino acid and C-terminal amino acid, and optionally wherein the two amino acids of one or two, preferably one, of the following amino acid pairs form a side chain-to-side chain bridged di-amino acid forming a bridge -b-: X₁ and Y₁; X₂ and Y₂; X₃ and Y₃; X₄ and Y₄; X₅ and Y₅; X₆ and Y₆; and wherein the amino acids that are not bridged are as follows: X₆ can be any aa; X₅ can be any aa; X₄ is a positively charged or polar amino acid (aa); X₃ can be any aa; X₂ is a negatively charged aa or an amide; X₁ is a hydrophobic aa; Y₁ is a is a negatively charged aa, or an amide; Y₂ can be any aa; Y₃ is a hydrophobic aa; Y₄ is a hydrophobic aa, Y₅ is a hydrophobic aa, and Y₆ is an aromatic aa, wherein the one or two, preferably one, side chain-to-side chain bridged di-amino acids are selected from the following structures:

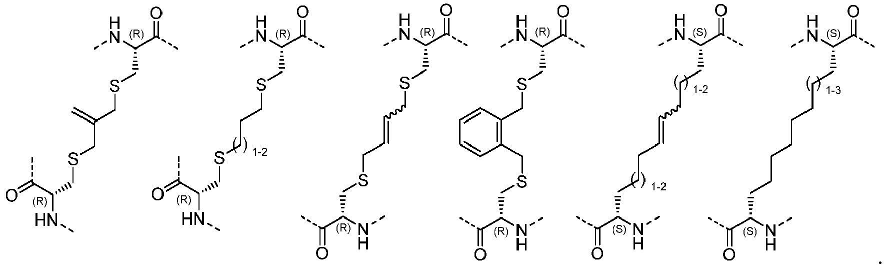

These one or two side chain-to-side chain bridged di-amino acids are preferably present between the amino acids X₂ and Y₂, between X₃ and Y₃ and/or between X₅ and Y₅, more preferably between X₂ and Y₂ and/or between X₅ and Y₅ more preferably between X₂ and Y₂ or between X₅ and Y₅. The linkers -a- and -c- are each preferably a turn mimetic.

In another preferred embodiment the cyclic peptide, preferably monocyclic or bicyclic peptide, of the invention comprises the amino acid sequence N-terminal-X₄X₃X₂X₁-a-Y₁Y₂Y₃Y₄-C-terminal, N-terminal- X₅X₄X₃X₂X₁-a-Y₁Y₂Y₃Y₄Y₅-C-terminal or N-terminal- X₆X₅X₄X₃X₂X₁-a-Y₁Y₂Y₃Y₄Y₅Y₆-C-terminal wherein -a- represents a linker, wherein the peptide comprises a linker -c- between the N-terminal amino acid and C-terminal amino acid, and optionally wherein the two amino acids of one or two, preferably one, of the following amino acid pairs form a side chain-to-side chain bridged di-amino acid forming a bridge -b-: X₁ and Y₁; X₂ and Y₂; X₃ and Y₃; and X₄ and Y₄; X₅ and Y₅; X₆ and Y₆; and wherein the amino acids that are not bridged are as follows: X₆ can be any aa; X₅ can be any aa; X₄ is Arg, or a corresponding non-natural amino acid; X₃ can be any aa; X₂ is selected from Asp, Glu, Asn, and Gln, or corresponding non-natural amino acids; X₁ is selected from Val, Leu and Ile, or corresponding non-natural amino acids; Y₁ is selected from Asp, Glu, Asn, and Gln; Y₂ can be any aa; Y₃ is selected from Val, Leu, Ile and Cys, or corresponding non-natural amino acids; Y₄ is selected from Val, Leu, Ile, and Cys, or corresponding non-natural amino acids; Y₅ is selected from Val, Leu, Ile, and Cys, or corresponding non-natural amino acids, and Y₆ is selected from Phe, Tyr, His and Trp, or corresponding non-natural amino acids, wherein the one or two, preferably one, bridges -b-result have the structure: 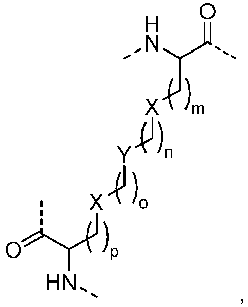 wherein m, n, o and p are independently 0, 1, 2 or 3, each X is independently CH2, S, O or NH, and Y is absent or selected from the following structures: 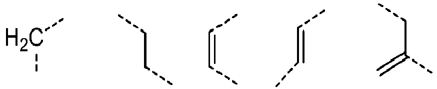 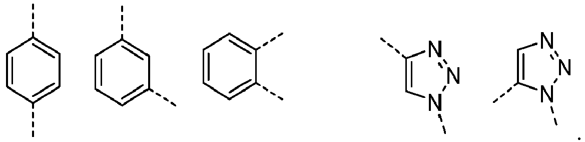 The one or two, preferably one, bridges -b- preferably involve a connector of at least 7 atoms, preferably 8-12, more preferably 8-10, more preferably 8, atoms between backbone of the peptide. These one or two bridges -b- are preferably present between the amino acids X₂ and Y₂, between X₃ and Y₃ and/or between X₅ and Y₅, more preferably between X₂ and Y₂ and/or between X₅ and Y₅, more preferably between X₂ and Y₂ or between X₅ and Y₅. The linkers -a- and -c- are each preferably a turn mimetic.

In another preferred embodiment the cyclic peptide, preferably monocyclic or bicyclic peptide, of the invention comprises the amino acid sequence N-terminal-X₄X₃X₂X₁-a-Y₁Y₂Y₃Y₄-C-terminal, N-terminal- X₅X₄X₃X₂X₁-a-Y₁Y₂Y₃Y₄Y₅-C-terminal or N-terminal- X₆X₅X₄X₃X₂X₁-a-Y₁Y₂Y₃Y₄Y₅Y₆-C-terminal wherein -a- represents a linker, wherein the peptide comprises a linker -c- between the N-terminal amino acid and C-terminal amino acid, and optionally wherein the two amino acids of one or two, preferably one, of the following amino acid pairs form a side chain-to-side chain bridged di-amino acid forming a bridge -b-: X₁ and Y₁; X₂ and Y₂; X₃ and Y₃; and X₄ and Y₄; X₅ and Y₅; X₆ and Y₆; and
wherein the amino acids that are not bridged are as follows: X₁ is Val; X₂ is Asp; X₃ is Asn; X₄ is Arg; X₅ is Thr; X₆ is Val; Y₁ is Asp; Y₂ is Ser; Y₃ is Leu; Y₄ is Leu; Y₅ is Val; and Y₆ is Phe, wherein the one or two, preferably one, bridges -b-result have the structure: 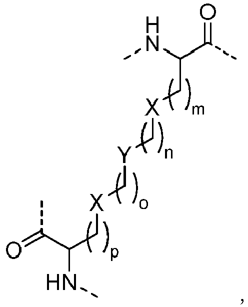 wherein m, n, o and p are independently 0, 1, 2 or 3, each X is independently CH₂, S, O or NH, and Y is absent or selected from the following structures: 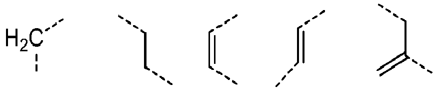 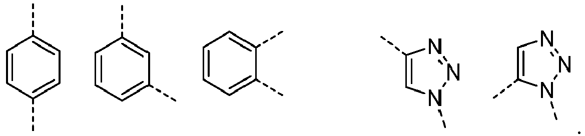 The one or two, preferably one, bridges -b- preferably result in a connector of at least 7 atoms, preferably 8-12, more preferably 8-10, more preferably 8, atoms between backbone of the peptide. These one or two bridges -b- are preferably present between the amino acids X₂ and Y₂, between X₃ and Y₃ and/or between X₅ and Y₅, more preferably between X₂ and Y₂ and/or between X₅ and Y₅ more preferably between X₂ and Y₂ or between X₅ and Y₅. The linkers -a- and -c- are preferably a turn mimetic.

In another preferred embodiment the cyclic peptide, preferably monocyclic or bicyclic peptide, of the invention comprises the amino acid sequence N-terminal-X₄X₃X₂X₁-a-Y₁Y₂Y₃Y₄-C-terminal, N-terminal- X₅X₄X₃X₂X₁-a-Y₁Y₂Y₃Y₄Y₅-C-terminal or N-terminal- X₆X₅X₄X₃X₂X₁-a-Y₁Y₂Y₃Y₄Y₅Y₆-C-terminal wherein -a- represents a linker, wherein the peptide comprises a linker -c- between the N-terminal amino acid and C-terminal amino acid, and optionally wherein the two amino acids of one or two, preferably one, of the following amino acid pairs form a side chain-to-side chain bridged di-amino acid forming a bridge -b-: X₁ and Y₁; X₂ and Y₂; X₃ and Y₃; and X₄ and Y₄; X₅ and Y₅; X₆ and Y₆; and wherein the amino acids that are not bridged are as follows: X₆ can be any aa; X₅ can be any aa; X₄ is Arg, or a corresponding non-natural amino acid; X₃ can be any aa; X₂ is selected from Asp, Glu, Asn, and Gln, or corresponding non-natural amino acids; X₁ is selected from Val, Leu and Ile, or corresponding non-natural amino acids; Y₁ is selected from Asp, Glu, Asn, and Gln; Y₂ can be any aa; Y₃ is selected from Val, Leu, Ile and Cys, or corresponding non-natural amino acids; Y₄ is selected from Val, Leu, Ile, and Cys, or corresponding non-natural amino acids; Y₅ is selected from Val, Leu, Ile, and Cys, or corresponding non-natural amino acids, and Y₆ is selected from Phe, Tyr, His and Trp, or corresponding non-natural amino acids, wherein the two amino acids that form a side chain-to-side chain bridged di-amino acid are selected from cysteines and non-natural amino acids that are capable of forming a side chain-to-side chain bridged di-amino acid, including homocysteine, penicillamine, any thiol derivative of a natural amino acid, (*S*)-2-(4'-pentenyl)glycine, (*S*)-2-(5'-hexenyl)glycine, and wherein the one or two, preferably one, bridges -b-result have the structure: 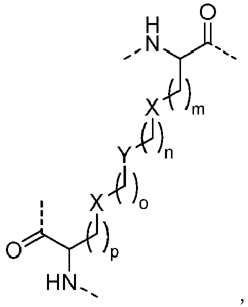 wherein m, n, o and p are independently 0, 1, 2 or 3, each X is independently CH2, S, O or NH, and Y is absent or selected from the following structures: 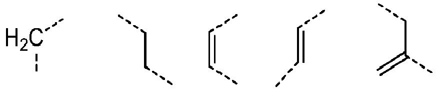 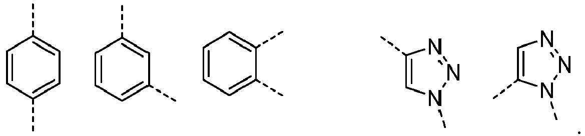 The one or two, preferably one, bridges -b- preferably involve a connector of at least 7 atoms, preferably 8-12, more preferably 8-10, more preferably 8, atoms between backbone of the peptide. These one or two bridges -b- are preferably present between the amino acids X₂ and Y₂, between X₃ and Y₃ and/or between X₅ and Y₅, more preferably between X₂ and Y₂ and/or between X₅ and Y₅ more preferably between X₂ and Y₂ or between X₅ and Y₅. The linkers -a- and -c- are preferably a turn mimetic.

A-b3, A-b4, A-b5, A-b6, A-b7, B-o, B-ox, B-b1, B-b6, B-b7, C-b4, C-b5, C-b6 and C-b7 as described herein.

In another preferred embodiment, the cyclic peptide is selected from peptides A-b5, A-b6, A-b7, B-b6, B-b7, C-b5, C-b6 and C-b7 as described herein.

In another preferred embodiment, the cyclic peptide is selected from peptides A-b5, A-b6, A-b7, C-b5, and C-b7 as described herein.

A cyclic peptide, preferably a monocyclic or bicyclic peptide, according to the invention comprises at least 8 amino acids. However, the cyclic peptide can be larger. A cyclic peptide according to the invention, preferably a monocyclic or bicyclic peptide, preferably comprises 8-30 amino acids in total. I.e. this number includes natural or non-natural amino acids in linker -a-, linker -c- and/or bridge - b-. More preferably the cyclic peptide comprises 8-20 amino acids in total, more preferably 8-16 amino acids in total, more preferably 10-16 amino acids in total.

Also provided is a linear peptide. Such linear peptides are intermediates in the synthesis of the cyclic peptides of the invention. In a preferred embodiment, a linear peptide of the invention comprising the amino acid sequence N-terminal-X₄X₃X₂X₁-a-Y₁Y₂Y₃Y₄-C-terminal, wherein X₄ is a positively charged or polar amino acid (aa); X₃ can be any aa; X₂ is a negatively charged aa or an amide; X₁ is a hydrophobic aa; Y₁ is a is a negatively charged aa, or an amide; Y₂ can be any aa; Y₃ is a hydrophobic aa; Y₄ is a hydrophobic aa; -a- represents a linker, or, the two amino acids of one or two of the following amino acid pairs are an amino acid capable of forming a side chain-to-side chain bridged di-amino acid forming a bridge -b-: X₁ and Y₁; X₂ and Y₂; X₃ and Y₃; and X₄ and Y₄ and the amino acids that are not bridged are as defined above.

Such linear peptide is represented by the following structure:

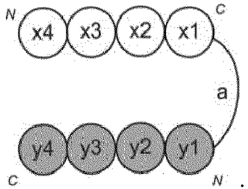

In an embodiment the linear peptide further comprises one or more of X₅ or X₆X₅ N-terminally, and/or Y₅ or Y₅Y₆ C-terminally, wherein
X₆ can be any aa;
X₅ can be any aa;
Y₅ is a hydrophobic aa, and
Y₆ is an aromatic aa, or
the two amino acids of one or two of the following amino acid pairs are an amino acid capable of forming a side chain-to-side chain bridged di-amino acid forming a bridge -b-: X₁ and Y₁; X₂ and Y₂; X₃ and Y₃; and X₄ and Y₄ and the amino acids that are not bridged are as defined above

Such linear peptide is represented by the following structure:

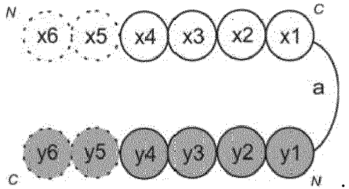

In a linear peptide of the invention, amino acids X₁-X₆ and Y₁-Y₆ and linker - a- and preferred embodiments of amino acids X₁-X₆ and Y₁-Y₆ and linker -a- is as defined herein above for cyclic peptides of the invention.

A cyclic peptide according to the invention can be prepared using any conventional method known in the art. Suitable methods are described in the Examples herein.

In one embodiment, the cyclic peptide is prepared by first synthesizing a linear peptide of the invention as described herein above. Linear peptides comprising natural amino acids, non-natural amino acids or a combination of natural and non-natural amino acids are for instance synthesized on solid-phase using Fmoc/t-Bu protecting group strategy, for instance as described in Jeganathan et al. (2019. Angew Chem Int Ed 58: 17351-173581). A detailed procedure is described in the Examples herein.

Monocyclic peptides comprising a linker -c- are for instance prepared by a combination of solid phase and solution phase peptide synthesis, whereby the head-to-tail cyclization is performed in solution. A detailed procedure is described in the Examples herein.

Monocyclic, bicyclic and tricyclic peptides according to the invention comprising one or two bridges -b- are for instance prepared starting from a linear peptide according to the invention or a monocyclic peptides according to the invention comprising a linker -c-. Disulfide bond formation between free thiol groups of for instance cysteine, homocysteine or penicillamine residues, a diselenium bond formation between two selenocyteines and bond formulation between for instance a cysteine, homocysteine or penicillamine residue and a selenocysteine can be performed by oxidation. Bridging with bis-electrophiles i.e. any structure of formula 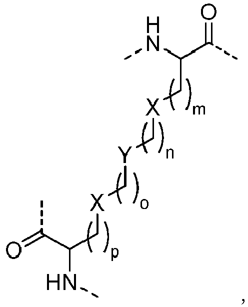 comprising a saturated or unsaturated carbon atom chain, such as diiodomethane, dibromoalkanes, 3-bromo-2-bromomethyl-1-propene, trans-1,4-dibromobut-2-ene, α,α'-dibromo-o-xylene and analogous bis-electrophiles, is for instance performed in ammonium carbonate buffer and acetonitrile or in a mixture of water and tetrahydrofuran in the presence of triethylamine. Detailed procedures are described in the Examples herein.

Linear peptides according to the invention, for instance as intermediates for monocyclic, bicyclic and tricyclic peptide of the invention can also be prepared using recombinant techniques in suitable host cells. The linear and cyclic peptides of the invention may contain unnatural amino acids. Peptide generating systems which utilize cellular systems, such as bacteria, or cell-free systems, such as free ribosomal systems, for peptide synthesis or expression can be used for incorporating non-natural amino acids into peptides using methods known in the art. One method is an auxotroph method, where the bacteria is starved of one amino acid (usually methionine) and another, structurally similar amino acid is presented, and incorporated using the codon which corresponds to the tRNA of the replaced amino acid (usually the methionine codon AUG, and the methionine tRNA, onto which the desired amino acid is loaded by the complementary tRNA synthetase). This method is only applicable for amino acids that are accepted by the replaced amino acids' tRNA synthetase. Methionine can be substituted by, for example, azido-homo-alanine. It is important to note that while using this method, methionine is not incorporated, all methionine residues in other peptides and enzymes are replaced by the new amino acid. For incorporation of an unnatural amino acid using the genetic code, a tRNA/codon pair for an unnatural amino acid is selected. The codons with which the amino acid can be incorporated into a peptide are so called 'stop codons' which usually terminate peptide elongation. The Amber (UAG), Ochre (UAA) and Opal (UGA) codons can be used, of which the Amber codon is most commonly used. This method in principle allows multiple amino acid incorporations, as there are three 'stop' codons. A complimentary tRNA of that codon, should be synthesized, onto which the desired amino acid is loaded (either chemically or enzymatically). Examples of amino acids that can be incorporated into a peptide via this method are para-acetyl phenylalanine, and para-azido phenylalanine. This method leaves more versatility to the amino acids that can be incorporated. For cellular systems, such as bacteria, it should be noted that the translation of the stop codon is in competition with peptide termination. This is not the case for cell-free systems, such as free ribosomal systems, where the release factors can be omitted. Recent efforts towards expanding and reprogramming the genetic code, have resulted in the incorporation of several unnatural amino acids into a single peptide, using an mRNA display system. In addition, monocyclic peptides can be generated by mRNA display.

The invention further provides a cyclic peptide according to the invention, for use as a medicament. The invention further provides a use of a cyclic peptide according to the invention in the preparation of a medicament for preventive or prophylactic treatment of an individual suffering from a tumor, or at risk of suffering from a tumor, or suffering of any other disease that is dependent on an activated Wingless/integrase1 (Wnt) signaling pathway.

The invention further provides a pharmaceutical composition, comprising a cyclic peptide according to the invention and a pharmaceutically acceptable excipient.

Said pharmaceutically acceptable excipient preferably is selected from a diluent, binder or granulating ingredient, a carbohydrate such as starch, a starch derivative such as starch acetate and/or maltodextrin, a polyol such as xylitol, sorbitol and/or mannitol, lactose such as α-lactose monohydrate, anhydrous α-lactose, anhydrous β-lactose, spray-dried lactose, and/or agglomerated lactose, sugars such as dextrose, maltose, dextrate and/or inulin, glidants (flow aids) and lubricants, and combinations thereof. Said excipient preferably is selected from, but not limited to, urea, L-histidine, L-threonine, L-asparagine, L-serine, L-glutamine, polysorbate, polyethylene glycol, propylene glycol, polypropylene glycol, or a combination of two or more of the above.

Said pharmaceutical composition may further comprise a buffer. Said buffer preferably is citrate-based buffer, preferably lithium-, sodium-, potassium-, or calcium- citrate monohydrate, citrate trihydrate, citrate tetrahydrate, citrate pentahydrate, or citrate heptahydrate; lithium, sodium, potassium, or calcium lactate; lithium, sodium, potassium, or calcium phosphate; lithium, sodium, potassium, or calcium maleate; lithium, sodium, potassium, or calcium tartarate; lithium, sodium, potassium, or calcium succinate; or lithium, sodium, potassium, or calcium acetate, or a combination of two or more of the above. The pH of said buffer may be adjusted, preferably to a pH of 7.27 - 7.37 by hydrochloric acid, sodium hydroxide, citric acid, phosphoric acid, lactic acid, tartaric acid, succinic acid, or a combination of two or more of the above.

Said pharmaceutical composition preferably is for use in a method of treating a patient suffering from a pre-malignant condition, a tumor or any other disease that is dependent on an activated Wnt signaling pathway. Said other disease may be selected from osteoporosis, a chronic lung disease, and a psychiatric disorder.

The invention further provides a use of a cyclic peptide according to the invention in the preparation of a medicament for preventive or prophylactic treatment of an individual suffering from a tumor, or at risk of suffering from a tumor.

A cyclic peptide according to the invention may be combined with one or more further compounds for preventive or prophylactic treatment of an individual suffering from a tumor, or suffering of any other disease that is dependent on an activated Wingless/integrase1 (Wnt) signaling pathway.

In an embodiment, said individual has, and/or is suffering from a pre-malignant condition, also termed pre-cancerous condition. The term pre-malignant condition refers to a clinically recognisable lesion that is associated with the development of malignant neoplasia. The identification of a pre-malignant condition in an individual helps to identify individuals that have an increased risk cancer development, thereby allowing prophylactic treatment to prevent the development of a malignant neoplasia. Pre-malignant lesions have been identified in especially epithelial organs and include actinic keratosis and Bowen's disease in skin, dysplastic nodule in liver, leukoplakia in the oral cavity, bronchial dysplasia in the bronchus, Barrett's disease in the esophagus (Barrett's esophagus), adenoma in the colorectal tract, intraepithelial neoplasia in the vulva and cervix, and intraepithelial neoplasia in the anal canal.

Progression of these pre-malignant lesions has been reported to involve the Wnt signaling pathway (Takacs et al., 2008. Science 319: 333-336; Clément et al., 2007. Expert Opinion Therapeutic Targets 11: 375-389). Hence, a cyclic peptide according to the invention that blocks activation of a T-cell factor (TCF)/LEF family member by β-catenin will aid in blocking or at least reducing progression of these pre-malignant lesions into a malignant neoplasia.

Said pre-malignant lesion preferably is selected from colorectal adenoma and Barrett's esophagus.

A role of the Wnt pathway is evident in a diverse set of tumors, including carcinomas such as gastrointestinal cancers, including colorectal carcinomas, adenocarcinomas such as esophageal adenocarcinoma and pancreatic ductal adenocarcinoma, and breast carcinoma. Hence, a cyclic peptide according to the invention that blocks activation of a TCF/LEF family member by β-catenin will aid in treatment of tumors such as a carcinoma, including a colorectal carcinoma, an adenoma such as esophageal adenocarcinoma and pancreatic ductal adenocarcinoma, and a breast carcinoma.

In addition, a role of the Wnt pathway has been suggested in other tumors, including leukemia and melanoma, while a role of the Wnt pathway for the function and maintenance of cancer stem cells is commonly accepted (Zhan et al., 2017. Oncogene 36: 1461-1473). Hence, a cyclic peptide according to the invention that blocks activation of a TCF/LEF family member by β-catenin will aid in treatment of further tumors, including leukemia and melanoma.

As is known to a person skilled in the art, the term surgery refers to a physical procedure in which a tumor, or at least part of a tumor, is removed from the body of an individual. Said procedure comprises making incisions in tissue, often skin or tissue aligning internal cavities in an individual. A small tumor can be excised in combination with a margin of healthy tissue surrounding the tumor.

The term radiation therapy, also termed radiation oncology, refers to method wherein ionizing radiation is used to kill especially tumor cells. The ionizing radiation is typically X-ray and can be applied either from the outside of the body, or by placing a radioactive source at or near a tumor inside a patient. The mode of action of radiation therapy is by causing DNA damage due to either ionization of DNA directly, or by ionization of water causing the formation of free radicals and indirectly damaging DNA.

For the preventive or prophylactic treatment of an individual suffering from a tumor or a pre-malignant lesion, the provision of a cyclic peptide according to the invention may be combined with one or more further anti-cancer drugs that affect cell growth, cell division and/or cell differentiation. In addition, treatment of an individual with a cyclic peptide according to the invention may be combined with surgery, radiation therapy, or a combination thereof.

Said one or more further anti-cancer drugs may include chemotherapeutic drugs such as an alkylating agent, for example a nitrogen mustard such as bendamustine, chlorambucil, cyclophosphamide, ifosfamide, mechlorethamine, and melphalan, a nitrosourea such as carmustine, lomustine, and streptozocin, an alkyl sulfonate such as busulfan, a triazine such as dacarbazine and temozolomide, and an ethylenimine such as altretamine and thiotepa; an antimetabolite such as 5-fluorouracil, hydroxyurea and methotrexate; an alkaloid such as taxane and camptothecan; a mitotic inhibitor such as vinblastine, paclitaxel and etoposide; an antitumor antibiotic such as anthracycline and chromomycin; an topoisomerase inhibitor such as camptothecin.

Said one or more further anti-cancer drugs also include one or more molecules for targeted therapy, immunotherapy and/or hormone therapy.

A molecule for targeted therapy is a molecule that specifically blocks growth of cancer cells by interfering with specific targeted molecules which are necessary for carcinogenesis and tumor growth, such as a tyrosine-kinase inhibitor and a phosphoinositide 3-kinase inhibitor, and a molecule that stimulates of the immune system of the individual to inhibit or kill cancer-associated tumor cells.

A molecule for immunotherapy is either a molecule that directs the immune system to attack tumor cells directly by targeting antigens displayed on tumor cells, and/or a molecule such as an antibody that targets antigens displayed on tumor cells. Examples of such molecules are immune checkpoint inhibitors such as anti-PD-1, anti-PD-Ll and anti-CTLA-4, CTLA B7-1 and CTLA B7-2 molecules.

A molecule for hormone therapy is a molecule that blocks and/or lowers a concentration of one or more specific hormones. This can be performed by either blocking the ability of an individual to produce said specific hormone or said specific hormones, or by interfering with how specific hormones behave in the human body. Some cancers, such as breast, prostate, ovarian and endometrial cancer require hormone stimulation such as steroid stimulation, to grow and/or develop. Hormone therapy specifically prevents the growing and division of hormone dependent/sensitive cancer cells. Examples of such hormone therapeutic molecules are hormone antagonists such as flutamide, goserelin, mitotane and tamoxifen, and aromatase inhibitors such as anastrozole, exemestane and letrozole.

The term "combination", as is used herein, refers to the administration of a cyclic peptide as defined herein, with one or more further anti-cancer drugs, to an individual in need thereof. Said cyclic peptide and one or more further anti-cancer drugs may be provided in one pharmaceutical preparation, or as two or more distinct pharmaceutical preparations. When administered as two distinct pharmaceutical preparations, they may be administered on the same day or on different days to a patient in need thereof, and using a similar or dissimilar administration protocol, e.g. daily, twice daily, biweekly, orally and/or by infusion. Said combination is preferably administered repeatedly according to a protocol that depends on the patient to be treated (age, weight, treatment history, etc.), which can be determined by a skilled physician.

The invention further provides a use of a cyclic peptide according to the invention in the preparation of a medicament for treating a disease selected from bone-related diseases such as osteoarthritis and osteoporosis, a chronic lung disease, and a psychiatric disorder.

The Wnt pathway is known to be involved in bone formation. Inhibitors of Wnt signaling have emerged as promising strategies to increase bone mass, lower adiposity and reduce fracture risk (Anastasilakis et al., 2011. Curr Opin Endocrinol Diabetes Obes 18: 383-388).

Alterations in the classical Wnt signaling pathway have been linked to chronic lung diseases, including idiopathic pulmonary fibrosis, pulmonary arterial hypertension, asthma and COPD (Baarsma and Königshoff, 2017. Thorax 72: 746-759). Blocking the interaction of beta-catenin with transcription factors such as TCF may provide an effective treatment of these chronic lung diseases.

The regulated expression of the Wnt signaling pathway in the brain is critical for many neurodevelopmental processes, and members of this pathway are candidate genes for several neuropsychiatric disorders, including schizophrenia, and Alzheimer's disease (Miyaokaet al., 1999. Schizophrenia Res 38: 1-6;Hennig et al., 2017. Mol Neuropsychiatry 3: 53-57; Anderton et al., 2000. Mol Med Today 6: 54 - 59). Inhibition of the interaction between beta-catenin and TCF transcription factors, may be of clinical use for treatment of such psychiatric disorders.

Said cyclic peptide according to the invention may be administered by oral administration, topical administration, and/or parenteral administration, including intramuscular, subcutaneous, intraperitoneal administration. A preferred mode of administration is oral administration and/or parenteral administration such as intramuscular, intravenous and/or subcutaneous administration.

The term "administering" as used herein includes all means of introducing the cyclic peptide and composition comprising the cyclic peptide as described herein to an individual. The compounds and compositions described herein may be administered in unit dosage forms and/or formulations containing conventional nontoxic pharmaceutically-acceptable carriers, adjuvants, and vehicles.

Said cyclic peptide according to the invention preferably is administered parenterally, by injection, infusion, or a combination thereof. A cyclic peptide according to the invention may be administered at 0.1-1000 milligram, such as 1-100 milligram, including about 50 milligram, or at 0.1-100 milligram per kilogram body weight, such as about 2-50 milligram/kg. The administration of a cyclic peptide is preferably performed at regular intervals, such as daily, weekly, twice weekly or 3 times weekly.

The invention further provides the use of the cyclic peptide according to the invention for inhibiting Wingless/integrase-1 (Wnt)-signaling in isolated tissues or cells. Said cyclic peptide may be used, for example, in *in vitro* studies directed at elucidating the Wnt signal transduction pathways in developmental processes and in adult tissues. Said studies may reveal further antagonists of Wnt signaling pathways that can be used in treatment of an individual in need thereof.

For the purpose of clarity and a concise description, features are described herein as part of the same or separate aspects and preferred embodiments thereof, however, it will be appreciated that the scope of the invention may include embodiments having combinations of all or some of the features described.

The invention will now be illustrated by the following examples, which are provided by way of illustration and not of limitation and it will be understood that many variations in the methods described and the amounts indicated can be made without departing from the spirit of the invention and the scope of the appended claims.

### EXAMPLES

### Example 1

### MATERIALS AND METHODS

### 1 Synthesis of linear peptides

Linear peptides were synthesized on solid-phase using Fmoc/t-Bu protecting group strategy in analogy to protocols described earlier [Jeganathan et al., 2019. Angew Chem Int Ed 58: 17351-173581]. Reagents were used without any additional purification and were purchased from Iris Biotech GmbH, Sigma Aldrich and Carl Roth. All reaction steps were performed in a syringe reactor at room temperature on an orbital shaker and unless stated otherwise, all procedures were performed with 1 mL of solvent or reagent solution per 50 mg resin. For all scales (10-100 umol) H-Rink amide ChemMatrix^{®} resin (Sigma-Aldrich, Art. No. 727768) was swollen in DMF (dimethylformamide) for 30 min. For amino acid (aa) coupling a solution of 4 eq. N-α-Fmoc (fluorenylmethyloxycarbonyl) protected amino acid, 4 eq. COMU ((1-cyano-2-ethoxy-2-oxoethyliden-aminooxy)dimethylamino-morpholino-carbenium hexafluorophosphate) and 4 eq. Oxyma (ethyl cyano (hydroxyimino) acetate) in DMF was prepared (0.3 mL per 50 mg resin). 8 eq. DIPEA (N,N-diisopropylethylamine) were added to the coupling solution. Subsequently, resin and coupling solution were mixed in a syringe reactor on an orbital shaker. After 30 min the reaction solution was discarded, and the amino acid coupling repeated once. After washing the resin with DMF (5×), Fmoc removal was performed by adding a solution of piperidine in DMF (2:8, v/v). After 5 min the solution was discarded and the Fmoc removal repeated. The resin was washed with DMF (3×), DCM (dichloromethane) (3×) and DMF (3×). Afterwards subsequent amino acids were added by repeating cycles of amino acid coupling and Fmoc removal.

Peptide synthesis was supported by automated solid phase peptide synthesis (SPPS), using the peptide synthesis robot Syro I (MultiSynTech), with a double coupling protocol of 4 eq. PyBOP (1st 40 min coupling, benzotriazol-1-yl-oxytripyrrolidino¬phosphonium hexafluorophosphate) and 4 eq HATU (2nd 40 min coupling, hexafluorophosphate azabenzotriazole tetramethyl uronium) as coupling reagents and DMF as solvent. Additionally, the coupling reaction with 4 eq. Fmoc-protected amino acid was supplemented with 4 eq. Oxyma and 8 eq. DIPEA. Before Fmoc removal was conducted with (25% (v/v) piperidine in DMF), a capping step using Ac₂O (acetic anhydride) and DIPEA in NMP (1:1:8, v/v/v) was performed. In between reaction steps, the resin was washed with DMF.

N-terminal acetylated peptides were synthesized by adding a solution of Ac₂O, DIPEA and DMF (1:1:8, v/v/v) to the immobilized peptide on resin. The reaction solution was discarded after 10 min and the acetylation was repeated. Subsequently, the resin was washed DMF (3×) and DCM (3×).

N-terminal fluorescently labeled peptides were prepared by coupling the linker PEG2 (Fmoc-O2Oc-OH, 8-(9-fluorenylmethyloxycarbonyl-amino)-3,6-dioxaoctanoic acid) as described above onto the N terminus. After Fmoc removal (see above), a solution of 6 eq. fluorescein isothiocyanate (FITC) and 12 eq. DIPEA in DMF (0.3 mL per 50 mg resin) was added to the resin. After 16 h the solution was discarded, and the resin was washed with DMF (3×) and DCM (3×).

For peptides 4 - 7, Fmoc-Glu-OAll (N-(fluorenylmethoxycarbonyl)-L-glutamic acid allyl ester) was loaded via the side chain onto the resin H-Rink amide ChemMatrix^{®} resin (Sigma-Aldrich, Art. No. 727768) and peptide elongation was proceeded as described above. Before removal of the last Fmoc protecting group and addition of fluorescein, peptide resins were suspended in a solution of 0.2 eq. Pd(PPh3)4 and 20 eq. PhSiH4 in dry DCM (3× for 10 min, Ar). Afterwards the resin was washed with DCM (3×) and DMF (3×). Fluorescein was attached as described above and after cleavage and deprotection, peptides 4 - 7 were obtained with a free C-terminus and the C-terminal residue glutamic acid being transformed to its amide analogue glutamine. Peptide cleavage and removal of side chain protecting groups was performed simultaneously, by adding a solution of TFA (trifluoroacetic acid), TIPS (triisopropylsilane) and water (95:2.5:2.5, v/v/v) to the resin. After 1 h the cleavage, solution was collected, and the resin was suspended twice more for 1 h in the cleavage solution. The cleavage solutions were pooled and TFA was evaporated. The crude peptides were obtained by precipitation in diethyl ether with subsequently centrifugation (15 min, 4000 rcf). After removal of the supernatant the crude peptide was dissolved in ACN (acetonitrile) and water (1:4, v/v).

Peptide purification was carried out via reverse-phase HPLC (high-performance liquid chromatography) on an Agilent semi preparative system 1100 (Column: Macherey-Nagel Nucleodur C18, 10 × 125 mm, 110 Å, 5 µm). Side-products and impurities were removed using various gradients of solvent A (H₂O + 0.1% TFA) and solvent B (ACN + 0.1% TFA) over 20-40 min with a flow rate of 6 mL min-1.

Pure peptides were analyzed by analytical reverse-phase HPLC coupled to ESI-MS (Agilent 1260 + quadrupole 6120, Column: Agilent Zorbax C18, 4.6 × 150 mm, 5 µm) with solvent A (H₂O + 0.1% TFA) and solvent B (ACN + 0.1% TFA) or solvent C (H₂O + 0.1 FA (formic acid) + 0.01% TFA) and D (ACN + 0.1 FA + 0.01% TFA) via a 30 min gradient from 5% to 65% or from 10% to 75% solvent B/D. Fluorescein-labeled peptides were quantified via absorption measurements (λ = 495 nm, ε495 = 77000 M⁻¹ cm⁻¹) in 100 mM sodium phosphate buffer (pH 8.5). Non-labeled peptides were quantified gravimetrically or via HPLC peptide standards. All synthesized peptides are listed in Table 1 and Table 2.

### 2 Synthesis of monocyclic peptides (12, A-o, B-o, C-o)

Cyclic peptides were obtained by a combination of solid and solution phase peptide synthesis in scales ranging from 0.1 mmol to 0.5 mmol. Before loading the first amino acid, 2-chlorotrityl chloride resin (Iris Biotech GmbH, BR-1065) was swollen in DCM for 30 min. Then, a solution of 2.5 eq. Fmoc-β-alanine (Iris Biotech GmbH, FAA1300) and 8 eq. DIPEA in DCM (0.3 ml per 50 mg resin) was added to resin and stirred for 1 h. Afterwards, the solution was discarded, and non-reacted linker groups were capped by adding a solution of MeOH (methanol), DIPEA and DCM (2:1:17, v/v/v). After 5 min, the solution was discarded, and the capping step repeated. Afterwards, peptide elongation of the linear peptide precursors was performed as described in section 1.1 with Fmoc-β-alanine being the N-terminal amino acid. The final Fmoc protecting group was removed, and peptides were cleaved from the resin, while keeping all side chain protecting groups intact. Therefore, the resin was suspended in a solution of acetic acid (AcOH), 2,2,2-trifluoroethanol (TFE) and DCM (10:10:80, v/v/v) for 2 h. The resin was removed by filtration and washed with DCM. The filtrate was collected, and the solvent was removed under reduced pressure. Residuals were dissolved in H₂O/ACN and lyophilized.

The intramolecular head-to-tail peptide cyclization was performed in solution. Crude linear peptide was dissolved in DCM/DMF (9:1) to a final concentration of 0.6 mM. Under vigorous stirring 4 eq. of DIPEA were added. After 15 min 2 eq. HATU (hexafluorophosphate azabenzotriazole tetramethyl uronium) or 2 eq. PyBOP (benzotriazol-1-yl-oxytripyrrolidinophosphonium hexafluorophosphate) and 2 eq. HOAt (1-hydroxy-7-azabenzotriazole) were added and the reaction was stirred for 5 h at room temperature. The reaction was quenched with saturated aq. NH₄Cl. The organic phase was washed with saturated NH₄Cl solution (3×), saturated NaCl solution (3×) and dried over Na₂SO₄. The solvent was removed under reduced pressure. Removal of the side-chain protecting groups and the subsequent purification was performed as described in section 1.1. Peptides were obtained as white powders. Yields and purities are listed in Table 2. All synthesized cyclic peptides are shown in figure 6.

### 3 Synthesis of bicyclic peptides

Disulfide bond formation (A-ox, B-ox, C-ox). Monocyclic peptide A-o (2 µmol), B-o (877 nmol) or C-o (2 µmol) was dissolved with 20% DMSO (dimethyl sulfoxide) in 100 mM ammonium carbonate buffer (pH 9) at a final concentration of 0.5 mM. The reaction was stirred for 24 h at room temperature. The reaction was quenched by addition of 0.1% aq. TFA and lyophilized overnight. Crude peptides were purified by preparative HPLC and characterized by analytical LC-MS (section 1.1). Peptides were obtained as white powders (A-ox: 45%, B-ox: 7%, C-ox: 44%). Overall yields of peptide synthesis and peptide purities can be found in Table 2.

Bicyclic peptides with a hydrocarbon bridge were synthesized in analogy to the above described bicycles, however including the following modifications. At the position of bridging amino acids building blocks for ring closing metathesis (RCM) were incorporated into the peptide sequence: (*S*)-*N*-Fmoc-2-(4'-pentenyl)glycine (Fmoc-S5*-OH) and (*S*)-*N*-Fmoc-2-(5'-hexenyl)glycine (Fmoc-S6*-OH). After the linear assembly until Fmoc-βAla, the resin was washed with DCM and DCE. The resin bound and protected peptides were then subjected to ring closing metathesis. Therefore, the resin was treated with a fresh catalyst solution of a 6 mM solution of Grubbs' first-generation catalyst in DCE (4.94 mg mL⁻¹, 20 mol% relative to the resin substitution). The resulting suspension was gently agitated with constant nitrogen flow for 1h. After the first round, the RCM reaction was repeated two times in the same condition. Subsequently, the resin was washed with DCE and DCM and then dried. Subsequently, peptides were cleaved and purified as described above.

All synthesized cyclic peptides are shown in figure 6.

**Table 1. Sequences and analytical details of linear peptides. Purity and mass-to-charge ratio was determined via analytical RP-HPLC-MS with absorption at 210 nM. With exception of peptides 4 - 7, all peptides possess a C-terminal amide modification. (Ac: acetyl group, Fl: fluorescein, PEG2: 2-[2-(2-aminoethoxy)ethoxy]acetic acid, B: L-norleucine, βA: β-alanine, p: D-proline).**

| **Peptide** | **Sequence** | **N-term.** | **Purity / %** | **m/z calc.** | **m/z found** | **Yield / %** |
|---|---|---|---|---|---|---|
| E-CBD | V T R N D V A P T L B S V P Q Y R P R P A N P D E I G N F I D E N L K A A D S D P T A P P Y D S L L V F | Ac | 90 | 1438.7 | 1438.8 [M+4H]⁴⁺ | 10 |
| | | Fl-PEG2 | >95 | 1561.7 | 1561.7 [M+4H]⁴⁺ | 12 |
| 1 | P T L B S V P Q Y R P R P A N P D E I G N F I D E N L K A A D S D P T A P P Y D S L L V F | Fl-PEG2 | >95 | 1830.5 | 1830.4 [M+3H]³⁺ | 10 |
| 2 | PQYRPRPANPDEIGNFIDENLKA ADSDPTAPPYDSLLVF | Fl-PEG2 | >95 | 1626.8 | 1626.9 [M+3H]³⁺ | 14 |
| 3 | E I G N F I D E N L K A A D S D P T A P P Y D S L L V F | Ac | >95 | 1547.3 | 1547.4 [M+2H]²⁺ | 9 |
| | | Fl-PEG2 | >95 | 1195.9 | 1196.0 [M+3H]³⁺ | 9 |
| 4 | Y R P R P A N P D E I G N F I D E N L K A A D S N P T A P P Y D S L L V F p P G V T R N D V A P T L B S V P Q | Fl-PEG2 | >95 | 1624.8 | 1624.8 [M+4H]⁴⁺ | 1 |
| 5 | Y R P R P A N P D E I G N F I D E N L K A A D S N P T A P P Y D S L L V F p P ß A V T R N D V A P T L B S V P Q | Fl-PEG2 | >95 | 1628.3 | 1627.9 [M+4H]⁴⁺ | 2 |
| 6 | Y R P R P A N P D E I G N F I D E N L K A A D S N P T A P P Y D S L L V F ßA ßA V T R N D V A P T L B S V P Q | Fl-PEG2 | 90 | 1597.5 | 1597.3 [M+4H]⁴⁺ | 2 |
| 7 | Y R P R P A N P D E I G N F I D E N L K A A D S N P T A P P Y D S L L V F ßA ßA ßA V T R N D V A P T L B S V P Q | Fl-PEG2 | >95 | 1615.3 | 1615.2 [M+4H]⁴⁺ | 2 |
| **8** | N P D E I G N F I D E N L K A A D S N P T A P P Y D S L L V F ßA ßA V T R N D V A P T L B S V P Q | Fl-PEG2 | >95 | 1882.6 | 1882.5 [M+3H]³⁺ | <1 |
| **9** | D S L L V F ßA ßA V T R N D V A P T L B S V P Q | Fl-PEG2 | 90 | 1480.7 | 1480.7 [M+2H]²⁺ | <1 |
| **10** | Y R P R P A N P D E I G N F I D E N L K A A D S N P T A P P Y D S L L V F ß A ß A V T R N D V | Fl-PEG2 | >95 | 1370.6 | 1370.5 [M+4H]⁴⁺ | <1 |
| **11** | DSLLVFβAβAVTRNDV | Fl-PEG2 | 95 | 1027.5 | 1027.1 [M+2H]²⁺ | <1 |
| **12-1** | VTRNDVpPDSLLVF | Ac | >95 | 806.9 | 807.1 [M+2H]²⁺ | 5 |
| | | Fl-PEG2 | >95 | 1053.5 | 1053.2 [M+2H]²⁺ | 12 |
| **TCF-4 tracer**^{[13]} | D E L I S F K D E G E Q E βA βA E R D L A D V K S S L V N | Fl-PEG2 | 95 | 1214.6 | 1214.9 [M+3H]³⁺ | 2 |
| **Tat** | GRKKRRQRRRPQ | Ac | >95 | 555.0 | 555.2 [M+3H]³⁺ | 16 |

**Table 2: Sequences and analytical details of monocyclic and bicyclic peptides. Purity and mass-to-charge ratio was determined via analytical RP-HPLC-MS (210 nM). Side chain modifications are highlighted in brackets. Cyclization positions are indicated with squared brackets. Cyc: Peptide was cyclized in a head-to-tail manner. Bicyc: Peptide was first cyclized in a head-to-tail manner and subsequently via cysteine side chains using biselectrophiles (e1: diiodo-methane, e2: 1,2-dibromoethane, e3: 1,3-dibromopropane, e4: 3-bromo-2-bromo-methyl-1-propene, e5: 1,4-dibromobutane, e6: trans-1,4-dibromobut-2-ene, e7: α,α'-dibromo-o-xylene, Fl: 5/6-carboxyfluorescein, βA: β-alanine, p: D-proline). Bicyc*: Peptide was first cyclized via ring closing metathesis using alkene bearing amino acids and subsequently in a head-to-tail manner in solution. (S5*: (S)-2-(4'-pentenyl)glycine S6*: (S)-2-(5'-hexenyl)glycine).**

| **Peptide** | | **Cross-linker Sequence** | | **Purity /%** | **m/z calc.** | **m/z found** | **Yield /%** |
|---|---|---|---|---|---|---|---|
| **12** | Cyc | - | [βA V T R N D V p P D S L L V F βA] | >95 | 848.5 | 848.6 [M+2H]²⁺ | 10 |
| **12^{K631-Fl}** | | | [βA V T R K(Fl) D Vp P D S LLVF βA] | >95 | 1035.0 | 1034.7 [M+2H]²⁺ | 4 |
| **A-o** | Cyc | - | [βA V C R N D V p P D S L L C F βA] | >95 | 851.4 | 851.5 [M+2H]²⁺ | 8 |
| **A-ox** | Bicyc | (disulfide) | [βA V [C R N D V p P D S L L C] F βA] | >95 | 850.4 | 850.5 [M+2H]²⁺ | 4 |
| **A-bl** | Bicyc | **e1** | [βA V [C R N D V p P D S L L C] F βA] | >95 | 857.4 | 857.6 [M+2H]²⁺ | 1 |
| **A-b2** | Bicyc | **e2** | [βA V [C R N D V p P D S L L C] F βA] | >95 | 864.4 | 864.5 [M+2H]²⁺ | 2 |
| **A-b3** | Bicyc | **e3** | [βA V [C R N D V p P D S L L C] F βA] | >95 | 871.4 | 871.6 [M+2H]²⁺ | 1 |
| **A-b4** | Bicyc | **e4** | [βA V [C R N D V p P D S L L C] F βA] | >95 | 877.4 | 877.6 [M+2H]²⁺ | 5 |
| **A-b5** | Bicyc | **e5** | [βA V [C R N D V p P D S L L C] F βA] | >95 | 878.4 | 878.6 [M+2H]²⁺ | 1 |
| **A-b6** | Bicyc | **e6** | [βA V[C R N D V p P D S L L C] F βA] | >95 | 877.4 | 877.6 [M+2H]²⁺ | 2 |
| **A-b6^{K631-Fl}** | | | [βA V [C R K(Fl) D V p P D S L L C] F βA] | >95 | 1064.0 | 1064.0 [M+2H]²⁺ | <1 |
| **A-b7** | Bicyc | **e7** | [βA V [C R N D V p P D S L L C] F βA] | 95 | 902.4 | 902.5 [M+2H]²⁺ | 2 |
| **B-o** | Cyc | **-** | [βA V T R C D V p P D S C L V F βA] | 95 | 837.9 | 838.0 [M+2H]²⁺ | 4 |
| **B-ox** | Bicyc | (disulfide) | [βA V T R [C D V p P D S C] L V F βA] | 86 | 836.9 | 837.0 [M+2H]²⁺ | <1 |
| **B-b1** | Bicyc | **e1** | [βA V T R [C D V p P D S C] L V F βA] | >95 | 843.9 | 844.1 [M+2H]²⁺ | <1 |
| **B-b2** | Bicyc | **e2** | [βA V T R [C D V p P D S C] L V F βA] | >95 | 850.9 | 850.9 [M+H]²⁺ | 2 |
| **B-b3** | Bicyc | **e3** | [βA V T R [C D V p P D S C] L V F βA] | >95 | 857.9 | 858.0 [M+2H]²⁺ | 2 |
| **B-b4** | Bicyc | **e4** | [βA V T R [C D V p P D S C] L V F βA] | >95 | 863.9 | 864.0 [M+2H]²⁺ | 1 |
| **B-b5** | Bicyc | **e5** | [βA V T R [C D V p P D S C] L V F βA] | >95 | 864.9 | 865.0 [M+2H]²⁺ | 1 |
| **B-b6** | Bicyc | **e6** | [βA V T R [C D V p P D S C] L V F βA] | 95 | 863.9 | 864.0 [M+2H]²⁺ | 1 |
| **B-b7** | Bicyc | **e7** | [βA V T R [C D V p P D S C] L V F βA] | >95 | 888.9 | 889.1 [M+2H]²⁺ | 4 |
| **C-o** | Cyc | - | [βA V T R N C V p P D C L L V F βA] | >95 | 850.4 | 850.6 [M+2H]²⁺ | 6 |
| **C-ox** | Bicyc | (disulfide) | [βA V T R N [C V p P D C] L L V F βA] | >95 | 849.4 | 849.6 [M+2H]²⁺ | 3 |
| **C-b1** | Bicyc | **e1** | [βA V T R N [C V p P D C] L L V F βA] | >95 | 856.4 | 856.6 [M+2H]²⁺ | 3 |
| **C-b2** | Bicyc | **e2** | [βA V T R N [C V p P D C] L L V F βA] | >95 | 863.4 | 864.0 [M+2H]²⁺ | 2 |
| **C-b3** | Bicyc | **e3** | [βA V T R N [C V p P D C] L L V F βA] | >95 | 870.4 | 870.6 [M+2H]²⁺ | 1 |
| **C-b4** | Bicyc | **e4** | [βA V T R N [C V p P D C] L L V F βA] | 95 | 876.5 | 876.6 [M+2H]²⁺ | 2 |
| **C-b5** | Bicyc | **e5** | [βA V T R N [C V p P D C] L L V F βA] | >95 | 877.5 | 877.6 [M+2H]²⁺ | 2 |
| **C-b6** | Bicyc | **e6** | [βA V T R N [C Vp PDC] L L V F βA] | 95 | 876.5 | 876.6 [M+2H]²⁺ | 2 |
| **C-b7** | Bicyc | **e7** | [βA V T R N [C V p P D C] L L V F βA] | 95 | 901.5 | 901.6 [M+2H]²⁺ | 4 |
| **St-1** | Bicyc* | | [βA V [S5* R N D V p P D S L L **S5*]** F βA] | ∼90 | 859.5 | 859.6 [M+2H]²⁺ | <1 |
| **St-2**^{#} | Bicyc* | | [βA V [**S5*** R N D V p P D S L L **S6*]** F βA] | ∼90 | 866.5 | 866.6 [M+2H]²⁺ | <1 |
| **St-3**^{#} | Bicyc* | | [βA V **[S6*** R N D V p P D S L L **S5*]** F βA] | ∼90 | 866.5 | 866.6 [M+2H]²⁺ | <1 |
| **St-4** | Bicyc* | | [βA V **[S6*** R N D Vp P D S L L **S6*]** F βA] | ∼90 | 873.5 | 873.6 [M+2H]²⁺ | <1 |
| **St-5** | Bicyc* | | [βA V T R **[S5*** D V p P D S **S5*]** L V F βA] | ∼90 | 846.0 | 846.1 [M+2H]²⁺ | <1 |
| **St-6**^{#} | Bicyc* | | [βA V T R N **[S5*** V p P D **S5*]** L L V F βA] | ∼90 | 858.5 | 858.7 [M+2H]²⁺ | 1 |

| | | | | | | | |
|---|---|---|---|---|---|---|---|
| #chromatogram shows cis and *trans* isomers. | | | | | | | |

Cysteine bridging with diiodomethane (A-b1, B-b1, C-b1), derived from [Kourra and Cramer, 2016. Chem Sci 7: 7007-7012]. Monocyclic peptide A-o (1 µmol), B-o (1 µmol) or C-o (1 µmol) was dissolved in THF (tetrahydrofuran) / water (1:1, v/v) to a final concentration of 1 mM. Under vigorous stirring, 1.5 eq. TCEP·HCl (tris(2-carboxyethyl) phosphine hydrochloride), 15 eq. TEA (triethylamine) and 10 eq. CH₂I₂ (e1) were added. The reaction was stirred for 24 h at room temperature. Subsequently, the solution was diluted with H₂O/ACN (1:1, v/v). Crude peptides were purified by preparative HPLC and characterized by analytical LC-MS (section 1.1). Peptides were obtained as white powders (A-b1: 15%, B-b1: 7%, C b1: 51%). Overall yields of peptide synthesis and peptide purities can be found in Table 2.

Cysteine bridging with dibromoalkanes (A-b2, A-b3, A-b5, B-b2, B-b3, B-b5, C-b2, C-b3, C-b5). Monocyclic peptide A o (1 µmol), B-o (1 µmol) or C-o (1 µmol) was dissolved in THF/water (1:1, v/v) to a final concentration of 1 mM. Under vigorous stirring 1.5 eq. TCEP·HCl, 75 eq. TEA and 50 eq. 1,2-dibromoethane (e2), 1,3-dibromopropane (e3) or 1,4-dibromobutane (e5) were added. The reactions were stirred at room temperature for 48 h, quenched by dilution with H₂O, purified by preparative HPLC and characterized by analytical LC-MS (section 1.1). Pure peptides were obtained as white powders (A-b2: 25%, A-b3: 14% A-b5: 7%, B-b2: 23%, B-b3: 27% B-b5: 7%, C-b2: 29%, C b3: 21% C-b5: 44%). Overall yields of peptide synthesis and peptide purities can be found in Table 2.

Cysteine bridging with 3-bromo-2-bromomethyl-1-propene (A-b4, B-b4, C-b4), derived from [Kale et al., 2018. Nat Chem 10: 715-723]. To a solution of 1 mM monocyclic peptide A-o (1 µmol), B-o (1 µmol) or C-o (1 µmol) in ACN and 100 mM ammonium carbonate buffer pH 9 (1:1, v/v), 4 eq. 3-bromo-2-bromomethyl-1-propene (e4) were added from a 100 mM stock solution in ACN. The reaction was stirred for 3 h at 30 °C and subsequently diluted with water. Crude peptides were purified by preparative HPLC and characterized by analytical LC-MS (section 1.1). Peptides were obtained as white powders (A-b4: 64%, B-b4: 17% C b4: 38%). Overall yields of peptide synthesis and peptide purities can be found in Table 2.

Cysteine bridging with trans-1,4-dibromobut-2-ene (A-b6, B-b6, C-b6), derived from [Kale et al., 2018. Nat Chem 10: 715-723]. To a solution of 1 mM monocyclic peptide A-o (1 µmol), B-o (1 µmol) or C-o (1 µmol) in ACN and 100 mM ammonium carbonate buffer pH 9 (1:1, v/v) 4 eq. trans-1,4-dibromobut-2-ene (e5) were added from a 100 mM stock solution in ACN. The reactions were stirred for 2 h at 30 °C and diluted with H₂O. Crude peptides were purified by preparative HPLC and characterized by analytical LC-MS (section 1.1). Peptides were obtained as white powders (A-b6: 24%, B-b6: 14% C-b6: 37%). Overall yields of peptide synthesis and peptide purities can be found in Table 2.

Cysteine bridging with α,α'-dibromo-o-xylene (A-b7, B-b7, C-b7), derived from [Kale et al., 2018. Nat Chem 10: 715-723]. Monocyclic peptide A-o (1 µmol), B-o (1 µmol) or C-o (1 µmol) was dissolved in ACN and 100 mM ammonium carbonate buffer pH 9 (1:1, v/v) to a final concentration of 1 mM. 4 eq. α,α'-dibromo-o-xylene (e7) were added from a 100 mM stock solution in ACN. After stirring the reaction for 1 h at 30 °C, the reaction was diluted with H₂O. Crude peptides were purified by preparative HPLC and characterized by analytical LC-MS (section 1.1). Peptides were obtained as white powders (A-b7: 30%, B-b7: 45%, C-b7: 60%). Overall yields of peptide synthesis and peptide purities can be found in Table 2.

### 4 Fluorescein labeling of peptides 12 and A-b6

The procedure is derived from [Roxin et al., 2012. Bioconjug Chem 23: 1387-1395] Precursors of fluorescently labeled peptides 12 and A-b6 were prepared as described in sections 2 and 3. Amino acid N631 was substituted with lysine (K631), enabling the labelling with fluorescein after cyclization or bicyclization. Purified peptide 12(K631) precursor (290 nmol) or A-L6(K631) precursor (100 nmol) were dissolved in 4% DIPEA in DMF (v/v) to a final concentration of 0.1 mM. From a 1 mM DMSO stock solution 1.5 eq. 5/6-carboxyfluorescein succinimidyl ester (ThermoFisher Scientific, 46409) were added to the peptide solution. The reaction was vigorous stirred for 3 h. Labeled peptide was precipitated with the addition of 30 mL diethyl ether and separated by centrifugation (4000 rcf, 10 min). The supernatant was discarded, and the pellet dissolved in water and ACN (1:1, v/v). Crude peptides were purified and characterized as described in section 1.1. Pure peptides were obtained as yellow powders (12(K631-Fl): 53%, A L6(K631 Fl): 3%). Overall yields of peptide synthesis and peptide purities can be found in Table 2.

### 5 Protein expression and purification

### 5.1 Full-length β-catenin

Protein expression and purification of full length β-catenin (aa 1 - 781) was based on an earlier published protocol [Dietrich et al., 2017. Cell Chem Biol 24: 958-968]. Briefly, *Escherichia coli* (*E. coli*) BL21(DE3) cells were transformed with a pET-28a(+) vector carrying the coding sequence for β-catenin and a C-terminal 3C protease recognition site followed by a His6-tag. A pre-culture of transformants was incubated overnight at 37 °C in TB-medium supplemented with 50 µg mL⁻¹ Kanamycin. Subsequently, 2 L of expression culture (TB-medium + 50 µg mL⁻¹ Kanamycin) were inoculated with the pre-culture to an optical density at 600 nm (OD600) of 0.1. Cells were incubated at 37 °C and induced with 0.5 mM IPTG (isopropyl-β-D-thiogalactoside) at an OD600 of 1.0. Afterwards, the expression culture was incubated at 20 °C for 16 h under agitation at 150 rpm. Subsequently, the cells were harvested (4 °C, 20 min, 4000 rpm, JA8.100 rotor Beckman Coulter) and resuspended in lysis buffer 1 (50 mM TRIS pH 8.0, 300 mM NaCl, 10 mM imidazol, 5% glycerol, 2 mM β-mercaptoethanol, 100 µM phenylmethylsulfonyl fluoride) with approx. 5 mg of DNAse and approx. 5 mg of lysozyme. Cells were lysed using a LM10 microfluidizer at 18000 psi and cell debris were separated by centrifugation (4 °C, 40 min, 21000 rpm, JA25.50 rotor Beckman Coulter). The supernatant was loaded on an affinity chromatography column (His-Trap FF crude, 5 mL, GE Healthcare) with a flow rate of 1 mL min⁻¹. A total amount of 10 CV (column volumes) wash buffer 1 (50 mM TRIS pH 8, 500 mM NaCl, 25 mM imidazol, 5% glycerol, 1 mM β-mercaptoethanol) with a flow rate of 2 mL min⁻¹ was applied. β-catenin was then eluted using 5 CV of elution buffer 1 (50 mM TRIS pH 8.0, 200 mM NaCl, 250 mM imidazol, 5% glycerol 1 mM β-mercaptoethanol). Afterwards, β-catenin was injected into a size exclusion chromatography (SEC, HiLoad 16/600 Superdex 200 pg, GE Healthcare) system using SEC buffer 1 (20 mM TRIS pH 8.0, 150 mM NaCl, 10% glycerol 5 mM β mercaptoethanol) and a flow rate of 1 mL min⁻¹. Protein containing fractions were pooled, β-catenin was concentrated (MWCO = 30 kDa) to 3.7 mg mL⁻¹, snap frozen in liquid nitrogen and stored at -80 °C (yield: 30 mg L⁻¹ expression culture).

### 5.2 β-catenin (aa 134 - 665)

*E. coli* BL21(DE3) cells were transformed with a pGEX-4T-1 vector carrying the coding sequence for β-catenin (aa 134 - 665) including the coding sequence for a N-terminal GST-tag followed by a TEV (Tobacco Etch Virus) protease cleavage site. A pre-culture of transformants was incubated at 37 °C overnight in TB-medium supplemented with 100 µg mL⁻¹ Ampicillin. Subsequently, 2 L of expression culture (TB-medium + 100 µg mL⁻¹ Ampicillin) was inoculated to an OD600 of 0.1. Cells were incubated at 37 °C and induced with 0.5 mM IPTG upon reaching an OD600 of 1.0. Cells were subsequently incubated at 20 °C for 16 h under agitation at 150 rpm. Subsequently, cells were harvested by centrifugation (4 °C, 20 min, 4000 rpm, JA8.100 rotor Beckman Coulter) and resuspended in lysis buffer 2 (50 mM TRIS pH 8.5, 500 mM NaCl, 5% glycerol, 2 mM DTT, 100 µM phenylmethylsulfonyl fluorid) with approx. 5 mg of DNAse and approx. 5 mg of lysozyme. Cells were lysed using a LM10 microfluidizer at an operational pressure of 18000 psi and cell debris were separated by centrifugation (4 °C, 40 min, 21000 rpm, JA25.50 rotor Beckman Coulter). The supernatant was loaded on an affinity chromatography column with a flow rate of 1 mL min⁻¹ (GSTPrep FF 16/10 Column, 20 mL, GE Healthcare). Subsequently, the column was washed with 3 CV wash buffer 2 (50 mM TRIS pH 8.5, 300 mM NaCl, 10% glycerol, 2 mM DTT) at a flow rate of 2 mL min⁻¹. For proteolytic cleavage of the GST-tag, 1 CV of wash buffer 2, including TEV protease (0.15 mg mL⁻¹) was continuously circulated over the column (4 °C, 16 h, 1 mL min⁻¹). Cleaved proteins were eluted with 1.5 CV wash buffer 2. Afterwards, β-catenin (aa 134 - 665) was injected into a SEC (HiLoad 16/600 Superdex 75 pg, GE Healthcare) using SEC buffer 2 (20 mM TRIS pH 8.5, 150 mM NaCl, 0.5% Glycerol, 2 mM DTT) and a flow rate of 1 mL min⁻¹. Protein containing fractions were pooled, β-catenin (aa 134-665) was concentrated (MWCO = 30 kDa) to 1.7 mg mL⁻¹, snap frozen in liquid nitrogen and stored at -80 °C (yield: 42 mg L⁻¹ expression culture).

### 6 Crystallization of β-catenin (aa 134 - 665)/peptide 12 complex

### 6.1 Crystal growth

β-Catenin (aa 134 - 665) was diluted with SEC buffer 2 (20 mM TRIS pH 8.5, 150 mM NaCl, 0.5% glycerol, 2 mM DTT) to 6.6 µM. Peptide 12 was dissolved to 1.8 mM in SEC buffer 2. Two equivalents of peptide 12 were added to the protein solution. The protein-peptide complex solution was washed with 2.5 times the initial volume of SEC buffer 2 using a centrifugal concentrator (MWCO = 10 kDa, Vivaspin^{®}500). Afterwards, the complex was concentrated to 2.2 mg mL-1 and used for crystallization in 24-well XRL-plates (Molecular Dimensions) in a hanging-drop vapor diffusion experiment with a drop ratio of 1 µL protein-peptide complex solution and 1 µL precipitant solution (100 mM HEPES pH 7.0, 4% PEG6000). The reservoir was filled with 700 µL of precipitant solution and the sealed plate was incubated at 20 °C. Crystal formation was observed after 24 h. Prior to measurement, crystals were stored in precipitant solution containing additional 30% glycerol.

### 6.2 Structure determination

Crystals were measured at the 104 beamline at the Diamond Light Source (DLS) at 100 K. The obtained diffraction images were processed with XDS [Kabsch, 2010. Acta Cryst D 66: 133-144]. The data set was used until 2.6 Å. The protein-peptide structure was solved by molecular replacement using Phaser [McCoy, 2007. Acta Cryst D 63: 32-417] and a search model derived from PDB ID 1jdh. Iterative rounds of model building and refinement were performed with COOT[8] and Refmac5 [Murshudov et al., 2011. Acta Cryst D 67: 355-367]. Geometrical model constraints for non-natural amino acids were obtained from the CCP4 monomer library [Winn et al., 2011. Acta Cryst D 67: 235-242]. Additional restraints for the remaining bonds within peptide 12 were generated with Acedrg [Long et al., 2017. Acta Cryst D 73: 112-122]. Collection- and refinement statistics are listed in Table 3.

Pre-screening measurements of bicyclic peptides were performed at a concentration of 30 µM peptide competitor, 10 nM labeled TCF-4 peptide and 250 nM β-catenin (aa 1 - 781) in a 384-well plate (Corning, Ref. 4515). Fluorescence polarization values were measured at room temperature (Tecan Spark 20M, λEx = 470 nm, λEm = 525 nm) in singlets after an incubation time of 1 h. Obtained values were normalized for 0% complex inhibition (no peptide competitor) and 100% complex inhibition (E-CBD activity at 30 µM).

**Table 3. Crystallographic table of the crystal structure of β-Catenin (aa 134 - 665) in complex with peptide 12 (PDB ID 7ar4).**

| **Structure** | **β-Catenin (aa 134 - 665) / peptide 12 (PDB ID 7ar4)** |
|---|---|
| **Data collection** | DLS (Beamline 104)/ DECTRIS EIGER2 X 16M |
| Space group | P2₁2₁2₁ / 19 |
| Cell dimensions | |
| a, b, c / Å | 71.38, 85.62, 142.71 |
| α, β, γ / ° | 90.00 90.00 90.00 |
| Wavelength / Å | 0.979507 |
| Resolution limits / Å | 85.62 - 2.60 (2.70 - 2.60) |
| No. of unique reflections | 27652 (2886) |
| Completeness / % | 100 (100) |
| Multiplicity | 13.36 (14.23) |
| I / σI | 12.83 (1.11) |
| CC_{1/2} | 0.999 (0.584) |
| R_{obs} | 0.164 (2.901) |
| Rₘₑₐₛ | 0.171 (3.009) |

| **Refinement** | refmac 5.8.0258 |
|---|---|
| Resolution limits / Å | 85.62 - 2.60 |
| R_{work} / R_{free} | 0.2026/0.2419 |
| R.m.s. deviations | |
| Bond lengths / Å | 0.006 |
| Bond angles / ° | 1.423 |
| B-factor / Å² | 72.408 |
| No. atoms (non-hydrogen) | 4084 |
| Protein | 3902 |
| Peptide | 120 |
| Ligand/Ion | 16 |
| Water | 46 |
| Ramachandran | |
| Favoured Regions / % | 96.3 |
| Allowed Regions / % | 3.1 |
| Outliers / % | 0.6 |

### 7 Fluorescence polarization assays

### 7.1 Competition of the β-catenin/TCF-4 interaction

Peptides were dissolved in DMSO (5 mM). Monocyclic peptides A-o, B-o and C-o were dissolved in FP buffer (20 mM TRIS pH 8.0, 150 mM NaCl, 10% glycerol, 5 mM β-mercaptoethanol, 0.01% Tween-20).

Peptide competitors were titrated in a 384-well plate (Corning, Ref. 4515) using FP buffer. In a separate vessel, a solution of 1µM β-catenin (aa 1 - 781) and 40 nM FITC-labeled TCF-4 peptide was prepared in FP buffer and incubated on ice for 30 min. Subsequently, the protein-peptide complex was added to the titration series of peptide competitor with a final concentration of 10 nM TCF-4 peptide, 250 nM β-catenin (aa 1-781) and 7.9 10⁻³ µM to 187.5 µM peptide competitor. After an incubation time of 1 h at room temperature, the fluorescence polarization (FP) values were measured at room temperature (Tecan Spark 20M, λ_{Ex} = 470 nm, λ_{Em} = 525 nm). Measurements were conducted in triplicates. For data analysis, the discrepancy between free tracer TCF-4 and TCF 4/peptide competitor in solution was determined and subtracted. Data points were fitted using a non-linear regression fit in Prism 5.0 (Graphpad).

### 7.2 Competition of the β-catenin/E-CBD interaction

In a 384-well plate non-labelled peptide competitor was titrated in FP-buffer (20 mM TRIS pH 8.0, 150 mM NaCl, 10% glycerol, 5mM β-mercaptoethanol, 0.01% Tween-20). In a separate vessel, a solution of 1 µM β-catenin (aa 1 - 781) and 40 nM FITC-labeled E-CBD peptide was prepared in FP buffer and incubated on ice for 30 min. Subsequently the protein-peptide complex was added to the titration series of peptide competitor with a final concentration of 10 nM E-CBD peptide, 250 nM β-catenin (aa 1 - 781) and 3 · 10⁻⁵ M to 2.01 · 10⁻¹⁰ M peptide competitor. Thereafter, the fluorescence polarization (FP) values were measured at room temperature (Tecan Spark 20M, λ_{Ex} = 470 nm, λ_{Em} = 525 nm). Measurements were conducted in triplicates. Data points were fitted using a non-linear regression fit in Prism 5.0 (Graphpad).

### 7.3 Direct binding of peptides to β-catenin

β-Catenin (aa 1-781) was serially diluted in FP buffer (20 mM TRIS pH 8.0, 150 mM NaCl, 10% glycerol, 5 mM β-mercaptoethanol, 0.01% Tween-20) on a 384-well plate (Corning, Ref. 4515). Fluorescently labeled peptides (100 µM DMSO stock solutions) were diluted in FP buffer to 40 nM and subsequently added to the dilution series of β-catenin (aa 1 - 781) to a final concentration of 10 nM peptide and 7 · 10⁻⁵ µM to 12.6 µM β-catenin. After incubation for 1 h on ice, FP values were measured at room temperature (Tecan Spark 20M, λ_{Ex} = 470 nm, λ_{Em} = 525 nm). All measurements were conducted in triplicates. For data analysis FP values were fitted using a non-linear regression in Graphpad's Prism 5.0.

### 8 Circular dichroism

Peptides were dissolved in CD buffer (5 mM sodium phosphate, pH 7.5) to a final concentration of 75µM. Measurements were performed using a Jasco CD spectrometer (J-1500) and quartz cuvette (1 mm pathlength, Hellma) at 20 °C. Spectra were recorded in 10 continuous scans at a scanning speed of 100 nm min⁻¹ (1 mdeg sensitivity, 0.5 nm resolution, 1.0 nm bandwidth, 2 s integration time). From each measurement, a buffer blank was subtracted and obtained ellipticity (mdeg) was transformed to mean residue ellipticity (MRE / deg cm² dmol⁻¹).

### 9 Reporter gene assay

HEK293T cells were cultured in high glucose, GlutaMAX^{™}, pyruvate DMEM medium (Thermofisher Cat# 31966021) supplemented with 10% Fetal Bovine Serum (Thermofisher Cat# A3160801) and Penicillin-Streptomycin (Thermofisher No. 15140148). Cells were seeded into a 96-well plate coated with 100 mg mL⁻¹ Poly-L-Lysine (Sigma No. A-005-C). 24 h after plating, cells were transfected with a M50 Super 8x TOPFlash β-catenin luciferase reporter plasmid, a gift from Randall Moon (Addgene plasmid #12456, http://n2t.net/addgene:12456, RRID: Addgene_12456) [Veeman et al., 2003. Curr Biol 13: 680-68512] and a pRL-TK renilla reporter plasmid (Promega No. E2241) for normalization, mixed at a 1:1 ratio in Opti-MEM^{™} I Reduced Serum Medium (Thermofisher No. 31985062), using X-tremeGENE^{™} 9 DNA Transfection Reagent (Sigma No. 6365779001) according to manufacturer's instructions. 24 h after transfection, the cell culture medium was replaced with fresh medium supplemented with 100 ng mL⁻¹ Wnt-3a (R&D Systems 5036-WN-10) reconstituted in PBS with 0.1% BSA or without Wnt-3a (No Wnt condition). Immediately after medium change, peptides, XAV939 (positive control) or DMSO (vehicle) were added and cells were incubated for 24 h prior to luciferase activity measurements. Luciferase assays were performed with a Dual-Glo^{®} Luciferase Assay System (Promega No. E2920) according to manufacturer's instructions using a CLARIOStar Plus microplate reader (BMG Labtech). Luciferase to renilla raw counts ratios were then used to calculate the relative response ratios using the No Wnt + DMSO-treated cells ratios as the uninduced reference and the Wnt + DMSO-treated cells ratios as the fully induced reference.

### RESULTS

### E-cadherin derived peptides bind β-catenin

β-Catenin consists of 781 amino acids and comprises a central armadillo repeat domain (aa 141-664), which is flanked by flexible N- and C-terminal regions. The armadillo repeats serve as interaction hub recognizing several protein binding partners. [Hahne and Grossmann, 2013. Bioorg Med Chem 21: 4020-4026]. The family of TCF transcription factors is among those binders and shares homologous β-catenin binding domains (CBDs) that bind a central region of the armadillo repeat domain. [Poy et al., 2001. Nat Struct Biol 8: 1053-1057; Graham et al., 2001. Nat Struct Biol 8: 1048-1052; Sampietro et al., 2006. Mol Cell 24: 293-300]. These CBDs usually encompass an α-helix and an extended sequence which are connected via a flexible crosslink (CBD of TCF-4, Figure 1a). The extended region includes a short β strand with two crucial hot-spot amino acids (D16 and E17 in TCF-4) [Knapp et al., 2001. J Mol Biol 306: 1179-1189] that bind β-catenin on the so-called 'binding site 3' [Hahne and Grossmann, 2013. Bioorg Med Chem 21: 4020-4026].

Herein, we aimed to design a ligand capable of targeting β-catenin binding site 3 to compete with TCF binding. When inspecting available β-catenin complex structures, we noticed that the CBD of E-cadherin, a protein that recruits β-catenin to cell-cell adhesions junctions, includes a small antiparallel β sheet which also binds to β-catenin's binding site 3 (Figure 1a). Initially, we considered a 52 aa sequence (E-CBD, aa 628-679, Figure 1a) that encompasses an α-helix (α1, aa 652 - 665) and the two antiparallel B-strands (β1: aa 628 - 633; β2: aa 674 - 679) including intervening crosslink regions. Notably, two of the five predicted hot-spots of E-CBD (D674 and L676) [Krüger and Gohlke, 2010. Nucleic Acids Res 38: W480-W48623] are located within strand β2 (Figure 1a). A fluorescently labeled version of E-CBD was synthesized with the one methionine (M638) present substituted by the chemically inert analogue norleucine. A fluorescence polarization (FP) assay applying full length β-catenin revealed a high binding affinity for E-CBD (dissociation constant (Kd) = 0.053 µM). Keeping β2 as the anchor point, a series of N-terminally truncated and labeled E CBD versions was synthesized (Figure 1b). While the deletion of 7 or 13 amino acids (peptides 1 and 2) only moderately affected affinity for β-catenin (Kd = 0.13 and 0.2 µM, respectively), a truncation to 28-mer 3 severely reduced binding (Kd approx. 3 µM). Seeking a short peptide sequence suitable for subsequent stabilization via chemical modification, we aimed to place strand β2 in the center of the primary peptide sequence, thereby facilitating an alternative truncation pattern. For that purpose, we took advantage of the spatial proximity of the C- and N-terminus in E-CBD (F679 and V628, respectively, d = 6.5 Å, Figure 1a), which we aimed to covalently link. In this alternative arrangement, the new peptide termini were generated by opening the sequence between Y643 (new N terminus) and Q642 (new C-terminus, Figure 1a). Between V628 and F679, different linker structures were considered. The D-proline-L-proline (DP-LP) [Robinson, 2013. Chimia 67: 885-8906; Nair et al., 1979. J Chem Soc Chem Commun 1183-1184; Späth et al., 1998. Helv Chim Acta 81: 1726-1738] motif was introduced in combination with an additional glycine (peptide 4) or β alanine (peptide 5) to align the two termini. Both peptides exhibited low affinities for β-catenin (in both cases: K_{d} approx. 2 µM). The incorporation of two β-alanines yielded peptide 6 with only mildly reduced affinity (Kd = 0.18 µM) when compared to E CBD (Kd = 0.053 µM). The introduction of three β alanine residues (peptide 7) resulted in a lower affinity (Kd = 0.38 µM). Using peptide 6 as a starting point, a series of truncated peptides was synthesized (Figure 1c). While the deletion of the N-terminal crosslink (6 aa) was tolerated (peptide 8, K_{d} = 0.053 µM), helix a1 appeared to be essential for binding (peptide 9, K_{d} > 5 µM). On the C-terminus, the deletion of the crosslink (9 aa) moderately reduced binding (peptide 10, K_{d} = 0.6 µM). As expected, the combination of the truncations in 9 and 10 resulted in a peptide with very low affinity (peptide 11, K_{d} > 5 µM). These truncation studies indicate that linear versions of the E-cadherin CBD require about 40 aa for sub-micromolar binding affinities.

### A cyclic peptide inhibits the β-catenin/TCF-4 interaction

Aiming for a smaller binder that includes strand β2 and would therefore target binding site 3 (Figure 1a), we considered macrocyclization of peptide 11. When assuming a binding mode analogous to the CBD of E cadherin, the termini of peptide 11 would be in proximity (V633 and D674, d = 5.2 Å). Aiming for the stabilization of the antiparallel β-sheet in the bound state, the DP-LP turn mimetic [Robinson, 2013. Chimia 67: 885-8906; Nair et al., 1979. J Chem Soc Chem Commun 1183-1184; Späth et al., 1998. Helv Chim Acta 81: 1726-1738] was incorporated to provide macrocycle 12 (Figure 2a). Initially, a linear precursor of 12 (H₂N-βA-VTRNDV-*D*P-*L*P-DSLLVF-βA-OH) was synthesized on solid-support using 2-chlorotrityl chloride resin. After cleavage, the side-chain-protected peptide was head-to-tail cyclized in solution. Then all protecting groups were removed, and after purification, macrocycle 12 was obtained in overall yields of about 10%. To evaluate the binding of 12, a competition FP assay was performed. Briefly, full-length β-catenin and a fluorescently labeled TCF-4-derived tracer peptide [Grossmann et al., 2012. Proc Natl Acad Sci USA 109: 17942-17947] (Figure 2e) were incubated with varying concentrations of competitor to provide half-maximal inhibitory concentrations (IC₅₀, Figure 2b). In this assay, macrocycle 12 showed good inhibition (IC₅₀ = 16 µM), being only threefold less potent than the 52 mer peptide E-CBD (IC₅₀ = 5.4 µM). In comparison, linear 28-mer peptide 3 (IC₅₀ approx. 100 µM) and a linear version of 12 lacking the β-alanine linker (peptide 12-l, IC₅₀ > 100 µM) showed greatly diminished competition.

To investigate the binding mode of peptide 12, co-crystallization with the armadillo repeat domain of β-catenin (aa 134 - 665) was pursued. After obtaining crystallization conditions from initial screening, crystals were optimized and analyzed by X-ray diffraction. We collected a dataset and included reflections up to 2.6 Å resolution. The structure was solved in space group P212121, (PDB ID 7ar4, Table 3) using molecular replacement with β-catenin as the search model (derived from PDB ID 1jdh). The obtained crystal structure shows one copy of the β-catenin armadillo repeat domain per asymmetric unit (data not shown). Except for one crosslink (aa 550-559), protein residues are well-resolved and superimpose closely with previously reported structures (PDB ID 1jdh and 2gl7). At binding site 3 of β-catenin , well-defined electron density was observed matching the molecular structure of macrocycle 12 (Figure 2c). In complex with β-catenin, 12 closely resembles the conformation of the two antiparallel strands β1 and β2 of E cadherin's CBD (Figure 2d, for Ramachandran plot of 12). This β hairpin is stabilized by six intramolecular hydrogen bonds within the β-sheet (two between each of the following aa pairs: T629/V678, N631/L676 and V633/D674, Figure 2c) and one additional hydrogen bond within the β alanine turn. The crystal structure also revealed that predicted E-CBD hot spot residues (L676, D674) closely overlay with the corresponding side chains in macrocycle 12 forming analogous contacts with β-catenin. Consequently, the binding site of 12 overlaps also with the hot-spot region of TCF (Figure 1a) which is in line with the observed inhibition of TCF-4 binding (Figure 2b).

### Bicyclization of β-hairnin structures

Utilizing the structure of macrocycle 12 in complex with β-catenin, the design of more constrained β hairpin structures was pursued. We aimed for the introduction of a cross-strand bridge and identified three potentially suitable amino acid pairs located in the core of the β-hairpin (A: T629/V678, B: N631/L676, C: D632/S675, Figure 3a). To allow the installation of different bridges at a late stage of the synthesis, we decided to introduce two cysteines which, after the initial head-to-tail cyclization, are reacted with a bis-electrophile (Figure 3b). This crosslinking chemistry proved already useful for the cyclization of α-helices and crosslink structures [Jo et al., 2012. J Am Chem Soc 134: 17704-17713; Timmerman et al., 2005. Chembiochem 6: 821-824; Kowalczyk et al., 2012. Bioorg Med Chem 20: 2661-2668; Fairlie and Dantas de Araujo, 2016. Biopolymers 106: 843-852; Heinis et al., 2009. Nat Chem Biol 5: 502-507]. A small library of bis-electrophiles (e1- e7, Figure 3b) was assembled resulting in bridge structures that ranged from one to four bridging atoms between the cysteines (b1 - b7, Figure 3c). Bridge b1, b2, b3 and b5 are fully saturated, while b4 and b6 include a double bond. Bridge b7 harbors the aromatic ortho-xylene moiety (Figure 3c). The monocyclic double-cysteine precursors (A-o, B-o and C-o) were synthesized in analogy to macrocycle 12, and subsequently reacted with each of the bis-electrophiles (e1 - e7). Conditions for this bicyclization reaction were adjusted to the reactivity of the corresponding bis-electrophile. While e4, e6 and e7 reacted rapidly in ammonium carbonate buffer and acetonitrile (1:1, v/v), the bicyclization with e1, e2, e3 and e5 was performed in a mixture of water and tetrahydrofuran (1:1, v/v) with large excess of the bis-electrophile (10 - 50 eq.) and triethylamine (15-75 eq.). In addition, the three disulfide-bridged derivatives (A-ox, B-ox and C-ox) were obtained by oxidation. All peptides were purified via HPLC providing product yields between 7 % and 64 % for the final bicyclization.

The generated 24 bicycles and 3 monocycles were subsequently assessed for their ability to inhibit the TCF-4/β-catenin interaction. Using the FP competition assay described above, all derivatives were screened at a concentration of 30 µM (Figure 3c, Table 4). For series A and C, we observed similar trends with low competition capabilities (< 25%) for the monocycles (A o, C-o) as well as the bicyclic derivatives with short cross-strand bridges (b1 and b2). For longer bridges, inhibitory potency increased with the highest activities observed for bicycles with four bridging atoms (A-b5, A-b6, A-b7 and C-b5, C-b7). The B-series exhibited overall lower competition without a clear trend regarding changes in the lengths of the cross-strand bridge. Subsequently, the concentration-dependent effect of the most active peptides (inhibition >50%, A-b5, A-b6, A-b7 and C-b5, C-b7) was investigated (Figure 3d and 3e). The two monocyclic peptides A-o and C-o showed considerably lower activity (IC₅₀ approx. 100 µM and below) than macrocycle 12 (IC₅₀ = 16 µM) indicating that cysteine variation affects binding of β-catenin. Notably, all selected bicycles showed efficient inhibitory activity exceeding that of macrocycle 12. For the tested A-series bicycles, obtained IC₅₀-values indicated a low dependency on the degree of saturation within the bridge (IC₅₀ = 8.5-11 µM for A-b5, A-b6 and A-b7). From all tested bicycles, ortho-xylene bridged C-b7 exhibited the highest inhibitory activity (IC₅₀ = 4.8 µM), which is comparable to the 52-mer peptide E-CBD (IC₅₀ = 5.4 µM).

To test the possibility of structurally different bridges, hydrocarbon connectors were included in series A (St-1, St-2, St-3, St4), B (St-5) and C peptides (St-6). For that purpose, connectors with different length were applied including eight (St-1, St-5, St-6) and nine carbon atoms (St-3, St-4). In a FP competition assay involving a fluorescently labelled version of E-CBD and β-catenin (Figure 4), St-3 showed highest completion (IC₅₀ = 3.8 µM) surpassing the activity of monocycle 12 in this assay (IC₅₀ = 4.4 µM).

### Bicyclic β-sheet mimetics inhibit Wnt signaling

Since some of the library members show inhibition of the TCF-4/β-catenin interaction in biochemical assays, we were interested if this also translates into the inhibition of the Wnt signaling pathway in a cellular context. Therefore, we chose the TOPFLASH reporter gene assay [Veeman et al., 2003. Curr Biol 13: 680-685] for activity testing which is sensitive to activated Wnt signaling and should respond to the inhibition of the TCF/β-catenin interaction. Briefly, the TOPFLASH reporter plasmid [Veeman et al., 2003. Curr Biol 13: 680-685] encoding a firefly luciferase gene under control of a TCF/β-catenin -dependent promoter, and a Wnt-independent control reporter plasmid encoding a renilla luciferase were co-transfected into HEK293T cells. The Wnt signaling pathway was activated by incubation with recombinant Wnt-3a protein ligand in the presence of inhibitor (t = 24 h). Wnt reporter activity was calculated based on the subsequently measured firefly and renilla luciferase signals: firefly/renilla ratios were normalized with respect to the absence and presence of Wnt-3a (0% and 100% rel. reporter activity, respectively). To verify assay functionality, we tested the known upstream Wnt pathway inhibitor XAV939 which promotes β-catenin degradation [Sakata and Chen, 2011. Chem Soc Rev 40: 4318-4331] and indeed shows the expected reduction in reporter activity (30% rel. reporter activity, Figure 5a). Initially, all series A, B and C bicycles with four-atom bridges (b5, b6 and b7) were tested (c = 20 µM,t = 24 h). Notably, the Wnt-independent renilla activity was only mildly affected (data not shown) indicating that HEK293T cells tolerate this treatment. Interestingly, macrocycle 12 did not display a meaningful inhibition of Wnt reporter activity, while considerable inhibition was observed for the bicycles of series A (A-b5, A-b6, and A-b7, Figure 5a). However, none of the series B and C derivatives showed significant reduction of reporter activity (Figure 5a). Among the tested bicycles, the highest inhibitory activity was observed for butene-bridged A-b6 (43% rel. reporter activity).

Due to its activity in the reporter gene assay, bicycle A-b6 (Figure 6I was chosen for follow-up characterization. For comparison, we included monocycle 12 and its linearized analog 12-1. Initially, a concentration-dependent TOPFLASH reporter gene assay was performed showing robust Wnt inhibition for A-b6 (IC₅₀ = 8 µM, Figure 5b) with high inhibitory activity at 50 µM (2% residual activity). Neither monocycle 12 nor linear analog 12-1 displayed inhibition under these conditions (Figure 5b). Having characterized binding in a competition format (Figure 2b and 3d), we sought to determine dissociation constants (Kd) in a direct FP assay. For that purpose, 12-1 was fluorescently labeled at the N-terminus. Since 12 and A-b6 do not possess an N-terminus, we substituted a non-interacting amino acid (N631) with a lysine, which was subsequently labeled with an N-hydroxysuccinimide ester of fluorescein. FP titration experiments with full-length β-catenin revealed highest affinity for A-b6 (Kd = 91 nM) followed by monocycle 12 (Kd = 156 nM)(Figure 5c). The linear analog 12 1 exhibited low affinity (Kd approx. 2 µM). These results follow the trend observed in above FP competition assays (IC₅₀ = 8.5 µM (A-b6), 16 µM (12), >100 µM (12-1)). To assess the conformation of these three compounds in aqueous solution, circular dichroism (CD) spectra were recorded (Figure 5d). For linear 12-1 (grey), a minimum slightly below λ = 200 nm was observed which is indicative of a dominant random coil character, albeit with some degree of β-sheet formation as a shoulder around λ = 215 nm was observed.[28] In contrast, the spectrum of monocycle 12 (blue) showed a minimum at λ = 215 nm indicating an increase in β-sheet character. Bicycle A b6 displayed the strongest β-sheet character as shown by the pronounced minimum at λ = 215 nm and the maximum around λ = 200 nm.[28]

**Table 4. Inhibition in percentage (%) of the TCF/β-catenin interaction corresponding to heat map shown in Figure 3c as determined by FP with a TCF-4 tracer peptide. Values were normalized in between the values for no peptide competitor (0% complex inhibition) and E-CBD's activity at 30 µM (100% complex inhibition). Experimental details are in section 1.7. #Measurement showed an increase of fluorescence polarization.**

| | Bridge: | o | ox | b1 | b2 | b3 | b4 | b5 | b6 | b7 |
|---|---|---|---|---|---|---|---|---|---|---|
| Position of cysteins | A | 23 | 15 | 17 | 16 | 30 | 38 | 61 | 68 | 61 |
| | B | 37 | 50 | 29 | 13 | 24 | 20 | 21 | 28 | 39 |
| | C | 17 | 7 | -18# | 11 | 18 | 46 | 72 | 43 | 94 |

## Claims

1. A cyclic peptide comprising the amino acid sequence N-terminal-X₄X₃X₂X₁-a-Y₁Y₂Y₃Y₄-C-terminal, wherein -a- represents a linker, and
- wherein the peptide comprises a linker -c- between the N-terminal amino acid and C-terminal amino acid, and/or
- wherein the two amino acids of one or two of the following amino acid pairs form a side chain-to-side chain bridged di-amino acid forming a bridge -b-: X₁ and Y₁; X₂ and Y₂; X₃ and Y₃; and X₄ and Y₄; and
wherein the amino acids that are not bridged are as follows:
X₄ is a positively charged or polar amino acid (aa);
X₃ can be any aa;
X₂ is a negatively charged aa or an amide;
X₁ is a hydrophobic aa;
Y₁ is a is a negatively charged aa, or an amide;
Y₂ can be any aa;
Y₃ is a hydrophobic aa; and
Y₄ is a hydrophobic aa.

2. The cyclic peptide according to claim 1, further comprising one or more of X₅ or X₆X₅N-terminally, and/or Y₅ or Y₅Y₆ C-terminally, wherein
X₆ can be any aa;
X₅ can be any aa;
Y₅ is a hydrophobic aa, and
Y₆ is an aromatic aa, and/or,
wherein the two amino acids of one or two of the following amino acid pairs form a side chain-to-side chain bridged di-amino acid forming a bridge -b-: X₁ and Y₁; X₂ and Y₂; X₃ and Y₃; X₄ and Y₄; X₅ and Y₅; and X₆ and Y₆.

3. The cyclic peptide according to claim 1, wherein the linker -a- is a turn mimetic.

4. The cyclic peptide according to any one of the preceding claims, wherein the linker -a- is selected from:
- a dipeptide Dextrorotatory-aa (Daa)- Levorotatory aa (Laa), wherein aa refers to any amino acid, optionally wherein one or both aa are N-methylated;
- a dipeptide aa-Gly or aa-Pro, wherein aa refers to any amino acid, optionally wherein one or both aa are N-methylated; and
- a non-alpha amino acid.

5. The cyclic peptide according to any one of the preceding claims, wherein the linker -a- is selected from DPro-LPro, DPro-LLeu, DPro-LGly, DPro-LAla, LAsn-Gly, Aib-Gly, Aib-DPro, L-ornithine, 5-aminovaleric acid, dibenzofuran, and benzodiazepine.

6. The cyclic peptide according to any one of the preceding claims, wherein the linker -c- is selected from:
- a dipeptide Dextrorotatory-aa (Daa)- Levorotatory aa (Laa), wherein aa refers to any amino acid, optionally wherein one or both aa are N-methylated;
- a dipeptide aa-Gly or aa-Pro, wherein aa refers to any amino acid, optionally wherein one or both aa are N-methylated;
- a non-alpha amino acid; and
- a connector comprising 7-12 atoms connecting the N-terminal and C-terminal amino acids of the peptide comprising the amino acid sequence N-terminal-X₄X₃X₂X₁-a-Y₁Y₂Y₃Y₄-C-terminal.

7. The cyclic peptide according any one of the preceding claims, wherein the linker -c- is selected from DPro-LPro, DPro-LLeu, DPro-LGly, DPro-LAla, DPhe-LPro, LAsn-Gly, Aib-Gly, Aib-DPro, DPhe-LPro, L-ornithine, 5-aminovaleric acid, dibenzofuran, benzodiazepine, 1-4 beta-alanines, and a PEG based amino acid.

8. The cyclic peptide according any one of the preceding claims wherein the linker -c- is selected from the following structures:

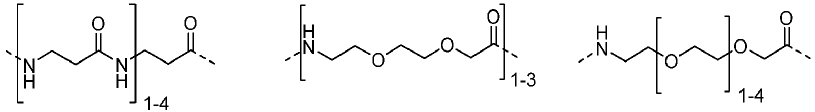

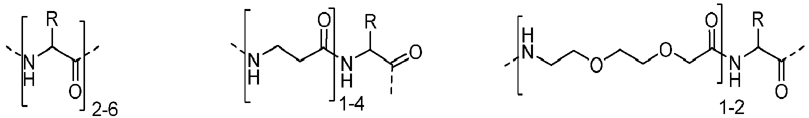

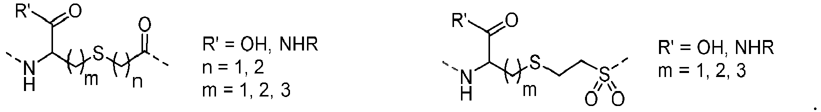

9. The peptide according to any one of the preceding claims, wherein the one or two side chain-to-side chain bridged di-amino acids forming one or two bridges -b-have the following structure: 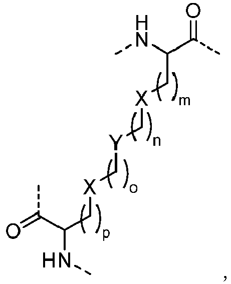
wherein m, n, o and p are each independently selected from 0, 1, 2 or 3, each X is independently CH₂, S, O or NH, and
Y is absent or selected from the following structures:

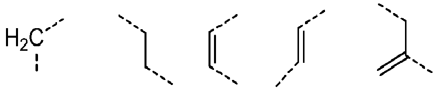

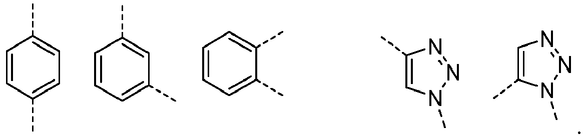

10. The cyclic peptide according to any one of the preceding claims, wherein the one or two bridges -b- are independently selected from a structure containing one or more thioether bonds and the following structures: 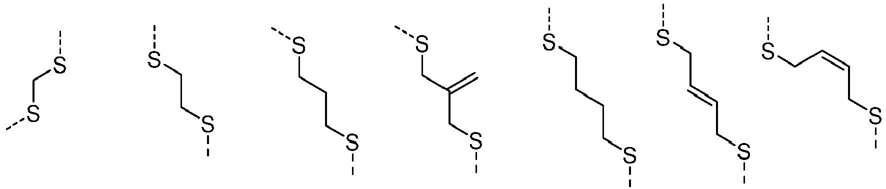 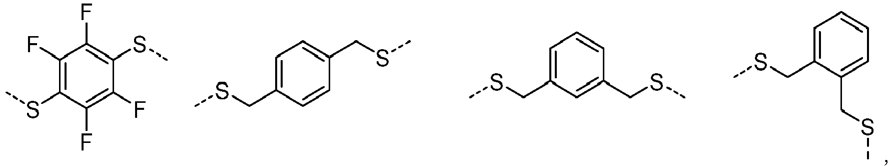 or the one or two side chain-to-side chain bridged di-amino acids forming a bridge -b- are independently selected from the following structures:

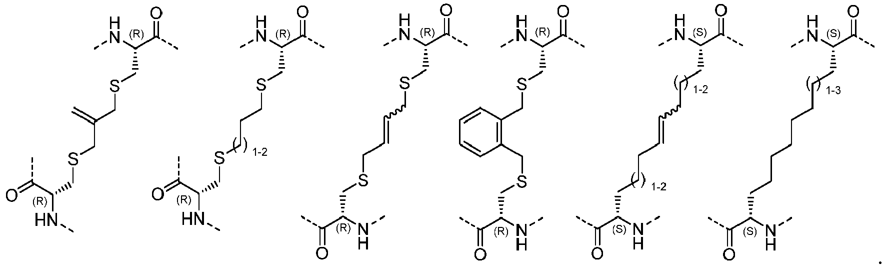

11. The peptide according to any one of the preceding claims, wherein the amino acids that are not bridged are as follows:
X₁ is selected from Val, Leu and Ile, or corresponding non-natural amino acids;
X₂ is selected from Asp, Glu, Asn, and Gln, or corresponding non-natural amino acids;
X₄ is Arg;
Y₁ is selected from Asp, Glu, Asn, and Gln, or corresponding non-natural amino acids;
Y₃ is selected from Val, Leu, Ile and Cys, or corresponding non-natural amino acids;
Y₄ is selected from Val, Leu, Ile, and Cys, or corresponding non-natural amino acids;
Y₅ is selected from Val, Leu, Ile, and Cys, or corresponding non-natural amino acids; and
Y₆ is selected from Phe, Tyr, His and Trp, or corresponding non-natural amino acids.

12. The cyclic peptide according to any one of claims 1-11, for use as a medicament.

13. The cyclic peptide according to any one of claims 1-11, for use in a method of preventing a pre-malignant condition in an individual to become cancerous, said pre-malignant condition preferably selected from colorectal adenoma and Barrett's esophagus, or for use in a method of treating an individual suffering from a tumor, wherein the tumor preferably is selected from a carcinoma such as a colorectal carcinoma, esophageal adenocarcinoma, pancreatic ductal adenocarcinoma and breast carcinoma.

14. The cyclic peptide for use according to claim 12, wherein said medicament is for treating a disease selected from osteoporosis, a chronic lung disease, and a psychiatric disorder.

15. Use of the cyclic peptide according to any one of claims 1-11, for inhibiting Wingless/integrase-1 (Wnt)-signaling in isolated tissues or cells.
